# EUROPEAN PATENT SPECIFICATION

(11) **EP 3 116 897 B1**
(45) Date of publication and mention of the grant of the patent: **02.03.2022**
(21) Application number: 15715172.1
(22) Date of filing: 11.03.2015
(51) Int. Cl.: C07K 7/62, A61K 38/12

(54) **POLYMYXIN DERIVATIVES AND THEIR USE IN COMBINATION THERAPY TOGETHER WITH DIFFERENT ANTIBIOTICS**
POLYMYXINDERIVATE UND DEREN VERWENDUNG BEI KOMBINIERTER THERAPIE IN ZUSAMMENHANG MIT VERSCHIEDENEN ANTIBIOTIKA
DÉRIVÉS DE POLYMYXINE ET LEUR USAGE DANS LA THÉRAPIE COMBINÉE EN UNION AVEC D'AUTRES ANTIBIOTIQUES

(30) Priority: 11.03.2014 GB 201404301; 26.11.2014 GB 201421019
(43) Date of publication of application: 18.01.2017
(73) Proprietor: Spero Therapeutics, Inc., Cambridge, Massachusetts 02139 (US)
(72) Inventor: BROWN, Pamela, Reading Berkshire RG1 9LS (GB); DAWSON, Michael, Reading Berkshire RG1 9LS (GB); SIMONOVIC, Mona, Reading Berkshire RG1 9LS (GB); BOAKES, Steven, Reading Berkshire RG1 9LS (GB); DUPERCHY, Esther, Reading Berkshire RG1 9LS (GB); STANWAY, Steven James, Ongar Essex CM5 0GS (GB); WILSON, Antoinette, Ongar Essex CM5 0GS (GB); MOSS, Stephen Frederick, Ongar Essex CM5 0GS (GB)
(74) Representative: Mewburn Ellis LLP
(86) International application number: PCT/EP2015/055046
(87) International publication number: WO 2015/135976

(56) References cited:
- WO-A1-2008/017734
- WO-A1-2010/075416
- WO-A1-2012/168820
- WO-A1-2013/072695
- US-B1- 8 415 307
- K OKIMURA ET AL.: "Antimicrobial acttivity of various aminocyclohexylcarbonyl-polymyxin B (2-10) derivatives", PEPTIDE SCIENCE, vol. 45, 2008, pages 243-244, XP002690948, PROTEIN RESEARCH FOUNDATION, MINOO, ISSN: 1344-7661
- N KATSUMA ET AL.: "Development of des-fatty acyl-Polymyxin decapeptide analogs with P. aeruginosa-specific antimicrobial activity", CHEMICAL AND PHARMACEUTICAL BULLETIN, vol. 57, no. 4, 2009, pages 332-336, XP002683206, Pharmaceutical Society of Japan ISSN: 0009-2363

## Description

### Field of the Invention

The present invention relates to novel compounds, combinations of compounds, pharmaceutical compositions comprising the compounds and the use of the compounds, pharmaceutical compositions and combinations for treatment, for example treatment of microbial infections, particularly those caused by Gram-negative bacteria.

### Background

In susceptible individuals, certain Gram-negative bacteria such as *Escherichia coli*, *Klebsiella pneumoniae*, *Pseudomonas aeruginosa* and *Acinetobacter baumannii* can cause serious infections, such as pneumonia, urinary tract infections, skin and skin structure infections such as wound infections, ear infections, eye infections, intra-abdominal infections, bacterial overgrowth in the gastrointestinal tract and bacteraemia/sepsis. The treatment of serious bacterial infections in clinical practice can be complicated by antibiotic resistance. Recent years have seen a rise in infections by Gram-negative bacteria which are resistant to many types of antimicrobials including broad spectrum antibiotics such as aminoglycosides, cephalosporins and even carbapenems. There is therefore a need to identify new antimicrobials that are effective against Gram-negative bacteria, in particular against multidrug resistant Gram-negative bacteria.

Polymyxins are a class of antibiotics produced by the Gram-positive bacterium *Bacillus polymyxa*. First identified in the late 1940s, polymyxins, particularly polymyxin B and polymyxin E (colistin, usually as its prodrug colistin methane sulphonate) were used in the treatment of Gram-negative infections. However, these antibiotics exhibited side effects such as neurotoxicity and nephrotoxicity. Nevertheless the polymyxins now play an important role in the therapy of MDR Gram-negative infections due to the lack of viable alternatives. However, their use in therapy is limited to treatment of last resort.

WO 2008/017734 tries to address this toxicity problem by providing polymyxin derivatives carrying at least two but no more than three positive charges. These compounds are said to be effective antibacterial agents with reduced renal toxicity. It is hypothesised in the disclosure that the reduced number of positive charges decreases the affinity of the compound for isolated rat kidney tissue which in turn may lead to a reduction in nephrotoxicity.

Certain des-fatty acyl polymyxin derivatives have also been disclosed with reduced acute toxicity in mice whilst retaining good activity against pseudomonads (Katsuma et al. Chem. Pharm. Bull. 2009; 57, 332-336; Sato et al. Chem. Pharm. Bull. 2011; 59, 597-602). The compounds were significantly less active than polymyxin B against *E. coli* and K. *pneumoniae.*

WO 2010/075416 provides urea linked aryl polymyxin decapeptides including CB182,804, which is reported to have similar activity but reduced renal toxicity compared with polymyxin B. Phenyl cyclopropane polymyxin derivatives are also described in US 8,415,307. These compounds are shown to have similar or reduced activity compared with polymyxin B.

WO 2012/168820 provides a further series of polymyxin derivatives reported to have reduced toxicity, and sometimes enhanced activity compared with polymyxin B, in which the diaminobutyrate group at position 3 in the tripeptide side chain is replaced by a diaminopropionate moiety.

There remains a need for less toxic polymyxin derivatives which offer therapeutic preparations with consistently potent activity across the target pathogens and acceptable toxicity.

The present inventors have previously described in WO 2013/072695, TW 101142961 and GCC 2012/22819, the contents of each of which are hereby incorporated in their entirety, polymyxin compounds for use in the treatment of microbial infections.

Surprisingly, the present inventors have found certain polymyxin derivatives which have reduced toxicity compared to polymyxin or colistin and are particularly effective against Gram-negative bacteria.

### Summary of the Invention

In a general aspect the present invention provides a polymyxin compound of formula (III), as described herein, and its use in a method of treatment or prophylaxis,. The compounds of formula (III) may be used to treat a microbial infection, such as a Gram-negative bacterial infection.

In a first aspect of the invention there is provided a polymyxin compound of formula (III), and pharmaceutically acceptable salts, protected forms, solvates and hydrates thereof, such as pharmaceutically acceptable salts, and hydrates thereof.

In a second aspect of the invention there is provided a pharmaceutical composition comprising the polymyxin compound of formula (III) together with one or more pharmaceutically acceptable carriers.

In a third aspect of the invention there is provided a polymyxin compound of formula (III) or a pharmaceutical composition comprising the polymyxin compound of formula (III) for use in a method of treatment of prophylaxis.

In a fourth of the invention there is provided a polymyxin compound of formula (III) or a pharmaceutical composition comprising the polymyxin compound of formula (III) for use in a method of treating a bacterial infection.

In an alternative aspect, the compounds of formula (III) or a pharmaceutical composition comprising the polymyxin compound of formula (III) are suitable for use in the treatment of fungal infections.

In a further aspect of the invention there is provided a polymyxin compound of formula (III) for use in a method of treatment or prophylaxis, in combination with an active agent, such as where the active agent has activity against Gram-negative bacteria.

Other aspects of the invention are discussed in detail herein.

### Detailed Description of the Invention

The present invention provides compounds of formula (III) and their use in medical treatment.

Broadly, the compounds of formula (III) are polymyxin compounds having an N terminal group that contains amino functionality. In addition to, or as an alternative to, the N terminal group has a nitrogen-containing heterocyclyl (or heterocyclylene) group and/or a nitrogen-containing heteroalkylene group. The presence of a basic amino group within the terminal group is associated with particular advantages, as discussed in further detail below.

The compounds of formula (III) have suitable antibacterial activity whilst also apparently exhibiting less toxicity, especially nephrotoxicity. The compounds may have comparable or improved biological activity compared to Polymyxin B or Colistin against one or more of *E. coli*, *P. aeruginosa*, *K. pneumonia*, or *A. baumannii* bacterial strains. Such compounds are useful alternatives to the polymyxin type compounds previously described in the art.

Some of the polymyxin compounds or polymyxin derivatives in the art are known or suspected to have a poor toxicity profile. For example, the use of compounds having a fatty acyl chain at the N terminal, such as Polymyxin B and Colistin, is associated with nephrotoxicity.

Vaara et al. (Antimicrob. Agents Chemother. 2008, 52, 3229) have suggested that the pharmacological and toxicity properties of a polymyxin compound may be altered with changes to the polymyxin polypeptide sequence. In particular, Vaara *et al.* have prepared a polymyxin compound having only three positive charges, whereas the polymyxin B nonapeptide carries five positive charges.

In contrast the present inventors have shown that adaptations to the N terminal of a polymyxin compound may reduce nephrotoxicity. As described herein, the N terminal has a substituent containing an amino group (which may be in the form of a nitrogen-containing heterocycle).

Furthermore, the compounds of formula (III) are capable of increasing the antimicrobial activity of a second antimicrobial agent, such as rifampicin. Such combinations have comparable or improved biological activity compared to the combination of the second agent with Polymyxin B or Colistin, for example against one or more of *E. coli, P. aeruginosa, K. pneumonia,* or *A. baumannii* strains. For example, compounds of formula (III) may have comparable biological activity compared to Polymyxin B or Colistin against one or more of *E*. *coli, P. aeruginosa, K. pneumonia,* or *A. baumannii* strains. However, when such compounds are used in combination with a second active agent, the combination has unexpectedly superior activity compared to the combination of Polymyxin B or Colistin with the same active agent. As noted above, the compounds of formula (III) may also posess an inherent antimicrobial activty.

Furthermore, the present inventors have found that each compound of formula (III) is active against a broad range of bacteria and each compound is capable of potentiating the activity of a second active agent, for example against *E. coli, P. aeruginosa, K. pneumonia,* or A. *baumannii* strains. In contrast the compounds previously described in the art have a varied profile of biological activity, and it is difficult to predict the extent to which a particular polymyxin compound will potentiate the activity of a second agent.

The compounds of formula (III) have excellent microbial activity whilst also exhibiting less toxicity compared to Polymyxin B or Colistin, for example as measured against HK-2 cells. In some cases the compounds are active even against microbial strains which are resistant or have reduced susceptibility to Polymyxin B and Colistin. The activity is associated with the presence of amino functionality at specific locations within the N terminal group. Further improvements in activity are also found where certain substituents are present in the N terminal group, and the chiral centres in the terminal group have a specific stereochemistry.

### Polymyxin Compounds of Formula (III)

The compounds of formula (III) are N-terminal derivatives of the polymyxin series of compounds. The core of the compound of formula (III) is a nonapeptide version of a polymyxin compound, such as deacylated polymyxin B nonapeptide (PMBN, Polymyxin 2-10).

The present inventors have found that the group attached to the N terminal of a polymyxin nonapeptide is an important determinant of biological activity and compound toxicity. The inventors have identified certain N terminal substituent groups that show enhanced activity and/or exhibit less toxicity compared to Polymyxin B or Colistin.

In the polymyxins, the amino acid residue at position 1 is a diamino butyric acid (Dab) residue which is acylated at its N-terminal with a fatty acyl chain. In the compounds of the invention, the N-terminal group of Polymyxin comprising Dab and the fatty acyl chain is replaced by an amine-containing moiety which is linked to a further substituent, but not linked *via* an amide bond.

Previously, it has been thought that the presence of the Dab amino acid residue at position 1 of Polymyxin B was not important for activity, and this amino acid could be deleted. Thus, polymyxin nonapeptides are known in the art for use in the treatment of microorganisms.

In preliminary work, the present inventors looked at the activity of three polymyxin B analogues against a range of microorganisms. As with other researchers, the inventors found that the deletion of the Dab residue at position 1 in Polymyxin B had little effect on the activity of the compound (compare compounds CC4 and CC6 in Table A below). However, when the inventors looked at a further analogue where the Dab residue was replaced with Gly, a substantial deterioration in biological activity was noted (compare CC5 and CC4). The inventors believe that the presence of amino functionality at the terminal of a polymyxin compound is important for maintaining activity. Thus, the compounds of the invention include amino functionality at the N terminal of a polymyxin compound. The polymyxin B nonapeptide structure is shown below, where -R is a modification of the N terminal group.

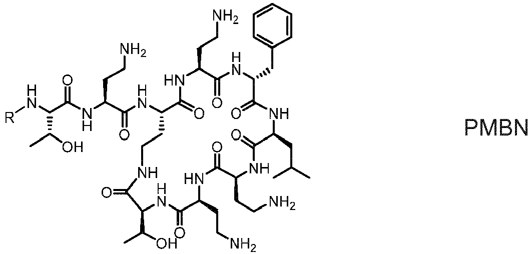

**Table A**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | NCTC 13441 | ATCC 25922 | ATCC BAA-2146 | ATCC 4352 | CCUG 59347 | ATCC 27853 | NCTC 13424 | ATCC BAA-747 |
| CC4 | 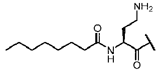 | 0.5 | 0.25 | 1 | 0.25 | 1 | 0.5 | ND | 1 |
| CC5 | 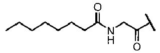 | 8 | 8 | 8 | 8 | 4 | 1 | >8 | >8 |
| CC6 | 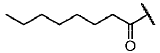 | 0.5 | 0.5 | 1 | 0.5 | 1 | 0.5 | 4 | 4 |

where the side chain -R is attached to the N terminal of the Polymyxin B nonapeptide (PMBN), and the data are MIC values at µg/mL.

The inventors believe that, for optimal activity, an amino substituent is required to mimic the Dab side chain in the naturally-occurring polymyxin structure. The inventors have therefore provided compounds of formula (III) where an amino group -NR¹⁶R¹⁷ or -N(R¹⁶)- is provided at a carbon atom that is β or γ to a group -X- at the N-terminal of a polymyxin nonapeptide. The group -X- may be regarded as equivalent to the carbonyl portion -C(O)- of an amino acid residue at position 1. The inventors have found that compounds where an amino group is provided solely at a carbon atom that is α to the group -X- have inferior biological activity (compare CC7 with example compounds D4 and D6 in Table B).

**Table B**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | *NCTC 13441* | *ATCC* 25922 | *ATCC BAA-*2146 | *ATCC 4352* | *CCUG* 59347 | ATCC 27853 | NCTC 13424 | ATCC BAA-747 |
| CC7 | 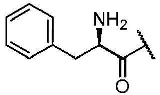 | 2 | 1 | 2 | 2 | 1 | 0.25 | 8 | 8 |
| D6 | 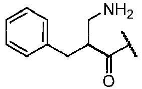 | 0.25 | 0.25 | 0.5 | 0.25 | 0.5 | 0.25 | 1 | 1 |
| | | *NCTC 13441* | *ATCC* 25922 | *ATCC BAA-*2146 | *ATCC 4352* | *CCUG* 59347 | ATCC 27853 | NCTC 13424 | ATCC BAA-747 |
| D4 | 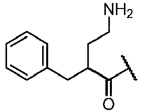 | 1 | 0.5 | 1 | 0.5 | 0.5 | 0.25 | 2 | 4 |

where the side chain -R is attached to the N terminal of the Polymyxin B nonapeptide, and the data are MIC values at µg/mL.

Compounds where the amine substituent is provided at a carbon atom that is β or γ to the group -X- at the N-terminal of PMBN have been described in WO 2013/072695. However, these compounds, if substituted, have a substituent on the carbon attached to the amine. The inventors have found that it is important that a further substituent is provided, and furthermore that this substituent is not on the carbon attached to the amine (compare B5 and B6 against example compounds D9 and D37 in Table C, where B5 and B6 are Examples 6 and 29 respectively in WO 2013/072695). Accordingly in the compounds of formula (III) an amino group -NR¹⁶R¹⁷ or -N(R¹⁶)- is connected to a methylene carbon group (-CH₂-).

**Table C**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | *NCTC 13441* | *ATCC* 25922 | *ATCC BAA-*2146 | *ATCC 4352* | *CCUG* 59347 | ATCC 27853 | NCTC ATCC 13424 747 | |
| B5 | 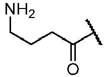 | 4 | 2 | 8 | 1 | 2 | 1 | 4 | 4 |
| B6 | 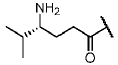 | 8 | 8 | ND | ND | ND | 4 | >8 | ND |
| D9 | 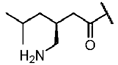 | 0.25 | 0.25 | 0.5 | 0.25 | 1 | 0.25 | 0.5 | 1 |
| D37 | 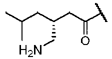 | 2 | 0.5 | 1 | 0.5 | 1 | 0.5 | 1 | 1 |

where the side chain -R is attached to the N terminal of the Polymyxin B nonapeptide, and the data are MIC values at µg/mL.

In some instances, the stereochemistry is an important determinant of activity, for example where an additional substituent is provided at the carbon atom that is α to the group -X-. In these instances, it is preferred that the stereochemistry at this position is the same as that of the L-Dab residue in Polymyxin B (compare compound D29 prepared from optically active carboxylic acid with diastereomers D24 and D25 in Table D, where B7 is Example 12 in WO 2013/072695). The data from Table D also shows the importance of a substituent group: compare B7 with D29, for example.

**Table D**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | NCTC 13441 | ATCC 25922 | ATCC BAA-2146 | ATCC 4352 | CCUG 59347 | ATCC 27853 | NCTC 13424 | ATCC BAA-747 |
| B7 | 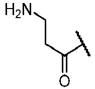 | >8 | 8 | 4 | 8 | 2 | 1 | >8 | >8 |
| D29 | 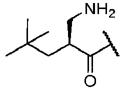 | ND | 1 | 0.5 | 0.5 | 1 | 0.5 | 0.5 | 0.5 |
| D24 | 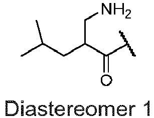 | >8 | >8 | >8 | >8 | >8 | 1 | >8 | >8 |
| D25 | 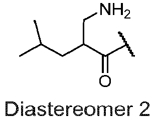 | 0.5 | 0.25 | 0.5 | 0.25 | 0.5 | 0.25 | 1 | 0.5 |

where the side chain -R is attached to the N terminal of the Polymyxin B nonapeptide, and the data are MIC values at µg/mL. Absolute stereochemistry is depicted by heavy or dashed wedges.

Provided that the amino group remains β- or γ- to the group -X-, the amine group may be part of a nitrogen-containing heterocycle. WO 2013/072695 describes compounds having a nitrogen-containing heterocycle at the N terminal of a nonapeptide. However such compounds are not substituted. The inventors have found that the addition of a substituent improves activity. The compounds of the invention, therefore, where the amine -N(R¹⁶)- is part of a ring structure, have a ring substituent (compare B8 with example compounds D2 and D39 in Table E, where B8 is Example 13 from WO 2013/072695).

**Table E**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | *NCTC 13441* | *ATCC* 25922 | *ATCC BAA-* | *ATCC* 4352 | *CCUG* 59347 | ATCC 27853 | NCTC 13424 | ATCC BAA-747 |
| B8 | 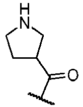 | 4 | 4 | 8 | 2 | 2 | 1 | 4 | 8 |
| D2 | 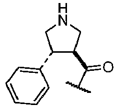 | 1 | 0.5 | 0.25 | 0.5 | 1 | 0.5 | 1 | 2 |
| D39 | 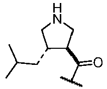 | 0.125 | 0.5 | 0.125 | 0.25 | 0.25 | 1 | 0.5 | 0.25 |

where the side chain -R is attached to the N terminal of the Polymyxin B nonapeptide, and the data are MIC values at µg/mL. The relative stereochemistry is depicted by heavy or dashed lines. Absolute stereochemistry is depicted by heavy or dashed wedges.

The compounds of formula (III) are characterised over the polymyxin decapeptides for the reason that the compounds of formula (III) do not possess the amide functionality of a polymyxin that is formed from the amino group at the α carbon of the L-Dab group at position 1 and the fatty acyl chain. In the compounds of the present invention, where an amino group is provided at the α carbon, it is not part of an amide group.

It is known that polymyxin decapeptide derivatives having a short acyl chain (e.g. butanoyl) connected to the L-Dab residue at position 1 *via* an amide bond have poor antibacterial activity. For instance the pentanoyl and butanoyl derivatives are reported to be 10-20 times less active than Polymyxin B (see de Visser et al. J. Pept. Res. 2003, 61, 298). Compound D9 (see Table C) has a short isobutyl moiety attached to an aminobutyric acid moiety. It is an analogue of the alkanoyl derivatives described by de Visser, yet it does not include the amide group of those derivatives. Compound D9 has a similar biological activity to the natural polymyxin, and it is 12 times less toxic than polymyxin B (as measured in relation to IC₅₀ values in a HK-2 assay).

A further example can be seen by comparing example compound D36 with reference compound CC8 (see Table F). Where an amino group is provided at the α carbon, it should not be part of an amide group.

**Table F**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | *NCTC 13441* | *ATCC* 25922 | *ATCC BAA-2146* | *ATCC* 4352 | *CCUG 59347* | ATCC 27853 | NCTC 13424 | ATCC BAA-747 |
| CC8 | 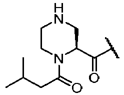 | 8 | >8 | 8 | >8 | 8 | 2 | 4 | 8 |
| D36 | 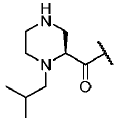 | 0.25 | 0.06 | ND | 0.25 | 1 | 0.25 | 0.25 | 0.5 |

where the side chain -R is attached to the N terminal of the Polymyxin B nonapeptide, and the data are MIC values at µg/mL. Absolute stereochemistry is depicted by heavy or dashed wedges.

As noted above, the presence of an amino group solely at the α carbon is not sufficient to provide good activity. An amino group at a β or γ carbon is required. Where an amino group, such as -NR¹⁶R¹⁷ or -N(R¹⁶)- is provided at the β or γ carbon, a *further* substituted amino group may be provided at the α carbon (and this amino group does not form part of an amide bond). Such compounds have good activity. This is seen in compound D51, whose activity is comparable to Polymyxin B (Table G).

**Table G**

| **Ex.** | **-R** | ***E. coli*** | | ***K. pneumoniae*** | | ***P. aeruginosa*** | | ***A. baumannii*** | |
|---|---|---|---|---|---|---|---|---|---|
| | | *NCTC 13441* | *ATCC* 25922 | *ATCC BAA-2146* | *ATCC* 4352 | *CCUG 59347* | ATCC 27853 | NCTC 13424 | ATCC BAA-747 |
| D51 | 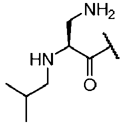 | 0.5 | 0.5 | 0.5 | 0.125 | 1 | 0.5 | 1 | 1 |
| PMB | 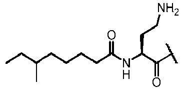 | 0.25 | 0.25 | 0.5 | 0.25 | 0.5 | 0.5 | 0.5 | 0.25 |

where the side chain -R is attached to the N terminal of the Polymyxin B nonapeptide, and the data are MIC values at µg/mL.

The present invention provides a compound of formula (III) and the use of this compound in a method of treatment. The compound of formula (III) is represented thus: 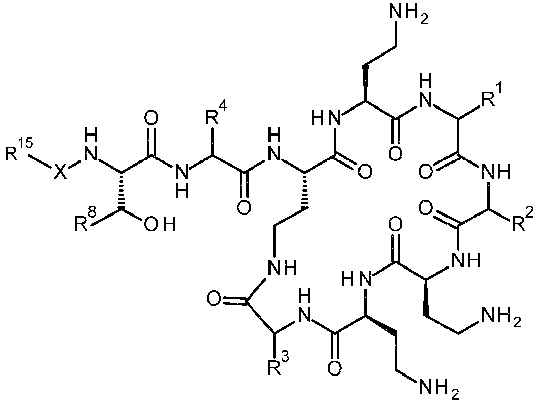 wherein:
- -X-: represents -C(O)-, -NHC(O)-, -OC(O)-, -CH₂- or -SO₂-;
- -R¹: together with the carbonyl group and nitrogen alpha to the carbon to which it is attached, is a phenylalanine, leucine or valine residue;
- -R²: together with the carbonyl group and nitrogen alpha to the carbon to which it is attached, is a leucine, iso-leucine, phenylalanine, threonine, valine or nor-valine residue;
- -R³: together with the carbonyl group and nitrogen alpha to the carbon to which it is attached, is a threonine or leucine residue;
- -R⁴: is C₁₋₆ alkyl substituted with one hydroxyl group or one amino group;
- -R¹⁵: is an amino-containing group: 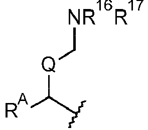
where:
- -R^{A}: is hydrogen or -L^{A}-R^{AA};
- -Q-: is a covalent bond or -CH(R^{B})-;
- -R^{B}: is hydrogen or -L^{B}-R^{BB};

or, where -Q- is -CH(R^{B})-, -R^{A} and -R^{B} together form a 5- to 10-membered monocyclic or bicyclic carbocycle, or -R^{A} and -R^{B} together form a 5- to 10-membered monocyclic or bicyclic heterocycle;
and, where -Q- is a covalent bond, -R^{A} is -L^{A}-R^{AA}, and where -Q- is -CH(R^{B})-one or both of -R^{A} and -R^{B} is not hydrogen;
-R¹⁶ is independently hydrogen or C₁₋₄ alkyl;
-R¹⁷ is independently hydrogen or C₁₋₄ alkyl;
or -NR¹⁶R¹⁷ is a guanidine group;
or -R¹⁷ and -R^{A} together form a 5- to 10-membered nitrogen-containing monocyclic or bicyclic heterocycle;
or, where -Q- is -CH(R^{B})-, -R¹⁷ and -R^{B} together form a 5- to 10-membered nitrogen-containing monocyclic or bicyclic heterocycle;
and where -R¹⁷ and -R^{A} together form a monocyclic nitrogen-containing heterocycle, each ring carbon atom in -R¹⁷ and -R^{A} is optionally mono- or di-substituted with -R^{C}, and the monocyclic heterocycle is substituted with at least one group selected from -R^{C}, -R^{N}, -R^{NA} and -L^{B}-R^{BB}, where present,
and where -R¹⁷ and -R^{B} together form a monocyclic nitrogen-containing heterocycle, each ring carbon atom in -R¹⁷ and -R^{B} is optionally mono- or di-substituted with -R^{C}, and the monocyclic heterocycle is substituted with at least one group selected from -R^{C}, and -R^{N}, where present, or the monocyclic heterocycle is optionally substituted when -R^{A} is -L^{A}-R^{AA},
and a monocyclic nitrogen-containing heterocycle optionally contains one further nitrogen, oxygen or sulfur ring atom, and where a further nitrogen ring atom is present it is optionally substituted with -R^{N}, with the exception of a further nitrogen ring atom that is connected to the carbon that is α to the group -X-, which nitrogen ring atom is optionally substituted with -R^{NA};
where -R¹⁷ and -R^{A} or -R¹⁷ and -R^{B} together form a bicyclic nitrogen-containing heterocycle, each ring carbon atom in -R¹⁷ and -R^{A} or -R¹⁷ and -R^{B} is optionally mono- or di-substituted with -R^{D};
and the bicyclic nitrogen-containing ring atom heterocycle optionally contains one, two or three further heteroatoms, where each heteroatom is independently selected from the group consisting of nitrogen, oxygen and sulfur, and where further nitrogen ring atoms are present, each further nitrogen ring atom is optionally substituted with -R^{N}, with the exception of a nitrogen ring atom that is connected to the carbon that is α to the group -X-, which nitrogen ring atom is optionally substituted with -R^{NA};
where -R^{A} and -R^{B} together form a 5- to 10-membered monocyclic carbocycle or heterocycle, each ring carbon atom in -R^{A} and -R^{B} is optionally mono- or di-substituted with -R^{C}, and a nitrogen ring atom, where present in the monocyclic heterocycle, is optionally substituted with -R^{N}, with the exception of a nitrogen ring atom that is connected to the carbon that is α to the group -X-, which nitrogen ring atom is optionally substituted with -R^{NA};
where -R^{A} and -R^{B} together form a 5- to 10-membered bicyclic carbocycle or heterocycle, each ring carbon atom in -R^{A} and -R^{B} is optionally mono- or di-substituted with -R^{D}, and a nitrogen ring atom, where present in the bicyclic heterocycle, is optionally substituted with -R^{N}, with the exception of a nitrogen ring atom that is connected to the carbon that is α to the group -X-, which nitrogen ring atom is optionally substituted with -R^{NA};
   and where R¹⁷ and -R^{A} or -R¹⁷ and -R^{B} together form a 5- to 10-membered nitrogen-containing monocyclic or bicyclic heterocycle, or where -R^{A} and -R^{B} together form a 5- to 10-membered monocyclic or bicyclic carbocycle, or together form a 5- to 10-membered monocyclic or bicyclic heterocycle, a carbon ring atom in -R¹⁷ and -R^{A}, -R¹⁷ and -R^{B}, or -R^{A} and -R^{B} is optionally alternatively substituted with oxo (=O);
each -R^{C} is independently -L^{C}-R^{CC};
each -R^{D} is independently selected from -R^{C}, -NO₂, halo, -OH, and -NH₂;
each -R^{N} is independently -L^{N}-R^{NN};
each -R^{NA} is independently -R^{L}-R^{NN} or -R^{NN};
-R^{AA}, -R^{BB}, and each -R^{CC} and -R^{NN} where present, is independently selected from C₁₋₁₂ alkyl, C₃₋₁₀ cycloalkyl, C₄₋₁₀ heterocyclyl, and C₅₋₁₂ aryl;
each -L^{A}- is independently a covalent bond or a linking group selected from -R^{L}-*, -O-L^{AA}-*, -N(R¹¹)-L^{AA}-*, and -C(O)-L^{AA}-*, where the asterisk indicates the point of attachment of the group -L^{A}- to -R^{AA};
each -L^{B}- and -L^{C}- is independently a covalent bond or a linking group selected from -R^{L}-*, -O-L^{AA}-*, -OC(O)-L^{AA}-*,-N(R¹¹)-L^{AA}-*, -N(R¹¹)C(O)-L^{AA}-*, -C(O)-L^{AA}-*, -C(O)O-L^{AA}-*, and -C(O)N(R¹¹)- L^{AA}-* , and optionally further selected from -N(R¹¹)S(O)-L^{AA}-*, -N(R¹¹)S(O)₂-L^{AA}-*, -S(O)N(R¹¹)-L^{AA}-*, and -S(O)₂N(R¹¹)-L^{AA}-* where the asterisk indicates the point of attachment of the group -L^{B}- to -R^{BB} or the group -L^{C}- to -R^{CC};
each -L^{N}- is independently a covalent bond or a group selected from -S(O)-L^{AA}-*, -S(O)₂-L^{AA}-*, -C(O)-L^{AA}-* and -C(O)N(R¹¹)-L^{AA}-*, where the asterisk indicates the point of attachment of the group -L^{N}- to -R^{NN};
and each -L^{AA}- is independently a covalent bond or -R^{L}-;
and each -R^{L}- is independently selected from C₁₋₁₂ alkylene, C₂₋₁₂ heteroalkylene, C₃₋₁₀ cycloalkylene and C₅₋₁₀ heterocyclylene, and where -L^{AA}- is connected to a group C₁₋₁₂ alkyl, -R^{L}- is not C₁₋₁₂ alkylene;
and each C₁₋₁₂ alkyl, C₃₋₁₀ cycloalkyl, C₄₋₁₀ heterocyclyl, C₅₋₁₂ aryl, C₁₋₁₂ alkylene, C₂₋₁₂ heteroalkylene, C₃₋₁₀ cycloalkylene and C₅₋₁₀ heterocyclylene group is optionally substituted, where -R^{S} is an optional substituent to carbon and -R¹² is an optional substituent to nitrogen;
each -R^{S} is independently selected from -OH, -OR¹², -OC(O)R¹² , halo, -R¹², -NHR¹², -NR¹²R¹³, -NHC(O)R¹², -N(R¹²)C(O)R¹², -SH, -SR¹², -C(O)R¹², -C(O)OH, -C(O)OR¹², -C(O)NH₂, -C(O)NHR¹² and C(O)NR¹²R¹³; except that -R¹² is not a substituent to a C₁₋₁₂ alkyl group; or where a carbon atom is di-substituted with -R^{S}, these groups may together with the carbon to which they are attached form a C₃₋₆ carbocycle or a C₅₋₆ heterocycle, where the carbocycle and the heterocycle are optionally substituted with one or more groups -R¹²;
each -R¹² is independently C₁₋₆ alkyl, C₁₋₆ haloalkyl, phenyl or benzyl;
each -R¹³ is independently C₁₋₆ alkyl, C₁₋₆ haloalkyl, phenyl or benzyl;
or -R¹² and -R¹³, where attached to N, may together form a 5- or 6-membered heterocyclic ring, which is optionally substituted with C₁₋₆ alkyl, C₁₋₆ haloalkyl, phenyl or benzyl;
each -R¹¹ is independently hydrogen or C₁₋₄ alkyl; and
   -R⁸ is hydrogen or methyl,
and pharmaceutically acceptable salts, protected forms, solvates and hydrates thereof, such as pharmaceutically acceptable salts, and hydrates thereof..

In one embodiment, -X- is -C(O)-.

In one embodiment, the carbon atom in R¹⁵ that is α to the group -X- is part of a methine (-CH-) group i.e. -R^{A} is not hydrogen.

In one embodiment, the carbon atom in R¹⁵ that is β to the group -X- is part of a methine (-CH-) group i.e. -R^{B} is not hydrogen.

In one embodiment, the carbon atom in R¹⁵ that is β to the group -X- is part of a methylene group. Thus, where -Q- is present, -R^{B} is hydrogen.

When -Q- is a covalent bond, the carbon atom in R¹⁵ that is β to the group -X- (the carbon in -CH₂NR¹⁶R¹⁷) is always part of a methylene group.

In one embodiment, the compound is in salt form, for example the compound is an acetate salt.

In one embodiment, the compound is in protected from, for example where amino and hydroxyl functionality is protected.

### Polymyxin B

Polymyxin B nonapeptide has the structure shown below: 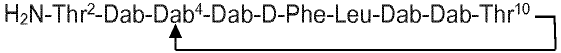 where positions 2, 4 and 10 are indicated (with reference to the numbering system used for the Polymyxin B decapeptide), and the amino acid residues are in the L-configuration, unless indicated.

The compounds of the invention are derivatives of polymyxin B nonapeptide , where (i) the N terminal amino group, -NH₂, is replaced with the group -NH-A-X-R⁵ or -NH-X-R¹⁵ as described herein and optionally (ii) the amino acid residues at 2, 3, 6, 7 and 10 positions are substituted with another amino acid residue.

For convenience, the compounds of the invention are represented by the formula (III) where the amino acids at positions 2, 3, 6, 7 or 10 are determined by the nature of the groups R⁸, R⁴, R¹, R² and R³ respectively. Compounds of the invention, which include the variants described above, are biologically active.

A variant of the compound is a compound in which one or more, for example, from 1 to 5, such as 1, 2, 3 or 4 amino acids are substituted by another amino acid. The amino acid may be at a position selected from positions 2, 3, 6, 7 or 10 (referring to the numbering of residues used in polymyxin B). The substitution may be for another amino acid or for a stereoisomer.

Set out below are various embodiments that apply to the compounds of formula (III). The embodiments are combinable, in any combination.

### -Q-

In one embodiment, -Q- is a covalent bond.

In one embodiment, -Q- is -CH(RB)-. In this embodiment, -R^{B} may be a group -L^{A}-R^{BB}, or -R^{B} together with -R¹⁷ may form a 5- to 10-membered nitrogen-containing monocyclic or bicyclic heterocycle, as described in further detail below.

Where -R¹⁷ and -R^{A} together form a nitrogen-containing heterocycle, the group -Q- is preferably a covalent bond.

In one embodiment, -Q- is -CH(R^{B})-, and forms part of a nitrogen-containing heterocycle. In this embodiment, -R^{B} may be hydrogen.

### -R¹⁶ and -R¹⁷

In one embodiment, -R¹⁶ is hydrogen.

In one embodiment, -R¹⁶ is C₁₋₄ alkyl, such as methyl, ethyl or propyl, such as methyl.

In one embodiment, -R¹⁷ is hydrogen.

In one embodiment, -R¹⁷ is C₁₋₄ alkyl, such as methyl, ethyl or propyl, such as methyl.

In one embodiment, -R¹⁷ and -R^{A} together with the carbon atoms to which they are attached, form a nitrogen-containing heterocycle. In one embodiment, -R¹⁷ and -R^{B} together with the carbon atoms to which they are attached, form a nitrogen-containing heterocycle. This is discussed in further detail below.

In one embodiment, -R¹⁶ is not ethyl when -R¹⁷ is hydrogen, methyl or ethyl.

In one embodiment, -R¹⁶ is not methyl when -R¹⁷ is hydrogen, methyl or ethyl.

In one embodiment, -R¹⁶ is hydrogen and -R¹⁷ is hydrogen.

In one embodiment, -NR¹⁶R¹⁷ is not a guanidine group.

### Nitrogen-Containing Heterocycle

The groups -R¹⁷ and -R^{A} may, together with the carbon atoms to which they are attached, form a nitrogen-containing heterocycle. Similarly, -R¹⁷ and -R^{B} may, together with the carbon atoms to which they are attached, form a nitrogen-containing heterocycle. The nitrogen in the nitrogen-containing heterocycle refers to the nitrogen atom in -N(R¹⁶)-.

The nitrogen-containing heterocycle may be a monocyclic or bicyclic nitrogen-containing heterocycle. A bicyclic nitrogen-containing heterocycle has two fused rings.

The nitrogen-containing heterocycle contains a total of 5 to 10 ring atoms. Where the nitrogen-containing heterocycle is monocyclic it may have 5 to 7 ring atoms, for example 5 to 6, such as 6, ring atoms. Where the nitrogen-containing heterocycle is bicyclic it may have 8 to 10 ring atoms, such as 9 to 10, such as 10, ring atoms. Each ring in the bicyclic heterocycle may have 5 to 7 ring atoms, for example 5 or 6, such as 6, ring atoms.

Where the nitrogen-containing heterocycle is bicyclic, one ring may be aromatic or partially unsaturated. The ring that is formed together with the carbon atoms α and β to the group -X-(the first ring) is not aromatic. It is the second ring, which is the ring fused to the first, that may be aromatic. The first ring is saturated, except for the carbon ring atoms that are shared with the second ring (bridge atoms), which may be part of the aromatic ring system of the second ring, for example.

Where the nitrogen-containing heterocycle is monocyclic, each carbon ring atom in -R¹⁷ and -R^{A} or each carbon ring atom in -R¹⁷ and -R^{B} is optionally mono- or di-substituted with -R^{C}. Where the nitrogen-containing heterocycle is bicyclic, each carbon ring atom in -R¹⁷ and -R^{A} or each carbon ring atom in -R¹⁷ and -R^{B} is optionally mono- or di-substituted with -R^{D}, as appropriate. A carbon ring atom may be unsubstituted or mono-substituted with -R^{D} if that carbon ring atom is part of an aromatic ring system, or is part of an unsaturated bond.

The group -R^{D} includes the group -R^{C}. In one embodiment, where the nitrogen-containing heterocycle is bicyclic, each carbon ring atom in the second ring is optionally mono-or di-substituted with -R^{D} and each carbon ring atom in the first ring in is optionally mono-or di-substituted with -R^{C}.

In one embodiment, the nitrogen-containing heterocycle is a monocyclic nitrogen-containing heterocycle.

In one embodiment, the nitrogen-containing heterocycle is a bicyclic nitrogen-containing heterocycle.

In one embodiment, one carbon ring atom in the nitrogen-containing heterocycle is mono- or di-substituted, such as mono-substituted, with -R^{C} or substituted with -L^{B}-R^{BB}, where present, for example mono-substituted with -R^{C}. In one embodiment, one carbon ring atom in -R¹⁷ and -R^{A} or -R¹⁷ and -R^{B} is mono- or di-substituted, such as mono-substituted, with - R^{C}, for example -L^{A}-R^{CC}. In these embodiments, the remaining carbon atoms in the nitrogen-containing heterocycle are unsubstituted. This embodiment is preferred when the nitrogen-containing heterocycle is monocyclic.

Where the nitrogen-containing heterocycle is bicyclic, each carbon ring atom in the nitrogen-containing heterocycle may be unsubstituted. Alternatively, where the nitrogen heterocycle is bicyclic one carbon ring atom in the nitrogen-containing heterocycle may be mono- or di-substituted, such as mono-substituted, with -R^{C} or -L^{B}-R^{BB}, such as with -R^{C}. For example, where the nitrogen heterocycle is bicyclic one carbon ring atom in -R¹⁷ and -R^{A} or -R¹⁷ and -R^{B} is mono- or di- substituted, such as mono-substituted, with -R^{C}, for example -L^{A}-R^{CC}. In these embodiments, the remaining carbon atoms in the nitrogen-containing heterocycle are unsubstituted.

The nitrogen-containing heterocycle may contain further hetero ring atoms independently selected from nitrogen, oxygen and sulfur. Where the nitrogen-containing heterocycle is a monocyclic, the heterocycle optionally contains one further nitrogen, oxygen or sulfur ring atom. Where the nitrogen-containing heterocycle is a bicyclic nitrogen-containing heterocycle, the heterocycle optionally contains one, two or three further heteroatoms, where each heteroatom is independently selected from the group consisting of nitrogen, oxygen and sulfur. In a bicyclic system, the further heteroatoms atoms may be provided in the first or second rings, such as the first ring.

In one embodiment, where a further heteroatom is provided, that heteroatom is nitrogen.

In one embodiment, one further heteroatom is provided, such as one further nitrogen heteroatom.

In one embodiment, the nitrogen-containing heterocycle does not contain a further heteroatom.

Where two heteroatoms are provided in a ring, they are not separated by an unsubstituted methylene group (-CH₂-) or a mono-substituted methylene group (e.g. -CH(R^{C})-), and optionally they are not separated by a di-substituted methylene group (e.g. -C(R^{C})₂-).

Where reference is made to a further nitrogen ring atom, the ring atom may be provided as a group -NH-, and the nitrogen atom may be optionally substituted with -R^{N} or -R^{NA}, as appropriate. A further nitrogen ring atom may be unsubstituted if it is part of an aromatic ring system, or is part of an unsaturated bond.

Where reference is made to a further sulfur ring atom, the sulfur ring atom may be provided as -S-, -S(O)- or -S(O)₂-, such as -S-.

Each further nitrogen ring atom is optionally substituted with a group -R^{N}, as appropriate, with the exception of a further nitrogen ring atom that is connected to the carbon that is α to the group -X-, which nitrogen ring atom is optionally substituted with -R^{NA}. This is shown schematically below for two exemplary R¹⁵-X- groups comprising monocyclic heterocycles containing a further nitrogen ring atom: 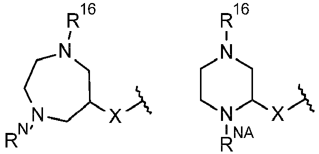 where the ring system on the right has a nitrogen ring atom that is connected to the carbon atom that is α to the group -X-. Such a nitrogen atom is optionally substituted with -R^{NA}, and is shown substituted with -R^{NA}. The ring system on the left has a nitrogen ring atom that is not connected to the carbon atom that is α to the group -X- (it is attached to a carbon β to the group -X-). Such a nitrogen atom is optionally substituted with -R^{N}, and is shown substituted with -R^{N}. In the exemplary ring structures shown above the carbon ring atoms are shown to be unsubstituted. As described herein, carbon ring atoms that are present in -R¹⁷ and -R^{A} are optionally mono- or di-substituted.

It is noted that the definitions for -R^{NA} do not encompass groups that would together with the further nitrogen ring atom form an amide group.

When a second ring is present and that second ring is an aromatic ring containing one or more further nitrogen atoms, a nitrogen atom in the aromatic ring may not be substituted with a group -R^{N}, as appropriate.

Where a further nitrogen ring atom is substituted with -R^{N} or -R^{NA}, as appropriate, each carbon ring atom in the nitrogen-containing heterocycle may be unsubstituted.

Where -R¹⁷ and -R^{A} together form a monocyclic nitrogen-containing heterocycle, the heterocycle is substituted with at least one group selected from -R^{C}, and -R^{N}, -R^{NA} and -L^{B}-R^{BB} i.e. at least one of these groups must be present as a ring substituent at the appropriate position. Thus, in this embodiment, where the nitrogen-containing heterocycle is monocyclic and does not contain a further nitrogen atom, at least one carbon ring atom must be substituted with -R^{C} or -L^{B}-R^{BB}, where present. Further, in this embodiment, where the nitrogen-containing heterocycle is monocyclic and contains a further nitrogen atom, and that nitrogen atom is unsubstituted, at least one carbon ring atom must be substituted with -R^{C} or -L^{B}-R^{BB}, where present. If a further nitrogen atom in the monocyclic nitrogen-containing heterocycle is substituted with a group -R^{N} or -R^{NA}, the carbon ring atoms may be unsubstituted or optionally mono- or di-substituted.

Where -R¹⁷ and -R^{B} together form a monocyclic nitrogen-containing heterocycle, the heterocycle is substituted with at least one group selected from -R^{C}, and -R^{N}, where present. Alternatively the heterocycle is optionally substituted if -R^{A} is -L^{A}-R^{AA}. In one embodiment, the monocyclic nitrogen-containing heterocycle is unsubstituted when the group -R^{A} is -L^{A}-R^{AA}.

If -R^{A} is hydrogen, the monocyclic nitrogen-containing heterocycle must be substituted with at least one group selected from -R^{C}, and -R^{N}, where present. Here, if the nitrogen-containing heterocycle is monocyclic and does not contain a further nitrogen atom, at least one carbon ring atom must be substituted with -R^{C}. Further, in this embodiment, where the nitrogen-containing heterocycle is monocyclic and contains a further nitrogen atom, and that nitrogen atom is unsubstituted, at least one carbon ring atom must be substituted with -R^{C}.

If a further nitrogen atom in the monocyclic nitrogen-containing heterocycle is substituted with a group -R^{N}, the carbon ring atoms may be unsubstituted or optionally mono- or di-substituted.

Where a nitrogen-containing heterocycle is bicyclic, each further nitrogen ring atom may be unsubstituted. Alternatively, where the nitrogen heterocycle is bicyclic one further nitrogen ring atom may be substituted with a group -R^{N}, except where the further nitrogen ring atom is connected to the carbon that is α to the group -X-, that further nitrogen ring atom is substituted with a group -R^{NA}.

In one embodiment, a monocyclic nitrogen-containing heterocycle is mono-substituted with -R^{C}. Thus, one carbon ring atom in the group -R¹⁷ and -R^{A} or -R¹⁷ and -R^{B} is mono-substituted with -R^{C}.

In one embodiment, a monocyclic nitrogen-containing heterocycle containing a further nitrogen ring atom is mono-substituted with a group -R^{C}, -R^{N} or -R^{NA}, for example mono-substituted with a group -R^{N} or -R^{NA} or mono-substituted with a group -R^{C}. Thus, one ring atom in the group -R¹⁷ and -R^{A} or -R¹⁷ and -R^{B} is mono-substituted.

The nitrogen-containing heterocycle may be selected from the group consisting of pyrrolidine, piperidine, piperazine, 1,4-diazepine, indoline, 1,2,3,4-tetrahydroquinoline, 1,2,3,4-tetrahydroisoquinoline, 1,2,3,4-tetrahydroquinoxaline, 1,2,3,4,6,7,8,8a-octahydropyrrolo[1,2-a]pyrazine, 1,2,3,4-tetrahydropyrrolo[1,2-a]pyrazine, 5,6,7,8-tetrahydro-1,6-naphthyridine and 1,2,3,4-tetrahydro-2,6-naphthyridine. In the bicyclic systems the aromatic ring, where present, is provided as the second ring.

The monocyclic nitrogen-containing heterocycles pyrrolidine, piperidine, piperazine, and 1,4-diazepine are substituted as discussed above.

The bicyclic nitrogen-containing heterocycles indoline, 1,2,3,4-tetrahydroquinoline, 1,2,3,4-tetrahydroisoquinoline and 1,2,3,4-tetrahydroquinoxaline may be substituted or unsubstituted, as discussed above.

A nitrogen-containing heterocycle may be selected from the group consisting of pyrrolidine, piperidine, piperazine, and 1,4-diazepine.

In one embodiment, a nitrogen-containing heterocycle is selected from pyrrolidine, piperidine and piperazine.

In one embodiment, a bicyclic nitrogen-containing heterocycle has a first ring selected from pyrrolidine, piperidine and piperazine fused to a second ring, which may be an aromatic ring.

Examples of the second ring include cyclohexane, benzene and pyridine ring

In one embodiment, the groups -R¹⁷ and -R^{A} together form a nitrogen heterocycle when -Q-is a covalent bond. Here, the group -NR¹⁶- is located on a carbon atom that is β to the group -X-.

In another embodiment, the groups -R¹⁷ and -R^{A} together form a nitrogen heterocycle when -Q- is not a covalent bond. Here, the group -NR¹⁶- is located on a carbon atom that is γ to the group -X-.

In one embodiment, -R¹⁷ and -R^{A} are selected from *-CH(R^{C1})CH(R^{C1})CH(R^{C1})-, *-CH(R^{C1})CH(R^{C1})-, and *-N(R^{NA})CH(R^{C1})CH(R^{C1})- where * indicates the point of attachment to the carbon α to the group -X-, -R^{C1} is hydrogen or -R^{C}, and at least one carbon or nitrogen atom is substituted with -R^{C} or -R^{NA}, as appropriate.

Exemplary nitrogen-containing heterocycle structures are given in the -R¹⁵ section below.

### Carbocycle and Heterocycle

In one embodiment, -R^{A} and -R^{B} together form a 5- to 10-membered carbocycle or heterocycle. Here, -Q- is not a covalent bond. The carbocycle or heterocycle may be substituted or unsubstituted.

A carbocycle or a heterocycle may be monocyclic or bicyclic. A bicyclic carbocycle or a heterocycle has two fused rings.

The carbocycle or a heterocycle contains a total of 5 to 10 ring atoms. Where the carbocycle or heterocycle is monocyclic it may have 5 to 7 ring atoms, for example 5 to 6, such as 6, ring atoms. Where the carbocycle or heterocycle is bicyclic it may have 8 to 10 ring atoms, such as 9 to 10, such as 10, ring atoms. Each ring in the bicyclic system may have 5 to 7 ring atoms, for example 5 or 6, such as 6, ring atoms.

Where the carbocycle or heterocycle is bicyclic, one ring may be aromatic or partially unsaturated. The ring that is formed together with the carbon atoms α and β to the group -X-(the first ring) is not aromatic. It is the second ring, which is the ring fused to the first, that may be aromatic. The first ring is saturated, except for the carbon ring atoms that are shared with the second ring (bridge atoms), which may be part of the aromatic ring system of the second ring.

A bicyclic heterocycle is a heterocycle having a heteroatom, such as N, S, or O in either the first or second ring.

In one embodiment, a heteroatom is present in the first ring. In one embodiment, a heteroatom is present in the second ring.

The heterocycle includes one or more heteroatoms independently selected from N, S, and O. In one embodiment heterocycle includes one or two, such as one heteroatom.

In one embodiment, the heteroatom is nitrogen.

In one embodiment, one heteroatom present, such as one nitrogen heteroatom.

Where the carbocycle or a heterocycle is monocyclic, each carbon ring atom in -R^{A} and -R^{B} is optionally mono- or di-substituted with -R^{C}.

Where the carbocycle or a heterocycle is bicyclic, each carbon ring atom in -R^{A} and -R^{B} is optionally mono- or di-substituted with -R^{D}, which includes -R^{C}.

Where reference is made to a nitrogen ring atom, the ring atom may be provided as a group -NH-, and the nitrogen atom may be optionally substituted with -R^{N} or -R^{NA}, as appropriate. A further nitrogen ring atom may be unsubstituted if it is part of an aromatic ring system, or is part of an unsaturated bond.

Where reference is made to a sulfur ring atom in the heterocycle, the sulfur ring atom may be provided as -S-, -S(O)- or -S(O)₂-, such as -S-.

In one embodiment, one carbon ring atom in the carbocycle or heterocycle is mono- or di-substituted, such as mono-substituted, with -R^{C} or -R^{D}, where appropriate. In this embodiment, the remaining carbon atoms in the carbocycle or heterocycle may be unsubstituted. This embodiment is preferred when the carbocycle or heterocycle is monocyclic.

In one embodiment, the heterocycle has a nitrogen ring atom and that atom is optionally substituted with -R^{N}, with the exception of a nitrogen ring atom that is connected to the carbon that is α to the group -X-, which nitrogen ring atom is optionally substituted with -R^{NA}. In one embodiment, where a nitrogen ring atom is present in the heterocycle, that ring atom may be substituted. In this embodiment, the remaining carbon atoms in the carbocycle or heterocycle may be unsubstituted. This embodiment is preferred when the heterocycle is monocyclic.

It is noted that the definitions for -R^{NA} do not encompass groups that would together with a nitrogen ring atom form an amide group.

When a second ring is present and that second ring is an aromatic ring containing one or more nitrogen atoms, a nitrogen atom in the aromatic ring may be substituted with a group - R^{N}, as appropriate.

In one embodiment, a monocyclic carbocycle is selected from cyclohexane and cyclopentane, which may be substituted as discussed above.

In one embodiment, a monocyclic heterocycle is selected from pyrrolidine, tetrahydrofuran, tetrahydrothiophene, piperidine, piperazine, 1,4-dioxane, morpholine, thiomorpholine and 1,4-diazepine, which may be substituted as discussed above.

In one embodiment, a monocyclic carbocycle is selected from indane and tetralin.

In one embodiment, a bicyclic heterocycle is selected from indoline, 1,2,3,4-tetrahydroquinoline, 1,2,3,4-tetrahydroisoquinoline, 1,2,3,4-tetrahydroquinoxaline, chromane, and dihydrobenzofuran, which may be substituted as discussed above.

### -R¹⁵

The group -R¹⁵ together with -X- may be regarded as an N terminal substituent group in the compounds of formula (III). -R¹⁵ contains an amino group which may be a group -NR¹⁶R¹⁷, or a group -NR¹⁶- where the nitrogen is present as a ring atom in a nitrogen-containing heterocycle.

In the compounds of the invention, the nitrogen group -NR¹⁶R¹⁷ must be bonded to one methylene group (i.e. a group -CH₂-). Thus, -R¹⁵ must contain a group -CH₂NR¹⁶R¹⁷.

When the nitrogen group -NR¹⁶- is provided in a nitrogen-containing heterocycle (i.e. -R¹⁷ and -R^{A} form a ring, or -R¹⁷ and -R^{B} form a ring), the nitrogen atom must be bonded to one neighboring carbon atom that is part of a methylene group. This is a requirement for the group -R¹⁵. However, the other neighboring ring carbon atom is not necessarily part of a methylene group (it may be a methylene or methine group). In one embodiment, the nitrogen atom in -NR¹⁶- is bonded to two ring methylene groups (i.e. both neighboring ring carbon atoms are provided in methylene groups). In one embodiment, the nitrogen atom in - NR¹⁶- is bonded to a carbon ring atom that is part of a methylene group and a carbon ring atom that is part of a methylene or methine group.

In one embodiment, -R¹⁵ is selected from the groups listed below. The groups shown below include groups where -R¹⁷ and -R^{A} together form a nitrogen-containing heterocycle.

In the embodiments below -R^{C1} is hydrogen or -R^{C}; -R^{N1} is hydrogen or -R^{NA}; -R^{D1} is hydrogen or -R^{D}; -R^{A} is hydrogen or -L^{A}-R^{AA}; -R^{B} is hydrogen or -L^{B}-R^{BB}; and -R¹⁶ is independently hydrogen or C₁₋₄ alkyl; -R¹⁷ is independently hydrogen or C₁₋₄ alkyl; or -NR¹⁶R¹⁷ is a guanidine group. As noted above, where -Q- is a covalent bond -R^{A} is -L^{A}-R^{AA}, and where -Q- is -CH(R^{B})- one or both of -R^{A} and -R^{B} is not hydrogen. Where the nitrogen-containing heterocycle is monocyclic, it should be substituted with at least one group selected from -R^{C}, and -L^{B}-R^{BB}, -R^{NA} and -R^{N}.

In one embodiment, -R¹⁵ is selected from the group consisting of:

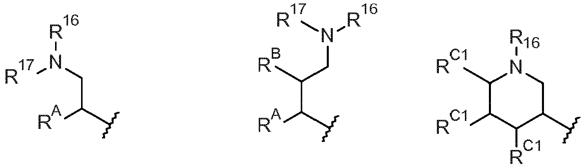

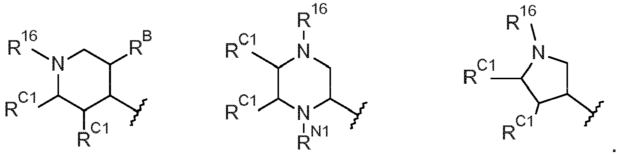

In one embodiment, -R¹⁵ is selected from the group consisting of:

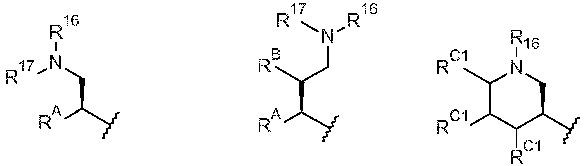

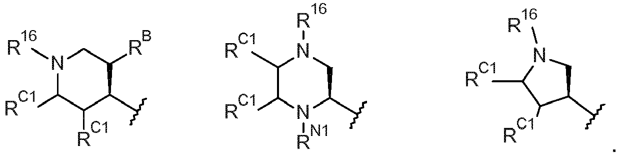

In one embodiment, -R¹⁵ is selected from the group consisting of:

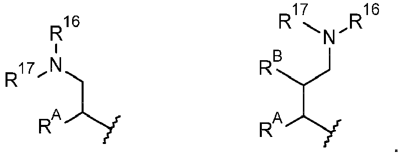

In one embodiment, -R¹⁵ is selected from the group consisting of:

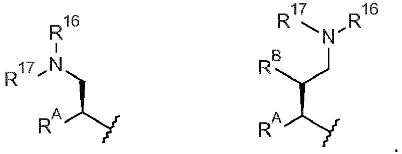

In one embodiment, -R¹⁵ is selected from the group consisting of:

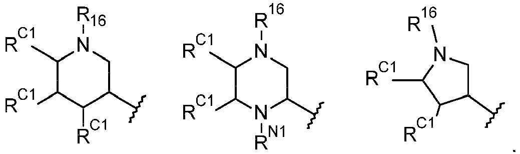

In one embodiment, -R¹⁵ is selected from the group consisting of:

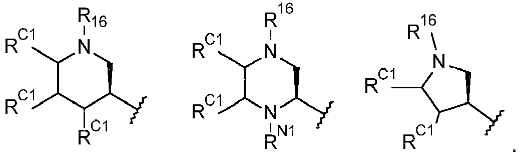

In one embodiment, -R¹⁵ is selected from the group consisting of:

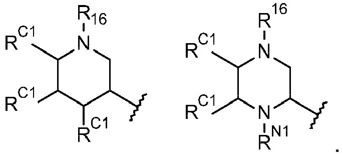

In one embodiment, -R¹⁵ is selected from the group consisting of:

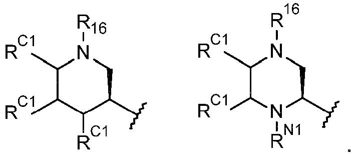

In one embodiment, -R¹⁵ is selected from the group consisting of:

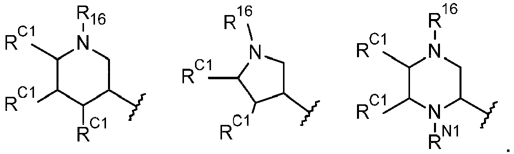

In one embodiment, -R¹⁵ is selected from the group consisting of:

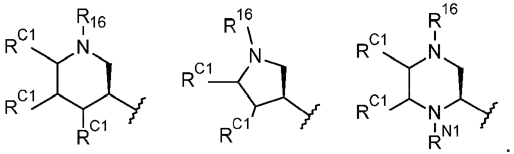

In one embodiment, -R¹⁵ is selected from the group consisting of:

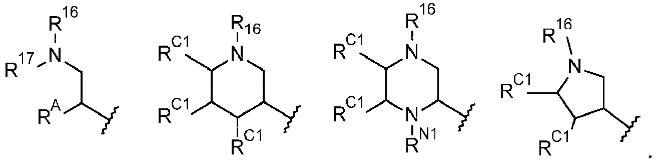

In one embodiment, -R¹⁵ is selected from the group consisting of:

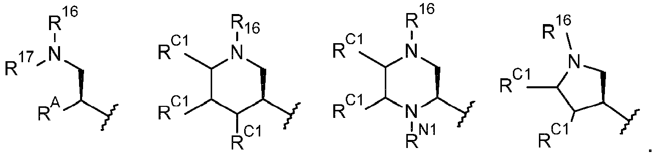

In one embodiment, -R¹⁵ is: 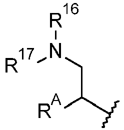 such as

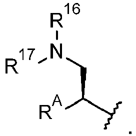

In one embodiment, -R¹⁵ is: such as 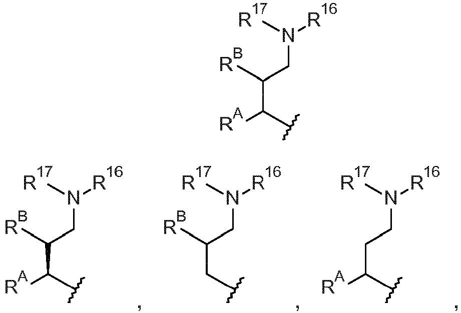 or

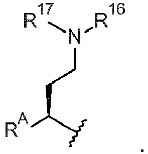

In one embodiment, -R¹⁵ is: 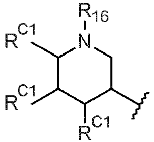 .such as,

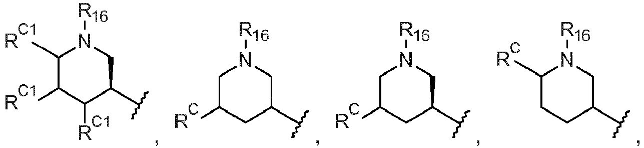

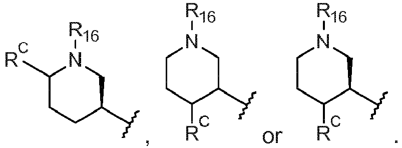

In one embodiment, -R¹⁵ is: 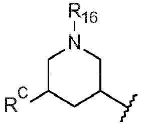 such as

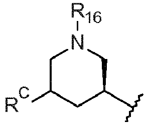

In one embodiment, -R¹⁵ is: 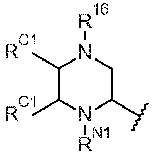 such as 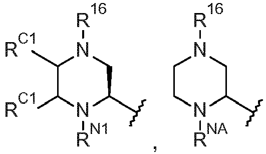 , or

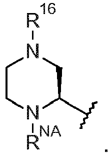

The structures shown above include examples where -R¹⁵ contains a nitrogen-containing heterocycle. These are compounds where the groups -R¹⁷ and -R^{A}, together with the carbon atoms to which they are attached, form a nitrogen heterocycle. The nitrogen heterocycles shown above are monocyclic nitrogen heterocycles.

Each carbon ring atom in the group -R¹⁷ and -R^{A} may be substituted with -R^{C1}. Where -R^{C1} is hydrogen, the carbon ring atom is unsubstituted.

A nitrogen ring atom in the group -R¹⁷ and -R^{A}, where present, is substituted with -R^{N1}. Where -R^{N1} is hydrogen, the nitrogen ring atom is unsubstituted.

Where the nitrogen-containing heterocycle contains a further nitrogen atom, it is preferred that the further nitrogen atom is substituted with -R^{N} or -R^{NA}, as appropriate. In this embodiment, the ring carbon atoms may be unsubstituted. Where the nitrogen-containing heterocycle does not contain a further nitrogen atom, one of the carbon ring atoms is substituted with -R^{C} or -L^{B}-R^{BB}, and preferably one of the carbon ring atoms group -R¹⁷ and -R^{A} is substituted with -R^{C}.

The compounds of the invention also include compounds where -R¹⁷ and -R^{A}, together with the carbon atoms to which they are attached, form a bicyclic nitrogen heterocycle. In this embodiment, it is not necessary for the carbon or nitrogen ring atoms in -R¹⁷ and -R^{A} to be substituted (i.e. each of -R^{D} and -R^{N} may be hydrogen).

Additionally or alternatively to the -R¹⁵ groups shown above, -R¹⁵ is selected from:

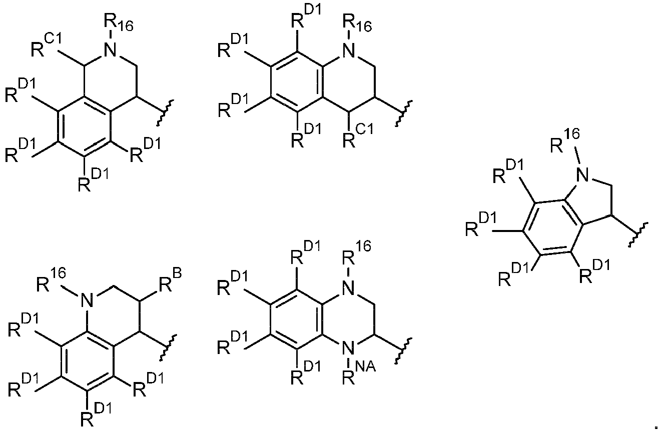

Additionally or alternatively to the -R¹⁵ groups shown above, -R¹⁵ is selected from:

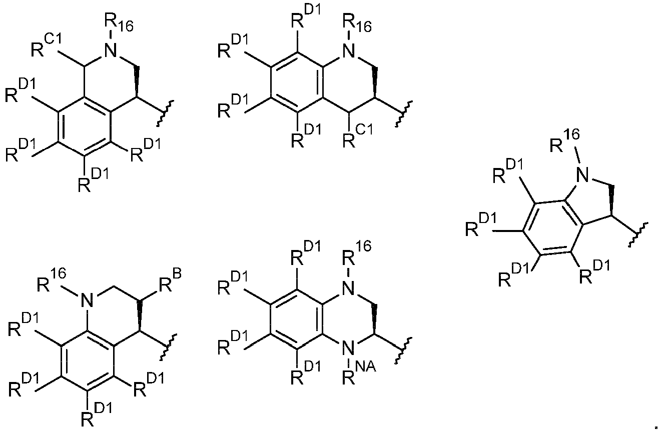

Additionally or alternatively to the -R¹⁵ groups shown above, in one embodiment -R¹⁵ is selected from: 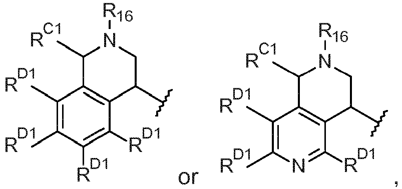 such as 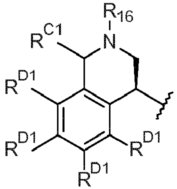 or

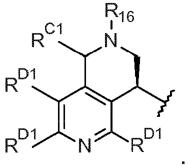

In one embodiment, -R^{A} and -R^{B} may together form a carbocycle or a heterocycle. The ring atoms of the carbocycle or heterocycle may be optionally substituted. A carbon ring atom may be optionally mono- or di-substituted with -R^{C}. A nitrogen ring atom, where present, may be optionally substituted with -R^{N}, except that a nitrogen ring atom that is connected to the carbon that is α to the group -X- is optionally substituted with -R^{NA}.

In the embodiments below -R^{C1} is hydrogen or -R^{C}; -R^{N1} is hydrogen or -R^{NA}; -R^{D1} is hydrogen or-R^{D}; and -R¹⁶ is independently hydrogen or C₁₋₄ alkyl; -R¹⁷ is independently hydrogen or C₁₋₄ alkyl; or -NR¹⁶R¹⁷ is a guanidine group. Where the nitrogen-containing carbocycle heterocycle is monocyclic, it is optionally substituted with at least one group selected from -R^{C}, and -R^{NA} and -R^{N}.

Additionally or alternatively to the -R¹⁵ groups shown above, in one embodiment -R¹⁵ is selected from:

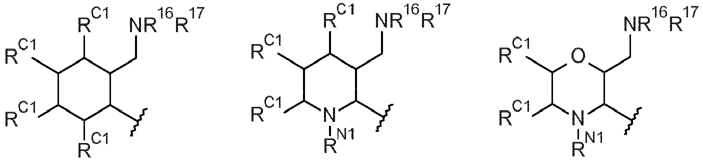

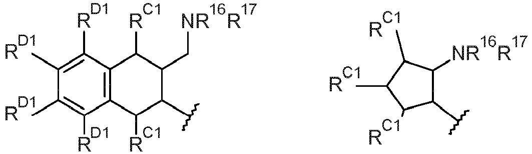

Additionally or alternatively to the -R¹⁵ groups shown above, in one embodiment -R¹⁵ is selected from:

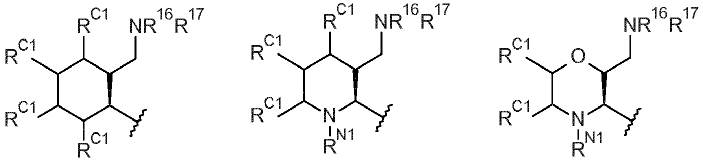

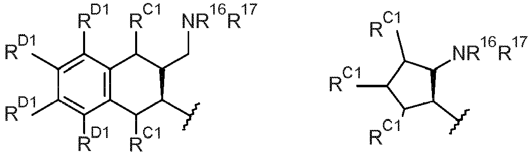

### -R^{A}

In one embodiment, -R^{A} is not hydrogen. In one embodiment, -R^{A} is -L^{A}-R^{AA}. In one embodiment, -R^{A} is -R^{AA}. In these embodiments, -R^{B}, if present, may be hydrogen.

In one embodiment, where -R^{A} is not hydrogen, for example where -R^{A} is -L^{A}-R^{AA} or -R^{A} and -R¹⁷ together form a nitrogen-containing heterocycle, -R¹⁵ is an amino-containing group:

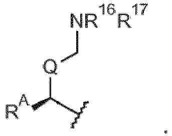

Where -R^{A} is -L^{A}-R^{AA} it is noted that this group does not encompass a substituent containing the group -C(O)N(R¹¹)-*, where the asterisk indicates the point of attachment to the carbon that is α to the group -X-. The inventors have found that where the group -C(O)N(R¹¹)-* is present, biological activity is reduced.

In one embodiment, -R^{A} and -R¹⁷ together form a 5- to 10-membered nitrogen-containing monocyclic or bicyclic heterocycle.

In one embodiment, -R^{A} and -R^{B} together form a 5- to 10-membered carbocycle or heterocycle. Here, -Q- is not a covalent bond.

In one embodiment, -R^{A} is not -NHEt or -NEt₂, for example where R¹⁵-X- is an N terminal substituent to Polymyxin B nonapeptide (PMBN).

In one embodiment, -R^{A} is not -NHR^{PA} or -N(R^{PA})₂, where each -R^{PA} is C₁₋₁₀ alkyl, such as C₈₋₁₀ alkyl, such as C₁₋₈ alkyl, such as C₁₋₄ alkyl, such as C₁₋₂ alkyl, for example where R¹⁵-X-is an N terminal substituent to Polymyxin B nonapeptide (PMBN).

In one embodiment, -R^{A} is not a group having an oxygen atom attached to the carbon that is α to the group -X-. In one embodiment, -R^{A} is not a group having a nitrogen atom attached to the carbon that is α to the group -X-. The definitions for the group -L^{A}-R^{AA} may be construed accordingly.

### -R^{B}

In one embodiment, -R^{B}, where present, is hydrogen. In one embodiment, -Q- is a covalent bond and -R^{B} is accordingly absent. In one embodiment, -R^{B} L^{B}-R^{BB}. In one embodiment, -R^{B} is -R^{BB}. In these embodiments, -R^{A} may be hydrogen.

In one embodiment, -R^{B} is not C₃₋₁₀ cycloalkyl, for example is not cyclohexyl.

In one embodiment, -R^{B} and -R¹⁷ together form a 5- to 10-membered nitrogen-containing monocyclic or bicyclic heterocycle.

In one embodiment, -R^{A} and -R^{B} together form a 5- to 10-membered carbocycle or heterocycle. Here, -Q- is not a covalent bond. -L^{B}-R^{BB}

Where -Q- is present and is part of a nitrogen-containing heterocycle and -R^{B} is L^{B}-R^{BB}, the nitrogen-containing heterocycle is optionally substituted. Thus each carbon ring atom in -R^{B} and -R¹⁷ is optionally substituted with -R^{C}, and each nitrogen ring atom in -R^{B} and -R¹⁷ is optionally substituted with -R^{N}.

In one embodiment one of -R^{A} and -R^{B} is hydrogen. The other of -R^{A} and -R^{B} is therefore not hydrogen.

It is noted that the group -L^{B}-R^{BB} encompasses a substituent containing the group -C(O)N(R¹¹)-*, where the asterisk indicates the point of attachment to the carbon that is β to the group -X-.

### -R^{C} -R^{N} and -R^{NA}

The groups -R^{A} and -R¹⁷ or -R^{B} and -R¹⁷ may together form a 5- to 10-membered nitrogen-containing monocyclic or bicyclic heterocycle, and -R^{A} and -R^{B} may together form a 5- to 10-membered monocyclic or bicyclic carbocycle, or together form a 5- to 10-monocydic or bicyclic heterocycle. The ring atoms that are present in the nitrogen-containing heterocycle and the carbocycle or heterocycle may be substituted or unsubstituted as described herein.

The nitrogen-containing heterocycle includes ring atoms that are part of -R^{A} and -R¹⁷ or -R^{B} and -R¹⁷. Where -R^{A} and -R¹⁷ or -R^{B} and -R¹⁷ form a nitrogen-containing monocyclic or bicyclic heterocycle, each carbon ring atom in the group -R^{A} and -R¹⁷ or the group -R^{B} and -R¹⁷ may be optionally substituted with -R^{C}. These carbon ring atoms may be mono- or di-substituted with -R^{C}. In one embodiment, each carbon ring atom is optionally mono-substituted with -R^{C}.

As described herein a nitrogen-containing monocyclic heterocycle must be substituted. The substituent may be present as a substituent to a ring atom that is part of -R^{A} and -R¹⁷ or -R^{B} and -R¹⁷. Thus, a group -R^{C}, -R^{N} or -R^{NA}, where appropriate, is present. Alternatively the substituent may be present at the carbon to the group -X- i.e. -L^{B}-R^{BB} is present.

The nitrogen-containing heterocycle may contain further nitrogen ring atoms. Each further nitrogen ring atom may be optionally substituted with -R^{N}, as appropriate. However, where the further nitrogen atom is bonded to the carbon that is α to the group -X-, that ring nitrogen atom is optionally substituted with -R^{NA}.

In one embodiment, -R^{A} and -R^{B} together form a 5- to 10-membered monocyclic or bicyclic carbocycle or heterocycle. In the monocycle, each ring carbon atom in -R^{A} and -R^{B} is optionally mono- or di-substituted with -R^{C}. These carbon ring atoms may be mono- or di-substituted with -R^{C}. In one embodiment, each carbon ring atom is optionally mono-substituted with -R^{C}. In the bicycle, each ring carbon atom in -R^{A} and -R^{B} is optionally mono-or di-substituted with -R^{D}. These carbon ring atoms may be mono- or di-substituted with -R^{D}.

A 5- to 10-membered monocyclic or bicyclic heterocycle may contain a nitrogen ring atom. Each nitrogen ring atom may be optionally substituted with -R^{N}, as appropriate. However, where the further nitrogen atom is bonded to the carbon that is α to the group -X-, that ring nitrogen atom is optionally substituted with -R^{NA}.

One of the carbon ring atoms that is part of -R^{A} and -R¹⁷, -R^{B} and -R¹⁷, or -R^{A} and -R^{B} may be substituted with oxo (=O). A ring carbon atom that is connected to the nitrogen atom in -N(R¹⁶)- is not substituted with oxo. Where such a carbon ring atom is substituted with oxo it may be joined to a further nitrogen ring atom (where such is present) to from an amide group. It is noted that a further nitrogen atom may be connected to the carbon atom that is α to the group -X-. The inventors understand that where an amide group is present within a nitrogen-containing heterocycle as a substituent to the carbon β to the group -X-, biological activity is not reduced.

In one embodiment, where a ring carbon atom is connected to a further nitrogen ring atom that is connected to the carbon atom that is α to the group -X-, that ring carbon atom is not substituted with oxo.

Similarly, where such a carbon ring atom is substituted with oxo it may be joined to a further oxygen ring atom (where such is present) and an ester group may be formed.

In one embodiment, the nitrogen-containing heterocycle does not include a ring amide, carbamate, urea or ester group.

In one embodiment, a further nitrogen ring atom connected to the carbon that is α to the group -X- is not part of an amide, carbamate or urea group.

In one embodiment, a further oxygen ring atom connected to the carbon that is α to the group -X- is not part of a carbamate or ester group.

Where -R¹⁷ and -R^{A} form a monocyclic nitrogen-containing heterocycle, one ring atom (formed together with the carbon atoms α and β to the group -X-) must be substituted. Here the monocyclic nitrogen heterocycle must have a substituent group present on a carbon ring atom or further nitrogen ring atom, where present. Thus at least one group -R^{C}, -R^{N}, -R^{NA} or -L^{B}-R^{BB} must be present as a substituent to the nitrogen-containing heterocycle. In one embodiment, at least one group -R^{C}, -R^{N} and -R^{NA} must be present as a substituent to the nitrogen-containing heterocycle.

In one embodiment, where -R¹⁷ and -R^{A} form a monocyclic nitrogen-containing heterocycle, one or two ring atoms in -R¹⁷ and -R^{A} are substituted. The remaining ring atoms in -R¹⁷ and -R^{A} are unsubstituted. In one embodiment, one ring atom in -R¹⁷ and -R^{A} is substituted.

In one embodiment, where R¹⁷ and -R^{A} form a monocyclic nitrogen-containing heterocycle, one carbon ring atom in -R¹⁷ and -R^{A} is substituted with -R^{C}, and the remaining ring atom in -R¹⁷ and -R^{A} are unsubstituted.

In one embodiment, where R¹⁷ and -R^{A} form a monocyclic nitrogen-containing heterocycle, and the heterocycle has a further nitrogen ring atom, the further nitrogen is substituted with -R^{N} or -R^{NA}, as appropriate, and the remaining ring atoms in -R¹⁷ and -R^{A} are unsubstituted. In one embodiment, where R¹⁷ and -R^{A} form a monocyclic nitrogen-containing heterocycle, and the heterocycle has a further nitrogen ring atom, one carbon ring atom in -R¹⁷ and -R^{A} is substituted with -R^{C}, and the remaining ring atoms in -R¹⁷ and -R^{A} are unsubstituted.

Where -R¹⁷ and -R^{B} form a monocyclic nitrogen heterocycle, the ring atoms in the ring (formed together with the carbon atom β to the group -X-) need not be substituted. If the group -R^{A} is hydrogen, the monocyclic nitrogen heterocycle must have a substituent group present on a carbon ring atom or further nitrogen ring atom, where present. However, if the group -R^{A} is not hydrogen, then the carbon ring atoms or further nitrogen ring atom, where present, need not be substituted.

In one embodiment, where -R¹⁷ and -R^{B} form a monocyclic nitrogen-containing heterocycle, one or two ring atoms in -R¹⁷ and -R^{B} are substituted. The remaining ring atoms in -R¹⁷ and -R^{B} are unsubstituted. In one embodiment, one ring atoms in -R¹⁷ and -R^{B} is substituted. In these embodiments, -R^{A} may be hydrogen.

In one embodiment, where R¹⁷ and -R^{B} form a monocyclic nitrogen-containing heterocycle, one carbon ring atom in -R¹⁷ and -R^{B} is substituted with -R^{C}, and the remaining ring atoms in -R¹⁷ and -R^{B} are unsubstituted.

In one embodiment, where R¹⁷ and -R^{B} form a monocyclic nitrogen-containing heterocycle, and the heterocycle has a further nitrogen ring atom, the further nitrogen is substituted with -R^{N}, and the remaining ring atoms in -R¹⁷ and -R^{B} are unsubstituted.

In one embodiment, where R¹⁷ and -R^{B} form a monocyclic nitrogen-containing heterocycle, and the heterocycle has a further nitrogen ring atom, one carbon ring atom in -R¹⁷ and -R^{B} is substituted with -R^{C}, and the remaining ring atoms in -R¹⁷ and -R^{B} are unsubstituted.

A bicyclic nitrogen-containing heterocycle may be unsubstituted. Here the second fused ring may be regarded as a substituent to the first ring.

In one embodiment, where R¹⁷ and -R^{A} form a bicyclic nitrogen-containing heterocycle, one carbon ring atom in -R¹⁷ and -R^{A} is substituted with -R^{D}, and the remaining ring atoms in -R¹⁷ and -R^{A} are unsubstituted.

In one embodiment, where R¹⁷ and -R^{A} form a bicyclic nitrogen-containing heterocycle, and the heterocycle has a further nitrogen ring atom, the further nitrogen is substituted with -R^{N} or -R^{NA}, as appropriate, and the remaining ring atoms in -R¹⁷ and -R^{A} are unsubstituted.

In one embodiment, where R¹⁷ and -R^{A} form a bicyclic nitrogen-containing heterocycle, and the heterocycle has a further nitrogen ring atom, one carbon ring atom in -R¹⁷ and -R^{A} is substituted with -R^{D}, and the remaining ring atoms in -R¹⁷ and -R^{A} are unsubstituted.

In one embodiment, a group -R^{D} is -R^{C} when it is provided as a substituent on the first ring of a bicyclic nitrogen-containing heterocycle.

### -R^{D}

In one embodiment, each -R^{D} is independently selected from -R^{C}, halo, -OH, and -NH₂.

In one embodiment, each -R^{D} is independently selected from -R^{C} and halo.

In one embodiment, each -R^{D} is independently -R^{C}.

In one embodiment, each -R^{D} is independently -L^{C}-R^{CC}.

A bicyclic nitrogen-containing heterocycle contains a first ring and a second ring. The first ring is the nitrogen heterocycle including the carbon atom that is 3 to the group -X-.

In one embodiment each carbon ring atom in -R¹⁷ and -R^{A} that is part of the first ring is optionally mono- or di-substituted with -R^{C}.

The second ring is the ring fused to the first ring. Each carbon ring atom in -R¹⁷ and -R^{A} that is part of the second ring is optionally mono- or di-substituted with -R^{D}.

### -L^{A}-

The group -L^{A}- may be a covalent bond.

Alternatively -L^{A}- may be a linking group. An asterisk is used to indicate the point of attachment of the group -L^{A}- to -R^{AA}. Thus, the remaining attachment point connects to the carbon that is α to the group -X-.

It is noted that -L^{A}- is not a group -N(R¹¹)C(O)-* where the asterisk is the point of attachment to -R^{AA}. The inventors have found that such groups have a poor biological activity, as discussed above.

In one embodiment, the linking group is selected from -R^{L}-*, -O-L^{AA}-*, -N(R¹¹)-L^{AA}-^{*}, and -C(O)-L^{AA}-^{*}.

In one embodiment, the linking group is selected from -R^{L}-*, -O-L^{AA}-*, and -C(O)-L^{AA}-*.

In one embodiment, the linking group is selected from -R^{L}-*, -N(R¹¹)-L^{AA}-*, and -C(O)-L^{AA}-*.

In one embodiment, the linking group is selected from -R^{L}-^{*} and -C(O)-L^{AA}-^{*}.

In one embodiment, the linking group is selected from -R^{L}-*, -O-L^{AA}-*, and -N(R¹¹)-L^{AA}-^{*}.

In one embodiment, the linking group is selected from -R^{L}-^{*} and -O-L^{AA}-*.

In one embodiment, the linking group is -R^{L}-*.

### -L^{B}-

The group -L^{B}- may be a covalent bond.

Alternatively -L^{B}- may be a linking group.

An asterisk is used to indicate the point of attachment of the group -L^{B}- to -R^{BB}. Thus, the remaining attachment point connects to the carbon that is β to the group -X- (i.e. the carbon atom in -CH(R^{B})-).

In one embodiment, the linking group is selected from R^{L}-*, -O-L^{AA}-*, -OC(O)-L^{AA}-*, -N(R¹¹)-L^{AA}-*, -C(O)-L^{AA}-*, and -C(O)O-L^{AA}-*.

In one embodiment, the linking group is selected from -R^{L}-*, -O-L^{AA}-*, -N(R¹¹)-L^{AA}-*, -C(O)-L^{AA}-*, -C(O)O-L^{AA}-*, and -C(O)N(R¹¹)-L^{AA}-^{*}.

In one embodiment, the linking group is selected from -R^{L}-*, -O-L^{AA}-*, -N(R¹¹)-L^{AA}-^{*}, -C(O)-L^{AA}-*, and -C(O)O-L^{AA}-*.

In one embodiment, the linking group is selected from -R^{L}-*, -O-L^{AA}-*, and -N(R¹¹)-L^{AA}-*.

In one embodiment, the linking group is -R^{L}-*.

Additionally or alternatively, the linking group is selected from -N(R¹¹)S(O)-L^{AA}-* and -N(R¹¹)S(O)₂-L^{AA}-^{*}.

In one embodiment, the linking group is -N(R¹¹)S(O)₂-L^{AA}-*.

In one embodiment, the linking group is -N(R¹¹)S(O)₂-*.

Additionally or alternatively, the linking group is selected from -S(O)N(R¹¹)-L^{AA}-*, and -S(O)₂N(R¹¹)-L^{AA}-*.

In one embodiment, the linking group is -S(O)N(R¹¹)-L^{AA}-*.

In one embodiment, the linking group is -S(O)₂N(R¹¹)-L^{AA}-*.

### -L^{C}-

The group -L^{C}- may be a covalent bond.

Alternatively -L^{C}- may be a linking group.

An asterisk is used to indicate the point of attachment of the group -L^{C}- to -R^{CC}. Thus, the remaining attachment point connects to the carbon ring atom.

In one embodiment, the linking group is selected from R^{L}-*, -O-L^{AA}-*, -OC(O)-L^{AA}-*,-N(R¹¹)-L^{AA}-*, -C(O)-L^{AA}-*, and -C(O)O-L^{AA}-*.

In one embodiment, the linking group is selected from -R^{L}-*, -O-L^{AA}-*, -N(R¹¹)-L^{AA}-*, -C(O)-L^{AA}-*, -C(O)O-L^{AA}-*, and -C(O)N(R¹¹)-L^{AA}-*.

In one embodiment, the linking group is selected from -R^{L}-*, -O-L^{AA}-*, -N(R¹¹)-L^{AA}-*, -C(O)-L^{AA}-*, and -C(O)O-L^{AA}-*.

In one embodiment, the linking group is selected from -R^{L}-*, -O-L^{AA}-*, and -N(R¹¹)-L^{AA}-*.

In one embodiment, the linking group is -R^{L}-*.

Additionally or alternatively, the linking group is selected from -N(R¹¹)S(O)-L^{AA}-* and -N(R¹¹)S(O)₂-L^{AA}-*.

In one embodiment, the linking group is -N(R¹¹)S(O)₂-L^{AA}-*.

In one embodiment, the linking group is -N(R¹¹)S(O)₂-*.

Additionally or alternatively, the linking group is selected from -S(O)N(R¹¹)-L^{AA}-*, and -S(O)₂N(R¹¹)-L^{AA}-*.

In one embodiment, the linking group is -S(O)N(R¹¹)-L^{AA}-*.

In one embodiment, the linking group is -S(O)₂N(R¹¹)-L^{AA}-*.

### -L^{AA}-

In one embodiment, a group -L^{AA}- is independently a covalent bond.

In one embodiment, a group -L^{AA}- is independently -R^{L}.

### -L ^{N}-

In one embodiment, a group -L^{N}- is independently a covalent bond.

In one embodiment, a group -L^{N}- is a linking group.

An asterisk is used to indicate the point of attachment of the group -L^{N}- to -R^{NN}. Thus, the remaining attachment point connects to the nitrogen ring atom.

The linking group may be independently selected from -S(O)-L^{AA}-*, -S(O)₂-L^{AA}-*, -C(O)-L^{AA}-* and -C(O)N(R¹¹)-L^{AA}-*. Thus, the linking groups may together with the nitrogen atom to which they are attached, form sulfinamide, sulfonamide, amide and urea functionality respectively.

In one embodiment, the linking group is independently selected from -S(O)₂-L^{AA}-*, -C(O)-L^{AA}-* and -C(O)N(R¹¹)-L^{AA}-*.

In one embodiment, linking is independently selected from -S(O)₂-L^{AA}-* and -C(O)N(R¹¹)-L^{AA}-*.

It is noted that the group -L^{N}- is present only as a substituent to a further ring nitrogen atom that is not connected to the carbon that is α to the group -X-. Where a further ring nitrogen atom is connected to the carbon that is α to the group -X-, it is optionally substituted with -R^{L}-R^{NN}. The group -R^{L}-R^{NN} does not allow for sulfinamide, sulfonamide, amide and urea groups connected to the carbon that is α to the group -X-. The presence of sulfinamide, sulfonamide, amide and urea functionality is believed to be tolerated at other ring positions.

### -R^{L}-

In one embodiment, each -R^{L}- is independently selected from C₁₋₁₂ alkylene, C₂₋₁₂ heteroalkylene, C₃₋₁₀ cycloalkylene and C₅₋₁₀ heterocyclylene.

However, where -L^{AA}- is connected to a group C₁₋₁₂ alkyl, -R^{L}- is not C₁₋₁₂ alkylene. In a further embodiment, where -L^{AA}- is connected to a group C₁₋₁₂ alkyl, -R^{L}- is not C₁₋₁₂ alkylene and it is not C₂₋₁₂ heteroalkylene.

Where -R^{L}- is a heteroalkylene it may be connected to -R^{AA}, -R^{BB}, -R^{CC}, or -R^{NN} *via* a heteroatom of the heteroalkylene group, such as N, O or S, where present, or a carbon atom of the heteroalkylene group. The other point of connection is made *via* a carbon atom of the heteroalkylene group, for example where the heteroalkylene is attached to a carbon atom or a heteroatom, such as N, O or S. The other point of connection may be made *via* a heteroatom of the heteroalkylene group, for example where the heteroalkylene is attached to a carbon atom. However, it is preferred that the other point of connection is made *via* a carbon atom of the heteroalkylene group, particularly where -R^{L}- is present in a group -L^{AA}-.

Where -R^{L}- is a heterocyclylene it may be connected to -R^{AA}, -R^{BB}, -R^{CC}, or -R^{NN} *via* a ring nitrogen heteroatom of the heterocyclylene group, where present, or a carbon ring atom of the heterocyclylene group. The other point of connection is made *via* a ring carbon atom of the heterocyclylene group, for example where the heterocyclylene is attached to a carbon atom or a heteroatom, such as N, O or S. The other point of connection may be made *via* a ring nitrogen heteroatom of the heterocyclylene group, for example where the heterocyclylene is attached to a carbon atom.

In one embodiment, a group -R^{L}- is independently selected from C₁₋₁₂ alkylene, and C₂₋₁₂ heteroalkylene.

In one embodiment, a group -R^{L}- is independently selected from C₁₋₁₂ alkylene and C₃₋₁₀ cycloalkylene.

In one embodiment, a group -R^{L}- is independently C₁₋₁₂ alkylene.

The group -R^{L}- may be substituted with one or more groups -R^{S}. Thus, each C₁₋₁₂ alkylene, C₂₋₁₂ heteroalkylene, C₃₋₁₀ cycloalkylene and C₅₋₁₀ heterocyclylene is optionally substituted with one or more groups -R^{S}. The specified groups may be unsubstituted or mono-substituted. The group -R^{S} may be present as a substituent to a carbon atom. A carbon atom may be optionally mono- or di-substituted with -R^{S}.

Where a nitrogen atom is present in a group, such as in a heterocyclylene group or a heteroalkylene group, that nitrogen atom may be optionally substituted with a group -R¹².

In one embodiment, a group -R^{L}- is unsubstituted.

In one embodiment, a C₁₋₁₂ alkylene group is selected from C₁₋₆ alkylene, C₁₋₄ alkylene, C₂₋₆ alkylene, and C₂₋₄ alkylene.

In one embodiment, an alkylene group is linear.

In one embodiment, a C₁₋₁₂ alkylene group is selected from -CH₂-, -CH₂CH₂-, and -CH(CH₃)-. In one embodiment, a C₁₋₁₂ alkylene group is -CH₂-, for example when it is connected to a cycloalkyl, heterocyclyl, or aryl group.

In one embodiment, a C₂₋₁₂ heteroalkylene group is selected from C₂₋₆ heteroalkylene, and C₂₋₄ heteroalklyene.

In one embodiment, a C₂₋₁₂ heteroalkylene group is selected from -CH₂O-*, -CH₂CH₂O-*, -CH₂NH-*, -CH₂CH₂NH-*, -CH₂N(R¹²)-*, and -CH₂CH₂N(R¹²)-*, where the asterisk indicates the point of attachment to -R^{AA}, -R^{BB}, -R^{CC}, or -R^{NN}. Thus, a heteroatom in the heteroalkylene group may be connected to -R^{AA}, -R^{BB}, -R^{CC}, or -R^{NN}. The other point of connection may be made *via* a carbon atom of the heteroalkylene group.

Where an S atom is present in the heteroalkylene group, it may be in the form S, S(O) or S(O)₂.

In one embodiment, the C₃₋₁₀ cycloalkylene is selected from cyclopropylene, cyclopentylene and cyclohexylene. In one embodiment, the C₃₋₁₀ cycloalkylene is cyclohexylene.

In one embodiment, the C₅₋₁₀ heterocyclylene is C₅₋₆ heterocyclylene.

In one embodiment, the C₅₋₁₀ heterocyclylene is selected from piperidinene, piperazinene, morpholinene and thiomorpholinene. The heterocyclylene may be connected to -R^{AA}, -R^{BB}, -R^{CC}, or -R^{NN} via a ring carbon or ring nitrogen atom. The other point of connection may be made *via* a carbon atom of the heterocyclylene group.

A nitrogen atom, where present, is optionally substituted with -R¹².

Where an S atom is present in the heterocyclylene group, it may be in the form S, S(O) or S(O)₂.

### -R^{AA}, -R^{BB}, -R^{CC}, and -R^{NN}

Each of -R^{AA}, -R^{BB}, -R^{CC}, and -R^{NN}, where present, is independently selected from C₁₋₁₂ alkyl, C₃₋₁₀ cycloalkyl, C₄₋₁₀ heterocyclyl, and C₅₋₁₂ aryl.

In one embodiment, a C₁₋₁₂ alkyl group is selected from C₁₋₆ alkyl, C₁₋₇ alkyl, C₁₋₄ alkyl, C₂₋₆ alkyl, C₂₋₄ alkyl, C₃₋₁₀ alkyl, C₃₋₇ alkyl, C₄₋₁₀ alkyl and C₆₋₁₀ alkyl.

In one embodiment, an alkyl group is linear.

In one embodiment, an alkyl group is branched.

In one embodiment, the C₁₋₁₂ alkyl group does not include C₈ alkyl.

In one embodiment, a C₃₋₁₀ cycloalkyl group is C₃₋₆ cycloalkyl or C₅₋₆ cycloalkyl.

In one embodiment, a C₃₋₁₀ cycloalkyl group is cyclohexyl.

In one embodiment, a C₄₋₁₀ heterocyclyl group is selected from C₅₋₁₀ heterocyclyl,

C₆₋₁₀ heterocyclyl, C₅₋₇ heterocyclyl and C₅₋₆ heterocyclyl.

In one embodiment, a C₄₋₁₀ heterocyclyl group is selected from tetrahydrofuranyl, pyrrolidinyl, tetrahydropyranyl, morpholinyl, thiomorpholinyl, piperidinyl and piperazinyl.

In one embodiment, a C₄₋₁₀ heterocyclyl group is selected from tetrahydropyanyl, morpholinyl, piperidinyl and piperazinyl.

Where an S atom is present in a heterocyclyl group, it may be in the form S, S(O) or S(O)₂. A nitrogen atom, where present, is optionally substituted with -R¹².

A heterocyclyl group may be connected *via* a ring nitrogen heteroatom atom or a ring carbon atom. Where the heterocyclyl group is a substituent to a nitrogen atom (e.g. present in the group -R^{L}-), the heterocyclyl group is connected to that nitrogen atom *via* a ring carbon atom.

An aryl group, particularly a heteroaryl group such as indole, may be connected *via* a ring nitrogen heteroatom atom or a ring carbon atom. Where the heteroaryl group is a substituent to a nitrogen atom, the heteroaryl group is connected to that nitrogen atom *via* a ring carbon atom. Typically, the aryl group is connected *via* a ring carbon atom.

In one embodiment, the C₅₋₁₂ aryl is selected from C₆₋₁₂ carboaryl and C₅₋₁₂ heteroaryl.

In one embodiment, the C₅₋₁₂ aryl is selected from phenyl, pyridyl, and naphthyl, optionally together with 1,3-benzodioxolyl and pyridonyl.

In one embodiment, the C₆₋₁₂ carboaryl is selected from phenyl, naphthyl, chromanyl, iso-chromanyl and 1,3-benzodioxolyl. The chromanyl, iso-chromanyl and 1,3-benzodioxolyl groups are connected *via* an aromatic ring carbon atom. Further discussion about the meaning of the term *carboaryl* is provided below with reference to the group -G.

In one embodiment, the C₆₋₁₂ carboaryl is selected from phenyl and naphthyl,

In one embodiment, the C₅₋₁₂ heteroaryl is selected from C₅₋₁₀ heteroaryl and C₅₋₆ heteroaryl. In one embodiment, the C₅₋₁₂ heteroaryl is selected from the group consisting of independently furanyl, thienyl, pyrrolyl, imidazolyl, pyrazolyl, oxazolyl, isoxazolyl, thiazolyl, isothiazolyl, pyridyl, pyrimidinyl, pyrazinyl, pyridazinyl, quinolinyl, isoquinolinyl, indolyl and pyridonyl.

Further discussion about the meaning of the term *heteroaryl* is provided below with reference to the group -G.

Each C₁₋₁₂ alkyl, C₃₋₁₀ cycloalkyl, C₄₋₁₀ heterocyclyl, and C₅₋₁₂ aryl group is optionally substituted with -R^{S} at carbon and -R¹² at nitrogen, where present. Each group may have one, two, three or more groups -R^{S}. In one embodiment, a heterocyclyl group or a heteroaryl group may have one, two, three or more groups -R¹².

In one embodiment, a group is mono-subsituted.

In one embodiment, a group is unsubstituted.

The group -R^{S} is present as a substituent to a carbon atom. A carbon atom may be optionally mono- or di-substituted with -R^{S}.

Where a nitrogen atom is provided, such as in a heterocyclyl group or a heteroaryl group, that nitrogen may be optionally substituted with a group -R¹².

In one embodiment, -R^{AA} is independently selected from C₁₋₁₂ alkyl and C₅₋₁₂ aryl.

In one embodiment, -R^{AA} is independently C₁₋₁₂ alkyl. In one embodiment, -R^{AA} is independently C₂₋₁₂ alkyl, such as C₃₋₁₂ alkyl.

In one embodiment, -R^{AA} is independently C₅₋₁₂ aryl.

In one embodiment, -R^{BB} is independently selected from C₁₋₁₂ alkyl, C₄₋₁₀ heterocyclyl, and C₅₋₁₂ aryl, for example when -L^{B}- is a covalent bond, or for example when -R^{A} is hydrogen. In one embodiment, -R^{BB} is independently selected from C₁₋₁₂ alkyl, C₃₋₁₀ cycloalkyl, C₄₋₁₀ heterocyclyl, and C₅₋₁₂ aryl, for example when -R^{B} is a substituent to a heterocycle ring carbon atom.

In one embodiment, -R^{BB} is independently selected from C₁₋₁₂ alkyl and C₅₋₁₂ aryl.

In one embodiment, -R^{BB} is independently C₁₋₁₂ alkyl. In one embodiment, -R^{BB} is independently C₂₋₁₂ alkyl, such as C₃₋₁₂ alkyl.

In one embodiment, -R^{BB} is independently C₅₋₁₂ aryl.

In one embodiment, a group -R^{NN} is independently selected from C₁₋₁₂ alkyl and C₅₋₁₂ aryl. In one embodiment, -R^{NN} is independently C₁₋₁₂ alkyl. In one embodiment, -R^{NN} is independently C₂₋₁₂ alkyl, such as C₃₋₁₂ alkyl.

In one embodiment, -R^{NN} is independently C₅₋₁₂ aryl.

### -R^{S}

The group -R^{S} is an optional substituent to each C₁₋₁₂ alkyl, C₃₋₁₀ cycloalkyl, C₄₋₁₀ heterocyclyl, C₅₋₁₂ aryl, C₁₋₁₂ alkylene, C₂₋₁₂ heteroalkylene, C₃₋₁₀ cycloalkylene and C₅₋₁₀ heterocyclylene group. Where a group is optionally substituted, it may be optionally substituted with one or more groups -R^{S}. A group may be optionally mono-substituted with -R^{S}.

The group -R^{S} is an optional substituent to a carbon atom. A carbon atom may be mono-, di- or tri-substituted.

In one embodiment, each -R^{S}, where present, is independently selected from -OH, -OR¹², halo, -R¹², -NHR¹², -NR¹²R¹³, -C(O)R¹², -COOH and -COOR¹².

In one embodiment, each -R^{S}, where present, is independently selected from -OR¹², halo, -R¹², -NHR¹², -NR¹²R¹³, -C(O)R¹², -COOH and -COOR¹².

In one embodiment, each -R^{S}, where present, is independently selected from -OR¹², halo, and -R¹².

Where -R^{S} is a substituent to an alkyl group, -R^{S} is not -R¹².

Where -R^{S} is halo it may be selected from fluoro, chloro, bromo and iodo, such as chloro and bromo, such as chloro.

In one embodiment, where a carbon atom is di-substituted with -R^{S}, these groups may together with the carbon to which they are attached form a C₃₋₆ carbocycle or a C₅₋₆ heterocycle, where the carbocycle and the heterocycle are optionally substituted with one or more groups -R¹². Where an S atom is present in the heterocycle group, it may be in the form S, S(O) or S(O)₂.

In one embodiment, a C₃₋₆ carbocycle is cyclopentane or cyclohexane, such as cyclohexane. In one embodiment, a C₅₋₆ heterocycle is selected from piperidine, piperazine, morpholine, thiomorpholine, tetrahydrofuran and tetrahydropyran.

### -R¹² and -R¹³

Each -R¹² and -R¹³ is independently C₁₋₆ alkyl, C₁₋₆ haloalkyl, phenyl or benzyl.

Where -R¹² and -R¹³ are both attached to N, they may together with the N atom form a 5- or 6-membered heterocycle, such as pyrrolidine, piperazine, piperidine, thiomorpholine or morpholine. The heterocyclic ring is optionally substituted with C₁₋₆ alkyl, C₁₋₆ haloalkyl, phenyl or benzyl.

In one embodiment, a -R¹² or -R¹³ group is independently C₁₋₆ alkyl, phenyl or benzyl.

In one embodiment, a -R¹² or -R¹³ group is independently C₁₋₆ alkyl.

In one embodiment, the C₁₋₆ alkyl is selected from methyl and ethyl.

In one embodiment, the C₁₋₆ haloalkyl is -CF₃.

### -R¹¹

In one embodiment, a group -R¹¹ is independently selected from hydrogen, methyl and ethyl. In one embodiment, -R¹¹ is independently hydrogen.

### -R¹

The -R¹ position corresponds to amino acid position 6 in the polymyxin compounds.

In one embodiment -R¹ together with the carbonyl group and nitrogen alpha to the carbon to which it is attached is a phenylalanine residue, for example a D-phenylalanine, or a leucine residue, such as a D-leucine residue.

### -R²

The -R² position corresponds to amino acid position 7 in the polymyxin compounds.

In one embodiment -R² together with the carbonyl group and nitrogen alpha to the carbon to which it is attached is a leucine or threonine residue, such as L-leucine or L-threonine.

### -R³

The -R³ position corresponds to amino acid position 10 in the polymyxin compounds.

In one embodiment -R³ together with the carbonyl group and nitrogen alpha to the carbon to which it is attached is a threonine residue, such as L-threonine.

### -R⁴

The -R⁴ position corresponds to the side chain of the amino acid position 3 in the polymyxin compounds.

The group -R⁴ together the carbonyl group and nitrogen alpha to the carbon to which it is attached, is an amino acid residue having an amino- or hydroxyl-containing side chain.

In one embodiment, -R⁴ is C₁₋₄ alkyl, having one amino or one hydroxyl substituent.

In one embodiment, -R⁴ has one amino substituent.

In one embodiment, -R⁴ has one hydroxyl substituent.

The amino group may be -NH₂, -NHMe or -NHEt. In one embodiment, the amino group is -NH₂.

In one embodiment, -R⁴ together with the carbonyl group and nitrogen alpha to the carbon to which it is attached, is α,γ-diaminobutyric acid (Dab), a serine residue, a threonine residue, a lysine residue, an ornithine residue, or α,β-diaminopropionic acid (Dap).

In one embodiment, -R⁴ together with the carbonyl group and nitrogen alpha to the carbon to which it is attached, is α,γ-diaminobutyric acid (Dab), a serine residue, a lysine residue, or α,β-diaminopropionic acid (Dap).

In one embodiment, -R⁴ together with the carbonyl group and nitrogen alpha to the carbon to which it is attached, is α,γ-diaminobutyric acid (Dab) or α,β-diaminopropionic acid (Dap), such as L-Dab or L-Dap.

In one embodiment, -R⁴ together with the carbonyl group and nitrogen alpha to the carbon to which it is attached, is α,γ-diaminobutyric acid (Dab) or α,β-diaminopropionic acid (Dap), such as L-Dab or L-Dap.

In one embodiment, -R⁴ together with the carbonyl group and nitrogen alpha to the carbon to which it is attached, is a lysine residue, such as L-Lys.

In one embodiment, -R⁴ together with the carbonyl group and nitrogen alpha to the carbon to which it is attached, is Dab, such as L-Dab.

Compounds of the invention where -R⁴ is a Dab side chain are obtainable from compounds such as Polymyxin B. Compounds where -R⁴ is a Dap side chain may be prepared using the methods described in WO 2012/168820. Compounds where -R⁴ is a serine side chain may be prepared using the methods described by Vaara *et al.* (see, for example, Antimicrob. Agents Chemother. 2008, 52, 3229).

### -R⁸

The amino acid residue including the group -R⁸ corresponds to position 2 in the polymyxins. In one embodiment, -R⁸ is methyl. The resulting amino acid is therefore Thr.

In one embodiment, -R⁸ is H. The resulting amino acid is therefore Ser.

### -X-

The group -X- may be selected from -NHC(O)-, -C(O)-, -OC(O)-, -CH₂- and -SO₂-.

In one embodiment -X- is selected from -C(O)-, -SO₂- and -CH₂-.

In one embodiment -X- is -C(O)-.

In one embodiment -X- is -SO₂-.

In one embodiment -X- is -CH₂-.

The right-hand side of the group -X- is the point of attachment to NH, the amino terminal of the amino acid at position 2. The left-hand side of the group -X- is the point of attachment to -R⁵ or -R¹⁵.

### Heterocyclyl, Cycloalkyl, and Aryl

For the avoidance of doubt, the index "C_{x-y}" in terms such as "C₄₋₇ heterocyclyl", and the like, refers to the number of ring atoms, which may be carbon atoms or heteroatoms (e.g., N, O, S). For example, piperidinyl is an example of a C₆heterocycyl group.

The term "heterocyclyl" in reference to the group -D refers to a group (1) which has one or more heteroatoms (e.g., N, O, S) forming part of a ring system, wherein the ring system comprises one ring or two or more fused rings, wherein at least one ring of the ring system is a non-aromatic ring, and (2) which is attached to the rest of the molecule by a non-aromatic ring atom (i.e., a ring atom that is part of a non-aromatic ring that is part of the ring system).

For example: piperidino and piperidin-4-yl are both examples of a C₆heterocycyl group; 2,3-dihydro-1H-indol-1-yl is an example of a C₉heterocycyl group; and both decahydro-quinolin-5-yl and 1,2,3,4-tetrahydroquinolin-4-yl are examples of a C₁₀heterocyclyl group.

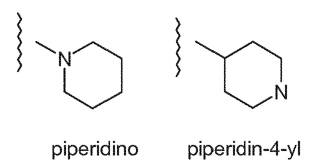

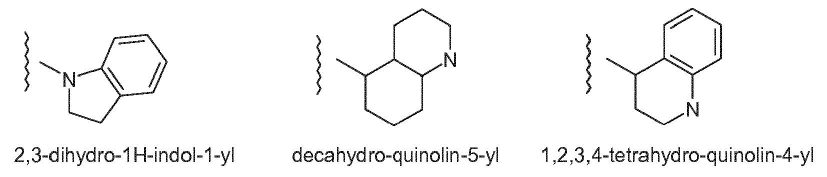

In one embodiment, where a heterocyclyl group contains two or more fused rings, each ring is non-aromatic.

In one embodiment, the heterocyclyl group comprises one ring.

For the avoidance of doubt, "cycloalkyl" refers to a group (1) which has a ring system comprising one ring or two or more fused rings, wherein one ring of the fused ring system may be an aromatic ring, and (2) which is attached to the rest of the molecule by a non-aromatic ring atom (i.e., a ring atom that is part of a non-aromatic ring that is part of the ring system). For example: cyclohexyl is an example of a C₆cycloalkyl group; and tetralin-2-yl is an example of a C₁₀ cycloalkyl group.

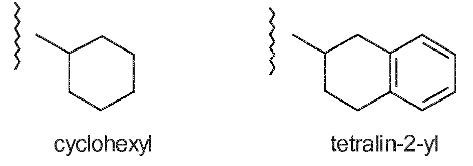

Where an aromatic ring is present, it may be optionally substituted. In one embodiment, where the cycloalkyl comprises two or more fused rings, each ring is non-aromatic.

In one embodiment, the cycloalkyl group comprises one ring.

For the avoidance of doubt, "heteroaryl" refers to a group (1) which has one or more heteroatoms (e.g., N, O, S) forming part of a ring system, wherein the ring system comprises one ring or two or more fused rings, wherein at least one ring of the ring system is an aromatic ring, and (2) which is attached to the rest of the molecule by an aromatic ring atom (i.e., a ring atom that is part of an aromatic ring that is part of the ring system). For example: pyridyl is an example of a C₆heteroaryl group; isoquinolyl is an example of a C₁₀heteroaryl group; and 1,2,3,4-tetrahydro-isoquinoline-7-yl is an example of a C₁₀heteroaryl group.

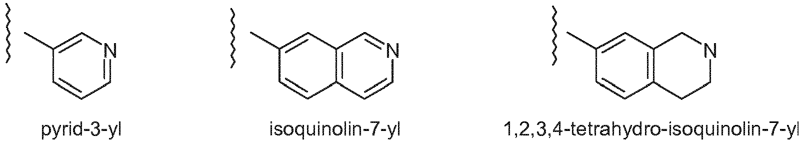

In one embodiment, where a heteroaryl comprises two or more fused rings, each ring is an aromatic ring.

In one embodiment, the heteroaryl group comprises one aromatic ring.

A heteroaryl group may also include a pyridonyl group, which may be regarded as a structure corresponding to a pyridinyl group having a 2- or 4- hydroxyl substituent.

In one embodiment, a heteroaryl group is not a pyridonyl group.

Similarly, "carboaryl" refers to a group (1) which has a ring system comprising one ring or two or more fused rings, wherein at least one ring of the ring system is an aromatic ring, and (2) which is attached to the rest of the molecule by an aromatic ring atom (i.e., a ring atom that is part of an aromatic ring that is part of the ring system). For example: phenyl is an example of a C₆ carboaryl group; and tetralin-6-yl is an example of a C₁₀ carboaryl group.

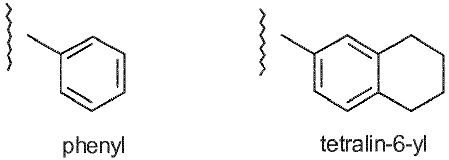

In one embodiment, where a carboaryl comprises two or more fused rings, each ring is an aromatic ring.

Where a non-aromatic ring is present, that ring may be a carbocycle (such as shown above for tetralin), or the ring may be a heterocycle, as shown below for the group dihydrobenzo[b][1,4]dioxin-6-yl.

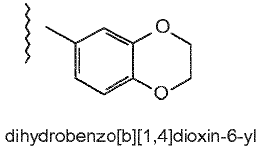

### Salts, Solvates and Other Forms

Examples of salts of compound of formula (III) include all pharmaceutically acceptable salts, such as, without limitation, acid addition salts of strong mineral acids such as HCI and HBr salts and addition salts of strong organic acids such as a methanesulfonic acid salt. Further examples of salts include sulphates and acetates such as trifluoroacetate or trichloroacetate.

In one embodiment, the salt is an acetate salt.

In one embodiment the compounds of the present disclosure are provided as a sulphate salt or a trifluoroacetic acid (TFA) salt. In one embodiment the compounds of the present disclosure are provided as acetate salts.

A compound of formula (III) can also be formulated as prodrug. Prodrugs can include an antibacterial compound herein described in which one or more amino groups are protected with a group which can be cleaved *in vivo,* to liberate the biologically active compound. In one embodiment the prodrug is an "amine prodrug". Examples of amine prodrugs include sulphomethyl, as described in e.g., Bergen et al, Antimicrob. Agents and Chemotherapy, 2006, 50, 1953 or HSO₃-FMOC, as described in e.g. Schechter et al, J.Med Chem 2002, 45(19) 4264, and salts thereof. Further examples of amine prodrugs are given by Krise and Oliyai in Biotechnology: Pharmaceutical Aspects, 2007, 5(2), 101-131.

In one embodiment a compound of formula (III) is provided as a prodrug.

A reference to a compound of formula (III) is also a reference to a solvate of that compound. Examples of solvates include hydrates.

A compound of formula (III) includes a compound where an atom is replaced by a naturally occurring or non-naturally occurring isotope. In one embodiment the isotope is a stable isotope. Thus a compound described here includes, for example deuterium containing compounds and the like. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Certain compounds of formula (III) may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

Note that, except as discussed below for tautomeric forms, specifically excluded from the term "isomers," as used herein, are structural (or constitutional) isomers (i.e., isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g., C₁₋₆alkyl includes n-propyl and iso-propyl; butyl includes n-, iso-, sec-, and tert-butyl; methoxyphenyl includes ortho-, meta-, and para-methoxyphenyl).

Unless otherwise specified, a reference to a particular compound includes all such isomeric forms, including mixtures (e.g., racemic mixtures) thereof. Methods for the preparation (e.g., asymmetric synthesis) and separation (e.g., fractional crystallisation and chromatographic means) of such isomeric forms are either known in the art or are readily obtained by adapting the methods taught herein, or known methods, in a known manner.

It is noted that certain substituent groups in the compounds of formula (III), such as R¹⁵, are given with specific stereochemical configurations.

One aspect of the present invention pertains to compounds in substantially purified form and/or in a form substantially free from contaminants.

In one embodiment, the substantially purified form is at least 50% by weight, e.g., at least 60% by weight, e.g., at least 70% by weight, e.g., at least 80% by weight, e.g., at least 90% by weight, e.g., at least 95% by weight, e.g., at least 97% by weight, e.g., at least 98% by weight, e.g., at least 99% by weight.

Unless specified, the substantially purified form refers to the compound in any stereoisomeric or enantiomeric form. For example, in one embodiment, the substantially purified form refers to a mixture of stereoisomers, i.e., purified with respect to other compounds. In one embodiment, the substantially purified form refers to one stereoisomer, e.g., optically pure stereoisomer. In one embodiment, the substantially purified form refers to a mixture of enantiomers. In one embodiment, the substantially purified form refers to an equimolar mixture of enantiomers (i.e., a racemic mixture, a racemate). In one embodiment, the substantially purified form refers to one enantiomer, e.g., optically pure enantiomer.

In one embodiment, the contaminants represent no more than 50% by weight, e.g., no more than 40% by weight, e.g., no more than 30% by weight, e.g., no more than 20% by weight, e.g., no more than 10% by weight, e.g., no more than 5% by weight, e.g., no more than 3% by weight, e.g., no more than 2% by weight, e.g., no more than 1% by weight.

Unless specified, the contaminants refer to other compounds, that is, other than stereoisomers or enantiomers. In one embodiment, the contaminants refer to other compounds and other stereoisomers. In one embodiment, the contaminants refer to other compounds and the other enantiomer.

In one embodiment, the substantially purified form is at least 60% optically pure (i.e., 60% of the compound, on a molar basis, is the desired stereoisomer or enantiomer, and 40% is the undesired stereoisomer or enantiomer), e.g., at least 70% optically pure, e.g., at least 80% optically pure, e.g., at least 90% optically pure, e.g., at least 95% optically pure, e.g., at least 97% optically pure, e.g., at least 98% optically pure, e.g., at least 99% optically pure.

The compounds of the invention also include the chemically protected forms thereof. For example amino functionality within the compound may be protected as the *tert*-butoxy (-Boc) form. As a further example, hydroxyl functionality within the compound may be protected as the *tert*-butyl (*t*-Bu) form. Protected froms are discussed in detail in the section below.

### Protected Forms

Compounds of formula (III) may be provided in a protected form. Here, reactive functionality, such as amino functionality, may be masked in order to prevent its reaction during a synthesis step. A protecting group is provided to mask the reactive functionality, and this protecting groups may be removed at a later stage of the synthesis to reveal the original reactive functionality.

A compound of formula (Ilia) is provided, and salts, solvates and hydrates thereof, which is a compound of formula (III) in protected form. For example, amino, hydroxyl, carboxyl and thiol functionality present in compound (III) may be protected with a protecting group, such as described herein. The compound of formula (IIIa) may be an intermediate for the preparation of the compound of formula (III). Thus, compound (III) may be prepared from compound (IIIa), for example by removal of the protecting groups ("deprotection").

In one embodiment, the protected form is a compound where amino, hydroxyl, thiol, and/or carboxyl functionality is protected (masked) by a protecting group. In one embodiment, the protected form is a compound where the side chain functionality of the amino acids residues with the compound are protected.

In the compound of formula (III), the amino acid residues at positions 5, 8 and 9 are Dab residues, and the side chain of the Dab residue includes amino functionality. The amino acid functionality of each Dab residue may be protected with an amino protecting group, as described herein.

In certain embodiments of the invention, -R³ together with the carbonyl group and nitrogen alpha to the carbon to which it is attached is an amino acid residue where the side chain includes hydroxyl functionality. An example of an amino acid residue including -R³ is threonine. The hydroxyl functionality of this residue may be protected with a hydroxyl protecting group, as described herein.

The group -R⁸ is part of an amino acid residue where the side chain includes hydroxyl functionality. The amino acid residue including group -R⁸ may be serine or threonine. The hydroxyl functionality of these residues may be protected with a hydroxyl protecting group, as described herein.

The group -R⁴ together with the carbonyl group and nitrogen alpha to the carbon to which it is attached is an amino acid residue where the side chain includes hydroxyl functionality or amino functionality. An example of an amino acid residue including -R⁴ is Dab. The hydroxyl or amino functionality of these residues may be protected with a hydroxyl or amino protecting group, as described herein.

In certain embodiments the group -R¹⁵ contains functionality such as amino, hydroxyl carboxyl or thiol functionality. The amino, hydroxyl carboxyl or thiol functionality may be protected with amino, hydroxyl carboxyl or thiol protecting groups, as described herein.

Protecting groups, such as those for amino acid residues, are well known and well described in the art.

Amino acids having side group protection, optionally together with amino and carboxy protection, are commercially available. Thus, a protected polymyxin compound may be prepared from appropriately protected amino acid starting materials.

Velkov *et al.* describe the step-wise preparation of polymyxin compounds on the solid-phase using suitably protected amino acids. The use of protected-forms of threonine and Dab is disclosed (see Supplementary Information).

Velkov *et al.* do not describe compounds of formula (III), for at least the reason that the group -R¹⁵ is not described or exemplified in Velkov *et al.*

Where a protecting group is used is it is removable under conditions that do not substantially disrupt the structure of the polymyxin core, for example conditions that do not alter the stereochemistry of the amino acid residues.

In one embodiment, the protecting groups are acid-labile, base labile, or are removable under reducing conditions.

Example protecting groups for amino functionality include Boc (*tert*-butoxycabonyl), Bn (benzyl, Bzl), CbZ (Z), 2-CL-Z (2-chloro), ivDde (1-[4,4-dimethyl-2,6-dioxocylcohex-1-ylidene]-3-methylbutyl), Fmoc (fluorenylmethyloxycarbonyl), HSO₃-Fmoc (sulfonylated Fmoc, such as 2-sulfo-Fmoc, as described in e.g. Schechter et al, J.Med Chem 2002, 45(19) 4264), Dde (1-[4,4-dimethyl-2,6-dioxocylcohex-1-ylidene]ethyl), Mmt (4-methoxytrityl), Mtt (4-methyltrityl), Nvoc (6-nitroveratroyloxycarbonyl), and Tfa (trifluroacetyl).

Example protecting groups for aromatic nitrogen functionality includes Boc, Mtt, Trt and Dnp (dinitrophenyl).

In one embodiment, the protecting group for amino functionality is selected from Boc, CbZ,

Bn and Fmoc and HSO₃-Fmoc.

In one embodiment, the protecting group for amino functionality is Boc, Fmoc or CbZ.

Example protecting groups for hydroxyl functionality include Trt (trityl), Bn (benzyl), tBu (*tert*-butyl), and 2-acetamido-2-deoxy-3,4,6-tri-Oacetyl-α-galactopyranosyl.

In one embodiment, the protecting group for hydroxyl functionality is Trt.

Further example protecting groups for hydroxyl functionality include silyl ether protecting groups, such as TMS, TES, TBS, TIPS, TBDMS, and TBDPS. Such protecting groups are removable with TBAF, for example.

Example protecting groups for carboxyl functionality include Bn (benzyl, Bz), tBu (*tert*-butyl), TMSET (trimethylsilylethyl) and Dmab ({1-[4,4-dimethyl-2,6-dioxocylcohex-1-ylidene]-3-methylbutyl}amino benzyl).

In some embodiments, only some types of functionality are protected. For example, only amino groups may be protected, such as amino groups in the side chain of an amino acid residue.

In one embodiment, amino groups and hydroxyl groups are protected.

### Active Agent

The compounds of formula (III) may each be used together with a second agent. The inventors have found that such combinations have greater biological activity than would be expected from the individual activity of both compounds. The compounds of formula (III) can be used to potentiate the activity of the second agent. In particular, the compounds of formula (III) may be used together with a second agent to enhance the antimicrobial activity of that agent, for example against Gram-negative bacteria.

Without wishing to be bound by theory it is believed that the compounds of formula (III) act on the outer membrane of a cell e.g. a Gram-negative bacterial cell, to facilitate the uptake of the second agent into that cell. Thus, agents that are otherwise incapable or poor at crossing the outer membrane may be taken up into a target cell by the action of the compounds of formula (III).

In one embodiment, the combination of a compound of formula (III) with the second agent is active against Gram-negative bacteria. Here, it is not essential that individually either of the compound of formula (III) or the second agent have activity against Gram-negative bacteria.

In one embodiment, the second agent is an agent having a measured MIC value against a particular microorganism, such as a bacterium, that is less than 10, less than 5, or less than 1 micrograms/mL. The microorganism may be a Gram-negative bacteria, such as a Gram-negative bacteria selected from the group consisting of *E. coli, S. enterica, K. pneumoniae, K. oxytoca; E. cloacae, E. aerogenes, E. agglomerans, A. calcoaceticus, A. baumannii; Pseudomonas aeruginosa, Stenotrophomonas maltophilia, Providencia stuartii, P. mirabilis,* and P. *vulgaris. maltophilia*

Examples of second agents that have activity against Gram-negative bacteria include beta-lactams, tetracyclines, aminoglycosides and quinolones.

In one embodiment, the second agent is an agent having a measured MIC value against a particular microorganism, such as a Gram-negative bacterium, that is more than 4, more than 8, more than 16 or more than 32 micrograms/mL. In this embodiment, the second agent may be active against Gram-positive bacteria. For example, the second agent is an agent having a measured MIC value against a particular Gram-positive bacterium that is less than 10, less than 5, or less than 1 micrograms/mL. Here, the compound of formula (III) acts to facilitate the uptake of the second agent into the Gram-negative bacterial cell. The second agent is therefore able to act on a target within the Gram-negative bacterial cell, which target may be the same as the second agent's target in a Gram-positive bacterial cell.

The Gram-positive bacteria may be selected from the group consisting of *Staphylococcus* and *Streptococcus* bacteria, such as *S*. *aureus* (including MRSA), *S*. *epidermis, E. faecalis,* and *E. faecium.*

Examples of second agents that have activity against Gram-positive bacteria (at the MIC values given above, for example), and moderate activity against Gram-negative bacteria, include rifampicin, novobiocin, macrolides, pleuromutilins. In one embodiment, a compound having moderate activity against Gram-negative bacteria may have a measured MIC value against a Gram-negative bacterium that is less than 32, less than 64, or less than 128 micrograms/mL.

Also suitable for use are agents having activity against Gram-positive bacteria and which are essentially inactive against Gram-negative bacteria. Examples include fusidic acid, oxazolidinines (e.g. linezolid), glycopeptides (e.g. vancomycin), daptomycin and lantibiotics. In one embodiment, a compound having essentially no activity against Gram-negative bacteria may have a measured MIC value against a Gram-negative bacterium that is more than 32, more then 64, more than 128, more than 256 micrograms/mL.

Under normal circumstances such agents are not necessarily suitable for use against Gram-negative bacteria owing to their relatively poor ability to cross the outer membrane of a Gram-negative bacterial cell. As explained above, when used together with a compound of formula (III), such agents are suitable for use.

In one embodiment, the active agent may be selected from the group consisting of rifampicin (rifampin), rifabutin, rifalazil, rifapentine, rifaximin, aztreonam, oxacillin, novobiocin, fusidic acid, azithromycin, ciprofloxacin, meropenem, tigecycline, erythromycin, clarithromycin and mupirocin, and pharmaceutically acceptable salts, solvates and prodrug forms thereof.

The present inventors have found that the polymyxin compounds of formula (III) may be used together with certain compounds in the rifamycin family to treat microbial infections. The rifamycin family includes isolates rifamycin A, B, C, D, E, S and SV, and synthetically derivatised versions of these compounds, such as rifampicin (rifampin), rifabutin, rifalazil, rifapentine, and rifaximin, and pharmaceutically acceptable salts and solvates thereof.

In one embodiment, the active agent is rifampicin (rifampin) and pharmaceutically acceptable salts, solvates and prodrug forms thereof.

### Methods of Treatment

The compounds of formula (III), or pharmaceutical formulations containing these compounds, are suitable for use in methods of treatment and prophylaxis. The compounds may be administered to a subject in need thereof. The compounds are suitable for use together with an active agent ("a second active agent"), for example a second active agent that is an antimicrobial agent.

The compounds of formula (III) are for use in a method of treatment of the human or animal body by therapy. In some aspects of the invention, a compound of formula (III) may be administered to a mammalian subject, such as a human, in order to treat a microbial infection.

The term "microbial infection" refers to the invasion of the host animal by pathogenic microbes. This includes the excessive growth of microbes that are normally present in or on the body of an animal. More generally, a microbial infection can be any situation in which the presence of a microbial population(s) is damaging to a host animal. Thus, an animal is "suffering" from a microbial infection when excessive numbers of a microbial population are present in or on an animal's body, or when the presence of a microbial population(s) is damaging the cells or other tissue of an animal.

The compounds may be used to treat a subject having a microbial infection, or at risk of infection from a microorganism, such as a bacterium.

The microbial infection may be a bacterial infection such as a Gram-negative bacterial infection.

Examples of Gram-negative bacteria include, but are not limited to, *Escherichia* spp., *Klebsiella* spp., *Enterobacter* spp., *Salmonella* spp., *Shigella* spp., *Citrobacter* spp., *Morganella morganii, Yersinia pseudotuberculosis* and other Enterobacteriaceae, *Pseudomonas* spp., *Acinetobacter* spp., *Moraxella, Helicobacter, Stenotrophomonas, Bdellovibrio,* acetic acid bacteria, *Legionella* and alpha-proteobacteria such as *Wolbachia* and numerous others.

Medically relevant Gram-negative cocci include three organisms, which cause a sexually transmitted disease (*Neisseria gonorrhoeae*), a meningitis (*Neisseria meningitidis*)*,* and respiratory symptoms (*Moraxella catarrhalis*)*.*

Medically relevant Gram-negative bacilli include a multitude of species. Some of them primarily cause respiratory problems *Haemophilus influenzae, Klebsiella pneumoniae, Legionella pneumophila, Pseudomonas aeruginosa*)*,* primarily urinary problems (*Escherichia coli, Enterobacter cloacae*)*,* and primarily gastrointestinal problems (*Helicobacter pylori, Salmonella enterica*)*.*

Gram-negative bacteria associated with nosocomial infections include *Acinetobacter baumannii,* which causes bacteremia, secondary meningitis, and ventilator-associated pneumonia in intensive-care units of hospital establishments.

In one embodiment the Gram-negative bacterial species is selected from the group consisting *of E. coli, S. enterica, K. pneumoniae, K. oxytoca;* E. cloacae, E. aerogenes, E. agglomerans, *A. calcoaceticus, A. baumannii; Pseudomonas aeruginosa, Stenotrophomonas maltophilia*, *Providencia stuartii, P. mirabilis,* and *P*. *vulgaris.*

In one embodiment the Gram-negative bacterial species is selected from the group consisting of *E*. *coli, K. pneumoniae, Pseudomonas aeruginosa,* and *A. baumannii.*

The compounds of formula (III) or compositions comprising the same are useful for the treatment of skin and soft tissue infections, gastrointestinal infection, urinary tract infection, pneumonia, sepsis, intra-abdominal infection and obstetrical/gynaecological infections. The infections may be Gram-positive or Gram-negative bacterial infections.

The compounds of formula (III) or compositions comprising the same are useful for the treatment of *Pseudomonas* infections including *P*. *aeruginosa* infection, for example skin and soft tissue infections, gastrointestinal infection, urinary tract infection, pneumonia and sepsis.

The compounds of formula (III) or compositions comprising the same are useful for the treatment of *Acinetobacter* infections including *A. baumannii* infection, for pneumonia, urinary tract infection and sepsis.

The compounds of formula (III) or compositions comprising the same are useful for the treatment of *Klebsiella* infections including *K. pneumoniae* infection, for pneumonia, urinary tract infection, meningitis and sepsis.

The compounds of formula (III) or compositions comprising the same are useful for the treatment of *E. coli* infection including *E. coli* infections, for bacteremia, cholecystitis, cholangitis, urinary tract infection, neonatal meningitis and pneumonia.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, alleviation of symptoms of the condition, amelioration of the condition, and cure of the condition. Treatment as a prophylactic measure (i.e., prophylaxis) is also included. For example, use with patients who have not yet developed the condition, but who are at risk of developing the condition, is encompassed by the term "treatment."

The term "therapeutically-effective amount," as used herein, pertains to that amount of a compound, or a material, composition or dosage form comprising a compound, which is effective for producing some desired therapeutic effect, commensurate with a reasonable benefit/risk ratio, when administered in accordance with a desired treatment regimen.

The term "treatment" includes combination treatments and therapies, as described herein, in which two or more treatments or therapies are combined, for example, sequentially or simultaneously.

### Combination Therapy

A compound of formula (III) may be administered in conjunction with an active agent. Administration may be simultaneous, separate or sequential.

The methods and manner of administration will depend on the pharmacokinetics of the compound of formula (III) and the second agent.

By "simultaneous" administration, it is meant that a compound of formula (III) and a second agent are administered to a subject in a single dose by the same route of administration.

By "separate" administration, it is meant that a compound of formula (III) and a second agent are administered to a subject by two different routes of administration which occur at the same time. This may occur for example where one agent is administered by infusion and the other is given orally during the course of the infusion.

By "sequential" it is meant that the two agents are administered at different points in time, provided that the activity of the first administered agent is present and ongoing in the subject at the time the second agent is administered.

Generally, a sequential dose will occur such that the second of the two agents is administered within 48 hours, preferably within 24 hours, such as within 12, 6, 4, 2 or 1 hour(s) of the first agent. Alternatively, the active agent may be administered first, followed by the compound of formula (III).

Ultimately, the order and timing of the administration of the compound and second agent in the combination treatment will depend upon the pharmacokinetic properties of each.

The amount of the compound of formula (III) to be administered to a subject will ultimately depend upon the nature of the subject and the disease to be treated. Likewise, the amount of the active agent to be administered to a subject will ultimately depend upon the nature of the subject and the disease to be treated.

### Formulations

In one aspect, the present invention provides a pharmaceutical composition comprising a compound of formula (III) together with a pharmaceutically acceptable carrier. The pharmaceutical composition may additionally comprise a second active agent. In an alternative embodiment, where a second agent is provided for use in therapy, the second agent may be separately formulated from the compound of formula optionally (III). The comments below made in relation to the compound of formula optionally (III) may therefore also apply to the second agent, as separately formulated.

While it is possible for the compound of formula (III) to be administered alone or together with the second agent, it is preferable to present it as a pharmaceutical formulation (e.g., composition, preparation, medicament) comprising at least one compound of formula (III), as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents. The formulation may further comprise other active agents, for example, other therapeutic or prophylactic agents.

Thus, the present invention further provides pharmaceutical compositions, as defined above, and methods of making a pharmaceutical composition comprising admixing at least one compound of formula (III), as described herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the compound. The composition optionally further comprises the second active agent in a predetermined amount.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

Suitable carriers, diluents, excipients, etc. can be found in standard pharmaceutical texts, for example, Remington's Pharmaceutical Sciences, 18th edition, Mack Publishing Company, Easton, Pa., 1990; and Handbook of Pharmaceutical Excipients, 5th edition, 2005.

The formulations may be prepared by any methods well known in the art of pharmacy. Such methods include the step of bringing into association the compound of formula (III) with a carrier which constitutes one or more accessory ingredients. In general, the formulations are prepared by uniformly and intimately bringing into association the compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations may suitably be in the form of liquids, solutions (e.g., aqueous, non-aqueous), suspensions (e.g., aqueous, non-aqueous), emulsions (e.g., oil-in-water, water-in-oil), elixirs, syrups, electuaries, mouthwashes, drops, tablets (including, e.g., coated tablets), granules, powders, losenges, pastilles, capsules (including, e.g., hard and soft gelatin capsules), cachets, pills, ampoules, boluses, suppositories, pessaries, tinctures, gels, pastes, ointments, creams, lotions, oils, foams, sprays, mists, or aerosols.

Formulations may suitably be provided as a patch, adhesive plaster, bandage, dressing, or the like which is impregnated with one or more compounds and optionally one or more other pharmaceutically acceptable ingredients, including, for example, penetration, permeation, and absorption enhancers. Formulations may also suitably be provided in the form of a depot or reservoir.

The compound may be dissolved in, suspended in, or admixed with one or more other pharmaceutically acceptable ingredients. The compound may be presented in a liposome or other microparticulate which is designed to target the compound, for example, to blood components or one or more organs. Where a liposome is used, it is noted that the liposome may contain both the compound of formula (III) and the second agent.

Formulations suitable for oral administration (e.g., by ingestion) include liquids, solutions (e.g., aqueous, non-aqueous), suspensions (e.g., aqueous, non-aqueous), emulsions (e.g., oil-in-water, water-in-oil), elixirs, syrups, electuaries, tablets, granules, powders, capsules, cachets, pills, ampoules, boluses.

Formulations suitable for buccal administration include mouthwashes, losenges, pastilles, as well as patches, adhesive plasters, depots, and reservoirs. Losenges typically comprise the compound in a flavoured basis, usually sucrose and acacia or tragacanth. Pastilles typically comprise the compound in an inert matrix, such as gelatin and glycerin, or sucrose and acacia. Mouthwashes typically comprise the compound in a suitable liquid carrier.

Formulations suitable for sublingual administration include tablets, losenges, pastilles, capsules, and pills.

Formulations suitable for oral transmucosal administration include liquids, solutions (e.g., aqueous, non-aqueous), suspensions (e.g., aqueous, non-aqueous), emulsions (e.g., oil-in-water, water-in-oil), mouthwashes, losenges, pastilles, as well as patches, adhesive plasters, depots, and reservoirs.

Formulations suitable for non-oral transmucosal administration include liquids, solutions (e.g., aqueous, non-aqueous), suspensions (e.g., aqueous, non-aqueous), emulsions (e.g., oil-in-water, water-in-oil), suppositories, pessaries, gels, pastes, ointments, creams, lotions, oils, as well as patches, adhesive plasters, depots, and reservoirs.

Formulations suitable for transdermal administration include gels, pastes, ointments, creams, lotions, and oils, as well as patches, adhesive plasters, bandages, dressings, depots, and reservoirs.

Tablets may be made by conventional means, e.g., compression or moulding, optionally with one or more accessory ingredients. Compressed tablets may be prepared by compressing in a suitable machine the compound in a free-flowing form such as a powder or granules, optionally mixed with one or more binders (e.g., povidone, gelatin, acacia, sorbitol, tragacanth, hydroxypropylmethyl cellulose); fillers or diluents (e.g., lactose, microcrystalline cellulose, calcium hydrogen phosphate); lubricants (e.g., magnesium stearate, talc, silica); disintegrants (e.g., sodium starch glycolate, cross-linked povidone, cross-linked sodium carboxymethyl cellulose); surface-active or dispersing or wetting agents (e.g., sodium lauryl sulfate); preservatives (e.g., methyl p-hydroxybenzoate, propyl p-hydroxybenzoate, sorbic acid); flavours, flavour enhancing agents, and sweeteners. Moulded tablets may be made by moulding in a suitable machine a mixture of the powdered compound moistened with an inert liquid diluent. The tablets may optionally be coated or scored and may be formulated so as to provide slow or controlled release of the compound therein using, for example, hydroxypropylmethyl cellulose in varying proportions to provide the desired release profile. Tablets may optionally be provided with a coating, for example, to affect release, for example an enteric coating, to provide release in parts of the gut other than the stomach.

Ointments are typically prepared from the compound and a paraffinic or a water-miscible ointment base.

Creams are typically prepared from the compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the compound through the skin or other affected areas. Examples of such dermal penetration enhancers include dimethylsulfoxide and related analogues.

Emulsions are typically prepared from the compound and an oily phase, which may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties, since the solubility of the compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for intranasal administration, where the carrier is a liquid, include, for example, nasal spray, nasal drops, or by aerosol administration by nebuliser, include aqueous or oily solutions of the compound. As an alternative method of administration, a dry powder delivery may be used as an alternative to nebulised aerosols.

Formulations suitable for intranasal administration, where the carrier is a solid, include, for example, those presented as a coarse powder having a particle size, for example, in the range of about 20 to about 500 microns which is administered in the manner in which snuff is taken, i.e., by rapid inhalation through the nasal passage from a container of the powder held close up to the nose.

Formulations suitable for pulmonary administration (e.g., by inhalation or insufflation therapy) include those presented as an aerosol spray from a pressurised pack, with the use of a suitable propellant, such as dichlorodifluoromethane, trichlorofluoromethane, dichoro-tetrafluoroethane, carbon dioxide, or other suitable gases. Additionally or alternatively, a formulaton for pulmonary administration may be formulated for administration from a nebuliser or a dry powder inhaler. For example, the formulation may be provided with carriers or liposomes to provide a suitable particle size to reach the appropriate parts of the lung, to aid delivery of an appropriate does to enhance retention in the lung tissue.

Formulations suitable for ocular administration include eye drops wherein the compound is dissolved or suspended in a suitable carrier, especially an aqueous solvent for the compound.

Formulations suitable for rectal administration may be presented as a suppository with a suitable base comprising, for example, natural or hardened oils, waxes, fats, semi-liquid or liquid polyols, for example, cocoa butter or a salicylate; or as a solution or suspension for treatment by enema.

Formulations suitable for vaginal administration may be presented as pessaries, tampons, creams, gels, pastes, foams or spray formulations containing in addition to the compound, such carriers as are known in the art to be appropriate.

Formulations suitable for parenteral administration (e.g., by injection), include aqueous or non-aqueous, isotonic, pyrogen-free, sterile liquids (e.g., solutions, suspensions), in which the compound is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the compound in the liquid is from about 1 ng/mL to about 100 µg/mL, for example from about 10 ng/mL to about 10 µg/mL, for example from about 10 ng/mL to about 1 µg/mL. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

Generally, the methods of the invention may comprise administering to a subject an effective amount of a compound of formula (III) so as to provide an antimicrobial effect. The compound of formula (III) may be administered at an amount sufficient to potentiate the activity of a second active agent. The second active agent is administered to a subject at an effective amount so as to provide an antimicrobial effect.

It will be appreciated by one of skill in the art that appropriate dosages of the compound of formula (III) or the active agent, and compositions comprising the compound of formula (III) or the active agent, can vary from patient to patient. Determining the optimal dosage will generally involve the balancing of the level of therapeutic benefit against any risk or deleterious side effects. The selected dosage level will depend on a variety of factors including, but not limited to, the activity of the particular compound of formula (III) or the active agent, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound of formula (III) or the active agent and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of a compound of formula (III) or the active agent is in the range of about 10 µg to about 250 mg (more typically about 100 µg to about 25 mg) per kilogram body weight of the subject per day. Where the compound of formula (III) or the active agent is a salt, an ester, an amide, a prodrug, or the like, the amount administered is calculated on the basis of the parent compound and so the actual weight to be used is increased proportionately.

### Kits

One aspect of the invention pertains to a kit comprising (a) a compound of formula (III), or a composition comprising a compound as defined in any one of formula (III), e.g., preferably provided in a suitable container and/or with suitable packaging; and (b) instructions for use, e.g., written instructions on how to administer the compound or composition.

The written instructions may also include a list of indications for which the compound of formula (III) is a suitable treatment.

In one embodiment, the kit further comprises (c) a second active agent, or a composition comprising the second active agent. Here, the written instructions may also include a list of indications for which the second active agent, together with the compound of formula (III), is suitable for treatment.

### Routes of Administration

A compound of formula (III), a second agent, or a pharmaceutical composition comprising the compound of formula (III) may be administered to a subject by any convenient route of administration, whether systemically/peripherally or topically (i.e., at the site of desired action).

Routes of administration include, but are not limited to, oral (e.g., by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### The Subject/Patient

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orang-utan, gibbon), or a human. Furthermore, the subject/patient may be any of its forms of development, for example, a foetus.

In one preferred embodiment, the subject/patient is a human.

It is also envisaged that the invention may be practised on a non-human animal having a microbial infection. A non-human mammal may be a rodent. Rodents include rats, mice, guinea pigs, chinchillas and other similarly-sized small rodents used in laboratory research.

### Methods of Preparation

Compounds of formula (III) can be prepared by conventional peptide synthesis, using methods known to those skilled in the art. Suitable methods include solution-phase synthesis such as described by Yamada et al, J. Peptide Res. 64, 2004, 43-50, or by solid-phase synthesis such as described by de Visser et al, J. Peptide Res, 61, 2003, 298-306, and Vaara et al, Antimicrob. Agents and Chemotherapy, 52, 2008. 3229-3236. These methods include a suitable protection strategy, and methods for the cyclisation step. Alternatively, compounds may be prepared from readily available polymyxins, for example by removal of the N-terminal amino acid of the polymyxin (residue 1). Such a method is described herein for the preparation of compounds based on residues 2-10 of polymyxins B and E.

As shown herein, it is possible to derivatise the N terminal group of a deacylated polymyxin compound, such as deacylated polymyxin B and deacylated polymyxin B nonapeptide, without derivatising the amino groups that are present in the side chains of the polymyxin compound. As described herein, the side chains of the polymyxin compound may be selectively protected without protecting the N terminal group. The N terminal group may then be reacted to provide the appropriate N terminal substituent. The side chain protection may subsequently be removed.

A protected Polymyxin can also be cleaved to the corresponding heptapeptide by cleavage of amino acids 1-3. Methods for this cleavage are described in WO 2012/168820, and WO 1988/00950. As shown herein this can be derivatised by coupling to appropriately substituted dipeptides or tripeptides to provide novel polymyxin derivatives.

### Other Preferences

Each and every compatible combination of the embodiments described above is explicitly disclosed herein, as if each and every combination was individually and explicitly recited.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described. Where technically appropriate embodiments may be combined and thus the disclosure extends to all permutations and combinations of the embodiments provided herein.

Certain aspects and embodiments of the invention will now be illustrated by way of example and with reference to the figures described above.

### Abbrevia tions

| **Abbreviation** | **Meaning** |
|---|---|
| PMBN | Polymyxin B nonapeptide |
| PMB | Polymyxin B |
| Thr | Threonine |
| Ser | Serine |
| DSer | D-serine |
| Leu | Leucine |
| Ile | Isoleucine |
| Phe | Phenylalanine |
| Dphe | D-phenylalanine |
| Val | Valine |
| Dab | α,γ-Diaminobutyric acid |
| DIPEA | *N*,*N*-diisopropylethylamine |
| BOC-ON | 2-(tert-Butoxycarbonyloxyimino)-2-phenylacetonitrile 1-ethyl-3-(3- |
| EDC | octaminopropyl)carbodiimide hydrochloride (Benzotriazol-1-yl- |
| PyBOP | oxy)tripyrrolidinophosphonium hexafluorophosphate 2-(7-aza-1H-benzotriazol-1-yl)- |
| HATU | 1,1-3,3-tetramethyluronium hexafluorophosphate |
| HOAt | 1-Hydroxy-7-azabenzotriazole |
| DCM | Dichloromethane |
| TFA | Trifluoroacetic acid |
| ND | Not determined |
| N/A | Not applicable |
| DMF | N,N-Dimethylformamide |
| PMBH | Polymyxin B heptapeptide (3-10) |
| PMBD | Polymyxin B decapepide |
| Pro | Proline |
| Dap | α,β-Diaminopropionic acid |
| Gly | Glycine |
| Thr | Threonine |
| His | Histidine |
| Phe | Phenylalanine |

### Examples

The following examples are provided solely to illustrate the present invention and are not intended to limit the scope of the invention, as described herein.

Nomenclature - Compounds are named based on the natural polymyxin core from which they are synthetically derived.

### Synthesis Examples

### Intermediate 1 - Polymyxin B nonapeptide

A mixture of EDTA (1.4 g), potassium chloride (1.1 g) and L-cysteine (0.12 g) was dissolved in water (475 mL) and potassium phosphate buffer (pH 7, 25 mL). The reaction was stirred at 37°C for 10 min then Polymyxin B (10.3 g) was added. After stirring for 2h at 37 °C papain (3.36 U/ mg) was added and stirred for a further 18h at 37 °C. The progress of the reaction was monitored by LC-MS using the conditions outlined in Table 1. The crude material was separated into 87 mL fractions and purified using 10 g SCX cartridge (× 6), eluting first with methanol (100 mL) and then 20% ammonia (aq, sp.g.880) in methanol (100 mL). The ammonia fractions were isolated and evaporated to give the product as beige solid, yield 4.95 g, 60% ***m*/*****z*** 482, [M+2H]²⁺.

**Table 1 - LC-MS conditions**

| | | | | | | |
|---|---|---|---|---|---|---|
| Micromass Platform | LC | | | | | |
| Column: | Zorbax 5µ C18 (2) 150 × 4.6 mm | | | | | |
| Mobile Phase A: | 10% Acetonitrile in 90% Water, 0.15 %TFA or 0.1 % formic | | | | | |
| Mobile Phase B: | 90% Acetonitrile in 10% Water, 0.15 %TFA or 0.1 % formic | | | | | |
| Flow rate: | 1 mL/min | | | | | |
| Gradient: | Time | 0 min | 100% | A | 0% | B |
| Time 10 min | 0% | A | 100% | B | | |
| Time 11 min | 0% | A | 100% | B | | |
| Time 11.2 min | 100% | A | 0% | B | | |
| Time 15 min | 100% | A | 0% | B | | |
| Cycle time 15 min | | | | | | |
| Injection volume: | 20 µL | | | | | |
| Detection: | 210 nm | | | | | |

### Intermediate 2 - Tetra-(Boc) Polymyxin B nonapeptide

Selective BOC protection of the free γ-amino groups on the Dab residues of polymyxin B nonapaptide was carried out using the procedure of H. O'Dowd et al, Tetrahedron Lett., 2007, 48, 2003-2005. Polymyxin B Nonapeptide (**Intermediate 1** 1.00 g, 1.0 mmol) was dissolved in water (4.4 mL), dioxane (4.4 mL), triethylamine (4.4 mL) and the mixture was stirred for 10 min prior to the addition of 2-(tert-butoxycarbonyloxyimino)-2-phenylacetonitrile (Boc-ON) (0.77 g; 3.11 mmol). After stirring for 18 h, a further addition of Boc-ON (0.1g, 0.4 mmol) was added and the mixture was stirred for a further 3 h. The progress of the reaction was followed by LC-MS, once complete the mixture was quenched by the addition of 20% methanolic ammonia (50 mL). The mixture was then evaporated to dryness and re-dissolved in methanol which was subsequently loaded onto silica. The crude material was purified using chromatography (eluent 0-20% methanol in dichloromethane) on silica gel (40 g) to afford tetra-(Boc) polymyxin B nonapeptide as a white solid (0.5 g, 36 %). TLC, R_{f} 0.2 (10% methanol in dichloromethane). m/z 1362.8[MH]⁺.

### Intermediate 3 - Colistin (Polymyxin E) nonapeptide

Colistin (polymyxin E, 5 g) was treated with immobilised papain (185 ELU/g), potassium phosphate buffer (25 mM; pH 7, 1.25 L), potassium chloride (30 mM), EDTA (10 mM) and cysteine (1 mM) at 37°C for 32 h with gentle agitation to produce colistin (polymyxin E) nonapeptide. The progress of the reaction was monitored by LC-MS using the conditions outlined in Intermediate 1, Table 1. The immobilized papain was removed by filtration and the filtrate was concentrated *in vacuo* to leave a solid residue which was re-suspended in 10% aqueous methanol and left at room temperature overnight. The supernatant was decanted and concentrated *in vacuo.* Colistin (Polymyxin E) nonapeptide was purified from the residue by SPE on C18 silica (10 gm), eluting with 0-25% aqueous methanol. Evaporation of the appropriate fractions gave the product as a white solid. *m*/*z* 465.32 [M+2H]²⁺.

### Intermediate 4 - Tetra-(Boc) Colistin (Polymyxin E) nonapeptide

Colistin (Polymyxin E) Nonapeptide (2.5 g, 2.69 mmol) was suspended in water (35 mL) with sonication. Dioxane (35 mL) and triethylamine (35ml) were added and the mixture was cooled in ice for 10 min prior to the addition of 2-(tert-butoxycarbonyloxyimino)-2-phenylacetonitrile (Boc-ON) (2.65 g; 10.76 mmol). The progress of the reaction was followed by LC-MS and reached completion after 10 minutes, whereupon the mixture was quenched by addition of 20% methanolic ammonia (25 mL). The liquid phase was decanted and the residual solid was re-dissolved in water and extracted sequentially with dichloromethane and iso-butanol. Based on LC-MS analysis, the decanted liquid and both dichloromethane and iso-butanol extracts were pooled together followed by concentration *in vacuo* to give yellow gum which was loaded onto flash chromatography (Si 60A- 35-70). The column was eluted with 0-20% methanol (containing 2% ammonia) in dichloromethane. The column fractions eluted with 7-10% methanol (containing 2% ammonia) in dichloromethane afforded tetra-(Boc) colistin (polymyxin E) nonapeptide as a white solid (1.18 g, 33 %). *m*/*z* 1329.7 [M+H]+.

### Intermediate 5 - Tri-(Boc) Polymyxin B heptapeptide

PMB sulphate (2 g) was dissolved in water (20 mL) followed by addition of 1,4 dioxane (40 mL) and left to stir for 10 minutes at room temperature. To the reaction mixture was added Boc anhydride (4.42 g) was added as solid and the reaction was stirred at room temperature and was monitored by HPLC. The reaction mixture was then adjusted to pH 6 using 1 M HCl , the precipitate which formed was filtered and washed with water (50 mL) and heptane (50 mL), to leave B_{OC5}PMB as a white solid (2.4 g, 85 %). This material (1 g) was dissolved in 1,4-butanediol (112.5 mL) and the mixture was stirred at 40°C overnight. To the solution potassium phosphate (75 mL, 0.12 5M pH 8.0) was added over one minute, causing the formation of a white suspension. The reaction was diluted by adding 112.5 mL butanediol and 75 mL potassium phosphate (0.125 M pH 8.0), but the white emulsion persisted. The temperature of the reaction was reduced to 37°C and then Savinase 16L (250 µL) was added and the reaction was stirred at room temperature overnight. As the reaction progressed the white emulsion cleared to form a transparent solution due to the formation of the more soluble PMBH-B_{OC3}. The reaction mixture was diluted with water (50ml) and was then extracted with DCM (100 mL) The DCM layer was collected and evaporated *in vacuo* to afford a colourless oil. The resulting oil was diluted in 50 % methanol (aq.) and was loaded onto four preconditioned 10 g Varian Bond Elut SCX cartridges and the flow through was collected. The cartridges were washed with two column volumes of 50 % methanol (aq.) and then PMBH-B_{OC3} was eluted from the column using two column volumes of 20% ammonia in methanol. The resulting eluent was evaporated to dryness *in vacuo* to afford purified PMBH-B_{OC3} (610 mg). *m*/*z* 1062.6 [M+H]⁺.

### Intermediate 7 - Thr(O-^{t}Bu) Tetra-(N-Boc) Polymyxin B nonapeptide

### Step 1 - (S)-2-((S)-2-Benzyloxycarbonylamino-3-tert-butoxy-butyrylamino)-4-tert-butoxycarbonylamino-butyric acid methyl ester

To a stirred suspension of (S)-2-Benzyloxycarbonylamino-3-tert-butoxy-butyric acid DHCA salt (3.65 g, 7.4 mmol) and (S)-2-Amino-4-tert-butoxycarbonylamino-butyric acid methyl ester HCI salt (2.0 g, 7.4 mmol) in a mixture of DCM (60 mL) and DMF (120 mL) was added N,N-diisopropylethylamine (3.85 mL, 22.1 mmol). To this stirred mixture was added 1-hydroxy-7-azabenzotriazole (1.0 g, 7.3 mmol) followed by N-(3-dimethylaminopropyl)-N'-ethylcarbodiimide HCI salt (1.42 g, 7.4 mmol). The mixture was stirred for 17 h at ambient temperature then filtered under suction to remove the insoluble by-product, which was discarded. The filtrate was concentrated to a yellow oil which was partitioned between a solvent mixture of EtOAc/Et₂O (1:1) (250 mL) and 0.5 M hydrochloric acid (200 mL). The aqueous phase was re-extracted with fresh solvent mixture (100 mL) and the combined organic extracts were successively washed with water (150 mL) and sat. NaHCO₃ solution (150 mL), dried (Na₂SO₄) and concentrated to a colourless oil (3.72g). This oil was purified by silica gel chromatography on a 100g SepPak cartridge, eluting with a solvent gradient of EtOAc/i-hexane (0-70%). Fractions containing the product (R_{f} 0.26 in EtOAc/i-hexane 3:7, visualized with KMnO₄ spray) were pooled and concentrated to give the title compounds as a colourless foam (3.58 g, 6.8 mmol, 92% yield). m/z 524 (MH⁺, 100%).

### Step 2 - (S)-2-((S)-2-Benzyloxycarbonylamino-3-tert-butoxy-butyrylamino)-4-tert-butoxycarbonylamino-butyric acid

A solution of lithium hydroxide monohydrate (0.861 g, 20.5 mmol) in water (16 mL) was added to a stirred solution of (S)-2-((S)-2-Benzyloxycarbonylamino-3-tert-butoxy-butyrylamino)-4-tert-butoxycarbonylamino-butyric acid methyl ester (3.58 g, 6.8 mmol) in methanol (64 mL) at ambient temperature and stirred for 19 h. To this solution was added 1M HCI (24 mL) resulting in a milky mixture (pH 1) which was quickly extracted with DCM (3 × 135 mL). The combined organic extract was dried (Na₂SO₄) and concentrated to give the title compound as a colourless foam (3.27 g, 6.4 mmol, 94% yield).M/z 532 [MNa]+, 1041 [2M+Na]+.

### Step 3 - CbzHNPMBN(OBu)(Boc)₄

(S)-2-((S)-2-Benzyloxycarbonylamino-3-tert-butoxy-butyrylamino)-4-tert-butoxycarbonylamino-butyric acid (1.73 g, 3.39 mmol) and **Intermediate 5** (3.0 g, 2.8 mmol) were charged to a flask to which dry DCM (85 ml) and dry DMF (17 mL) were added with stirring. To the stirred solution was added *N*,*N*-diisopropylethylamine (1.46ml, 8.4mmol) and after stirring for 5 min., O-(7-azabenzotriazol-1-yl)-N,N,N'N'-tetramethyluronium hexafluorophosphate (1.29 g, 3.39 mmol) was added in a single portion. The mixture was sonicated for 2 minutes then left to stir at ambient temperature for 18 h. The reaction mixture was then evaporated and the residue re-evaporated from toluene (3 × 100 mL). The residue was dried under vacuum for 3 h to ensure removal of toluene. Water (50ml) was added to this material and the mixture was rapidly stirred for 3 h with occasional sonication. The title compound was collected by suction filtration as a fine, white solid and washed with water (2 × 25 mL) then dried under vacuum for 15h (4.6 g, 3.0 mmol, 100% yield). m/z 1554[MH+].

### Step 4 - Title Compound

The product from step 3 (5.41 g, 3.48 mmol), ammonium formate (6.6 g, 104.4 mmol) and 10% Pd-C (2.0 g) were charged to a flask under N₂. MeOH (270 mL) was added and the mixture was stirred under N₂ for 4.5h. LCMS showed MH⁺ for product and loss of starting material. The mixture was filtered under suction through a pad of celite and washed through with MeOH (50 mL). The filtrate and washings were evaporated to a colourless oil which was partitioned between a solvent mixture of EtOAc/MeOH (4:1)(250 mL) and water (250 mL). The aqueous phase was further extracted with the same, fresh solvent mixture (2 × 100 mL). The combined organic extracts were dried (Na₂SO₄) and evaporated to a colourless oil (~ 6g). This material was purified by chromatography on silica gel (100 g SepPak column) eluting with a gradient of MeOH/EtOAc (0-4%). Fractions containing the product (R_{f} 0.30 in EtOAc/MeOH/NH₄OH ₈₈₀ 95:5:1, visualized with KMnO₄ spray) were pooled and evaporated to give the title compound as a crispy foam (4.0g, 2.8mmol, 81 % yield). m/z 1420 [MH+].

### Intermediate 9 - Tri-(BOC)-Polymyxin E heptapeptide

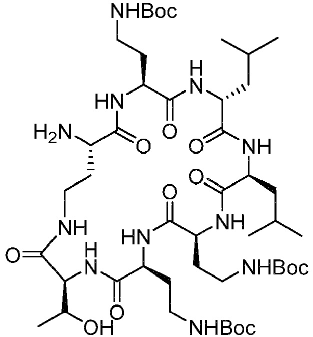

Colistin sulphate (5.0 g, 3.95 mmol) was dissolved in acetonitrile (50 mL) and water (25 mL) and stirred at room temperature for 10 minutes. Triethylamine (3.2 mL, 23.0 mmol) was added and the mixture stirred for a further 10 minutes. Di-*tert*-butyl dicarbonate (5.0 g, 23.0 mmol) was subsequently added in one portion and the mixture stirred for 16 hours. Savinase (15 mL) was then added, followed by 4 M sodium hydroxide solution (0.5 mL) and the reaction mixture stirred at room temperature for 5 days. The mixture was diluted with ethyl acetate and water. After separation of the layers, the organic phase was washed with 0.1M sodium hydroxide solution (x2), then water. The organic layer was dried over magnesium sulphate, filtered and the solvent evaporated at reduced pressure. The residue was purified by silica gel chromatography eluting with 0 - 100% (80:20:2 EtOAc:MeOH:880NH₃) in ethyl acetate to yield the title compound (2.28g, 56%). **m/z** 1028, [MH]⁺

### Intermediate 10 - Thr(O-^{t}Bu) Tetra-(N-Boc) Polymyxin E nonapeptide

The title compound was prepared from *(S*)-2-((*S*)-2-Benzyloxycarbonylamino-3-tert-butoxy-butyrylamino)-4-tert-butoxycarbonylamino-butyric acid and **Intermediate 9** according to the method for **Intermediate 7** steps 3 and 4. **m/z** 1385, [MH]⁺

### Intermediate 11 - Tetra- (N-Boc)-L-Thr(O-^{t}Bu)-L-Dap-Polymyxin B heptapeptide

The title compound was prepared from (S)-2-((S)-2-Benzyloxycarbonylamino-3-tert-butoxy-butyrylamino)-3-tert-butoxycarbonylamino-propionic acid and **Intermediate 5** according to the method for **Intermediate 7** steps 3 and 4. **m/z** 1405, [MH]⁺

### Intermediate 12 - Tetra- (N-Boc)-L-Ser (O-^{t}Bu)-L-Dap-Polymyxin B heptapeptide

The title compound was prepared from(S)-2-((S)-2-(((benzyloxy)carbonyl)amino)-3-(tert-butoxy)propanamido)-3-((tert-butoxycarbonyl)amino)propanoic acid and **Intermediate 5** according to the method for **Intermediate 7** steps 3 and 4. **m/z** 1391 [MH]⁺

### Intermediate 13 -Tetra- (N-Boc)-L-Thr(O-^{t}Bu)-D-Ser-Polymyxin B heptapeptide

The title compound was prepared from N-(N-((benzyloxy)carbonyl)-O-(tert-butyl)-L-threonyl)-O-(tert-butyl)-D-serine and **Intermediate 5** according to the method for **Intermediate 7** steps 3 and 4. **m/z** 1362 [MH]⁺

### Intermediate 14 - Tetra- (N-Boc)-L-Thr(O-^{t}Bu)-L-Dap-Polymyxin E heptapeptide

The title compound was prepared from (S)-2-((S)-2-Benzyloxycarbonylamino-3-tert-butoxy-butyrylamino)-3-tert-butoxycarbonylamino-propionic acid and Intermediate 9 according to the method for **Intermediate 7** steps 3 and 4. **m/z** 1372, [MH]⁺

### Intermediate 15 - Tetra- (N-Boc)-L-Thr(O-^{t}Bu)-D-Dap-Polymyxin B heptapeptide

The title compound was prepared from (R)-2-((S)-2-Benzyloxycarbonylamino-3-tert-butoxy-butyrylamino)-3-tert-butoxycarbonylamino-propionic acid and Intermediate 5 according to the method for **Intermediate 7** steps 3 and 4. **m/z** 1406, [MH]⁺

### Intermediate 16 - (S)-4-(Boc)amino-2-hydroxybutanoyl Thr(O-^{t}Bu) Tetra-(N-Boc) polymyxin B nonapeptide [Thr-10(O-triethylsilyl)]

### Step 1

Intermediate 7 (Thr(O-^{t}Bu) Tetra-(N-Boc) Polymyxin B nonapeptide) (250 mg, 0.18 mmol) was dissolved in dichloromethane (2 mL), treated with 2,6-lutidine (1 mL) and cooled to 0°C under a nitrogen atmosphere. The solution was treated dropwise with (triethylsilyl)trifluoromethane sulphonate (0.26 mL, 3.3 equiv), then allowed to warm to room temperature overnight. The solution was quenched with saturated aqueous ammonium chloride solution (10 mL) and the product extracted into diethyl ether (2 × 20mL). The organic extracts were combined, dried over anhydrous magnesium sulphate and evaporated under reduced pressure. The residue was chromatographed on silica eluting with 0-20% (10%ammonia in methanol) in dichloromethane. Product-containing fractions were combined and evaporated to a white solid (117 mg, 43%). 400MHz ¹H nmr (CDCl₃)(*inter alia*) 0.46 (6H, q, SiCH₂),0.80-0.88 (12H, m, incl t, SiCH₂CH₃), 1.12 (9H, s, O^{t}Bu), 1.34-1,40 (36H, m, BOC).

### Step 2

The triethyl silyl protected nonapeptide from Step 1 (117 mg) was reacted with (S)-4-((tert-butoxycarbonyl)amino)-2-hydroxybutanoic acid (33 mg) under the conditions described for coupling method 2A step 1 to afford the Title compound (95 mg, 71%). 400 MHz ¹H nmr (CDCl₃)(*inter alia*) 0.48 (6H, q, SiCH₂),0.80-0.88 (12H, m, incl t, SiCH₂CH₃), 1.22 (9H, s, O^{t}Bu), 1.36-1,42 (36H, m, BOC) .

### Method 1 - General method of preparation of nonapeptide amide derivatives

Step 1. The corresponding carboxylic acid (5 equiv with respect to the polymyxin substrate) was dissolved in dichloromethane (2 mL/mmol). *N*,*N*-Diisopropylethyalmine (5.0 equiv.) and 2-(1H-7-Azabenzotriazol-1-yl)-1,1,3,3-tetramethyl uronium hexafluorophosphate (HATU) (5.0 equiv ) were then added to the reaction mixture. After 30 min stirring at room temperature compound of **intermediate 2** or **intermediate 4** (1.0 equiv.) was added. After 16 h the completion of the reaction was confirmed by LC-MS and the reaction mixture was evaporated to dryness and purified using column chromatography on silica gel (eluent 0-10% methanol in dichloromethane). The appropriate fractions were concentrated to leave the product as a colourless oil (typical yield 58 %).

Step 2. The product from Step 1 was dissolved in dichloromethane (20 mL/mmol). Trifluoroacetic acid (60 equiv.) was added and the mixture was stirred at room temperature for 16 h, after which time LC-MS confirmed completion of the reaction. The reaction mixture was concentrated *in vacuo* to leave the trifluoroacetate salt as a colourless oil. To this was added water (10 mL/mmol) and the mixture was sonicated for 5 min. To the resulting suspension was added 1 M NaHCO₃ until the mixture reached pH 9. The mixture was then passed through a 10 g C18 SPE column, eluting sequentially with 0, 40, 50, 60, 70, 80 and 100 % aqueous methanol. Product-containing fractions were pooled and evaporated. The residue was suspended in water and 0.1 M H₂SO₄ added until pH 7 was reached. The solution was lyophilised overnight to afford the sulphate salt as a white solid. Compound purity was assessed by HPLC using the conditions outlined in Table 2.

**Table 2 - Analytical HPLC conditions**

| | | | | | | |
|---|---|---|---|---|---|---|
| Column: | Zorbax 5µ C18 (2) 150 × 4.6 mm | | | | | |
| Mobile Phase A: | 10% Acetonitrile in 90% Water, 0.15 %TFA or 0.1 % Formic acid | | | | | |
| Mobile Phase B: | 90% Acetonitrile in 10% Water, 0.15 %TFA or 0.1 % Formic acid | | | | | |
| Flow rate: | 1 mL/min | | | | | |
| Gradient: | Time | 0 min | 100% | A | 0% | B |
| Time 10 min | 0% | A | 100% | B | | |
| Time 11 min | 0% | A | 100% | B | | |
| Time 11.2 min Cycle time 15 min | 100% | A | 0% | B | | |
| Injection volume: | 20 µL | | | | | |
| Detection: | 210 nm | | | | | |

### Method 2 - General method of preparation of nonapeptide amides

Step 1 - The BOC protected nonapeptide was prepared using the conditions of Method 1. After completion if the reaction, the crude reaction mixture was adsorbed onto silica and chromatographed on a silica cartridge, eluting with 0-20% methanol in dichloromethane. Product-containing fractions were combined and evaporated to a white foam. The product thus obtained was re-purified by silica gel chromatography to obtain the product as a white foam.

Step 2 - The purified product from Step 2 was dissolved in dichloromethane (2 mL), treated with TFA (1 mL) and the mixture stirred at room temperature for 1 hour. The solvent was evaporated and the residue azeotroped with toluene, to leave a white solid. This was dissolved in water (10 mL) and washed with dichloromethane (5 mL). The aqueous phase was evaporated to low volume and lyophilised overnight to afford the TFA salt of the product as a white solid.

### Method 2A - Further General method of preparation of nonapeptide amides

Step 1 - The protected polymyxin substrate (0.07 mmol) was dissolved in dichloromethane (4 mL), and treated with the corresponding carboxylic acid (1.5 equiv with respect to the polymyxin substrate), *N*,*N*-Diisopropylethyalmine (3.0 equiv.), followed by HATU (2.0 equivalent). After 16 h the completion of the reaction was confirmed by LC-MS and the reaction mixture was evaporated to dryness. Water (~10 mL) was added and the mixture triturated then stirred vigorously for 1 h. The resultant precipitate was collected by filtration and dried *in vacuo* overnight.

Step 2 - The Boc-protected derivative from Step 1 was dissolved in dichloromethane (3 mL) and treated with TFA (1 mL). The reaction mixture was stirred at room temperature until

LCMS confirmed complete deprotection. The solvent was evaporated and the residue chromatogaphed by prep HPLC using the conditions of Method 3, step 6. Product-containing fractions were combined, evaporated to low volume, and lyophilised to afford the product as the TFA salt.

### Method 3 - General method for the preparation of dipeptide amide derivatives of polymyxin B heptapeptide

### Step 1 - Coupling of carboxylic acids to methyl esters of amino acid 1

The appropriate carboxylic acid (1.1 equiv.), the appropriate (N-Boc or OBu*^{t}*) amino acid methyl ester hydrochloride (1 equiv.), EDC hydrochloride (1.1 equivs.) and HOAt (1.1 equiv.) were charged to a flask. DCM (8 mL/mmol with respect to the amino acid methyl ester) was added and to the stirred mixture under nitrogen was added DIPEA (3 equiv.) to give a yellow solution. The solution was stirred for 18 h, diluted with an equal volume of DCM and the solution washed successively with water (16 mL/mmol with respect to amino acid) and sodium hydrogen carbonate solution (16 mL/mmol). The solution was dried (Na₂SO₄) and evaporated to a residue. The residue was purified by chromatography on silica gel (gradient elution with EtOAc/iso-hexane). Relevant fractions were pooled and evaporated to afford the desired methyl ester product *(m*/*z* [M+H]⁺ detectable in the LCMS spectrum). Where a racemic acid is used, the product is obtained as a mixture of diastereoisomers.

### Step 2 - Hydrolysis of the methyl ester product from step 1

To a stirred solution of the product from step 1 (1 equiv.) in methanol (5 mL/mmol with respect to the methyl ester) was added a solution of lithium hydroxide monohydrate (3 equiv.) in water (0.5 mL/mmol of reagent). The resulting solution was stirred at ambient temperature for 24 h then poured into water (25 mL/mmol with respect to methyl ester). This solution was adjusted to pH 1 by the addition of 1 M hydrochloric acid (3 equiv.) and the mixture was extracted with DCM (3 x). The combined organic extracts were dried (Na₂SO₄) and evaporated to afford the desired carboxylic acid *(m*/*z* [M+H]⁺ detectable in the LCMS spectrum) . Where a racemic acid is used in step 1, the product is obtained as a mixture of diastereoisomers.

### Step 3: Coupling of the carboxylic acid product from step 2 with the methyl ester of amino acid 2

This step was carried out in the same manner as that described in step 1, using the carboxylic acid from step 2 and the appropriate (N-Boc or OBu*^{t}*) amino acid methyl ester hydrochloride. The methyl ester product *(m*/*z* [M+H]⁺ detectable in the LCMS spectrum) was isolated as described in step 1. Where a racemic acid was used in step 1, the product is obtained as a mixture of diastereoisomers.

### Step 4 - Hydrolysis of the methyl ester product from step 3

This step was carried out in the same manner as that described in step 2, using the methyl ester from step 3. The carboxylic acid product *(m*/*z* [M+H]⁺ detectable in the LCMS spectrum) was isolated as a mixture of diastereoisomers.

### Step 5 - Coupling of the carboxylic acid product from step 4 with Tri-(Boc) Polymyxin B heptapeptide (intermediate 5)

PyBoP (2 equiv.) was added to a stirred solution of the carboxylic acid from step 4 (2 equiv.) in dry DCM (15 mL/mmol with respect to acid). DIPEA (2 equiv.) was then added and the solution stirred for 30 min. To this solution was then added a solution of **Intermediate 5** (1 equiv.) in dry DCM (12 mL/mmol with respect to acid) and dry DMF (1.5 mL/mmol with respect to acid) and the whole mixture was stirred for 16 h. The mixture was then evaporated to a thick oil which was partitioned between EtOAc and water. The organic phase was washed with saturated sodium hydrogen carbonate solution then brine, dried (Na₂SO₄) and evaporated to a foam. The material was purified by chromatography on silica gel (gradient elution with MeOH/EtOAc) to afford the polypeptide product *(m*/*z* [M+H]⁺ detectable in the LCMS spectrum). Where a racemic acid was used in step 1, the product is obtained as a mixture of diastereoisomers.

### Step 6 - Deprotection of the Polymyxin B heptapeptide product from step 5

TFA (30 mL/mmol with respect to polypeptide) was added to a stirred solution of the polypeptide from step 5 in DCM (60 mL/mmol). The solution was stirred for 3.5 h then evaporated and dried under vacuum for 1 h. The residue was purified by HPLC (conditions below) and lyophilised to afford the TFA salt of the product as a white solid. Where a racemic acid was used in step 1, the product is obtained as a mixture of diastereoisomers. (See Table 4 for examples).

**Table 3 - Prep HPLC conditions**

| | | | | | | |
|---|---|---|---|---|---|---|
| Column: | Sunfire C18 OBD 5µm × 30mm × 150mm | | | | | |
| Mobile Phase A: | Acetonitrile + 0.15 %TFA | | | | | |
| Mobile Phase B: | water + 0.15 %TFA | | | | | |
| Flow rate: | 25 mL/min | | | | | |
| Gradient: | Time | 0 min | 3% | A | 97% | B |
| Time 2 min | 3% | A | 97% | B | | |
| Time 25 min | 40% | A | 60% | B | | |
| Time 30 min | 97% | A | 3% | B | | |
| Time 32 min | 97% | A | 3% | B | | |
| Detection: | 210 nm | | | | | |

### Method 3A

The coupling was carried out as described in Method 3, using a CBZ-protected amino acid at Step 3. An additional CBZ deprotection step (Step 5A) was included prior to step 6.

Step 5A - CBZ Deprotection:
A mixture of the protected intermediate from Step 5 (0.0573mmol) and 10% Pd/C paste (10 mg) in ethanol (4 mL) was stirred under an atmosphere of hydrogen for 18 h. Further 10% Pd/C paste (10 mg) was added and stirring was continued for a further 24 h. The reaction mixture was filtered through a pad of celite and the filtercake was washed with ethanol (2 x). The combined organics were evaporated to afford a crude oil. This was purified by reverse phase preparatory HPLC using the conditions of Method 3 step 6 to afford the desired product as a colourless glass (20%). *(m*/*z* [M+H]⁺ detectable in the LCMS spectrum).

### Method 3B

Method 3B consists of steps 1-2 of Method 3A followed by coupling to BOC-protected PMBN **(Intermediate 2**) to give the protected decapeptide. Deprotection following Method 3A, Step 5A for CBZ deprotection and Step 6 for Boc deprotection affords the desired compound.

### General Preparation of Acetate Salts.

A TFA salt (50 mg) was dissolved in water, and taken to pH 9 with 1 M NaHCO₃. The mixture was then passed through a 1 g C18 SPE column, eluting with water (20 mL) followed by 80% methanol/water. Product-containing fractions were pooled treated with 0.1 M acetic acid (10 equiv.). The solution was concentrated under reduced pressure, then lyophilised overnight to afford the acetate salt as a white solid. Compound purity was assessed by HPLC using the conditions outlined in Table 2.

### Alternative Preparation of Acetate Salts

A TFA salt (20 mg) was dissolved in water (0.5 mg) and applied to a column of AG1-X2 resin (BioRad) (1 g, acetate form, pre-washed with 10% acetic acid in water, followed by 1% acetic acid in water). The column was eluted with water (8 mL) and the eluant concentrated under reduced pressure, then lyophilised overnight to afford the acetate salt as a white solid (14 mg). Compound purity was assessed by ¹H NMR and HPLC using the conditions outlined in Table 2.

### Synthesis of Carboxylic Acids

Carboxylic acids used for the assembly of polymyxin derivatives were secured either via commercial sources, or prepared using methods known to those skilled in the art. Experimental details of the following carboxylic acids serve as representative examples for the synthesis of similar acid intermediates used in the synthesis of the compounds of the present invention.

*Trans-1-tert-butoxycarbonyl-5-octyl-piperidine-3-carboxylic acid (relative stereochemistry)*

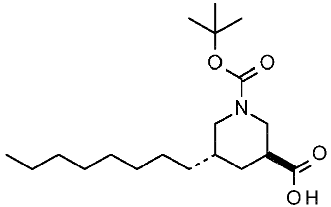

### (i) - Ethyl 5-oct-1-ynylpyridine-3-carboxylate

To a solution of ethyl-5-bromonicotinate (1.15 g, 5.0 mmol) in ethyl acetate (20ml) under nitrogen was added triethylamine (1.1 mL, 7.5 mmol), 1-octyne (1.1 mL, 7.5 mmol), bis(triphenylphosphine)palladium (II) dichloride (176 mg, 0.25 mmol) and copper iodide (10 mg, 0.05 mmol). The reaction mixture was stirred at 50°C for 16 hours, then filtered under suction through Celite and washed through with ethyl acetate. The filtrate was evaporated at reduced pressure. The residue was purified by silica gel chromatography eluting with 0 - 50% ethyl acetate in iso-hexane to yield the title compound (1.20 g, 93%), m/z 260 (MH+), C16H21NO2 exact mass 259.157.

### (ii) - Ethyl 5-octylpiperidine-3-carboxylate

To a solution of ethyl 5-oct-1-ynylpyridine-3-carboxylate (1.20 g, 4.60 mmol) in acetic acid (100 mL) was added platinum oxide (100 mg). The reaction mixture was hydrogenated for 16 h., then filtered under suction through Celite and washed through with ethyl acetate. The filtrate was evaporated at reduced pressure and then the residue was partitioned between ethyl acetate and water. The pH of the aqueous layer was adjusted to pH10 by the addition of .880 ammonia. After separation of the layers, the aqueous phase was re-extracted with ethyl acetate and then the combined organic layers were passed through a hydrophobic frit. The solvent was evaporated at reduced pressure and the residue purified by silica gel chromatography eluting with 0 - 100% (80:20:2 ethyl acetate:methanol:.880 ammonia) in ethyl acetate gave ethyl 5-octylpiperidine-3-carboxylate (876 mg, 71%). m/z 270 (MH+), C16H31NO2 exact mass 269.235.

### (iii) - Cis-O1-tert-butyl O3-ethyl 5-octylpiperidine-1,3-dicarboxylate and trans-O1-tert-butyl O3-ethyl 5-octylpiperidine-1,3-dicarboxylate (relative stereochemistry)

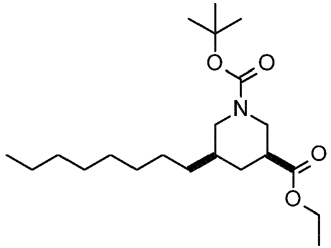

and

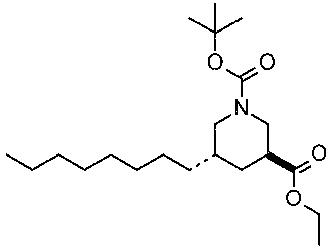

To a solution of ethyl 5-octylpiperidine-3-carboxylate (870mg, 3.20mmol) in dichloromethane (50 mL) was added triethylamine (680 µL, 4.8 mmol), followed by di-tert-butyl dicarbonate (1.06 g, 4.8 mmol). The reaction mixture was stirred at room temperature for 16 hours and then concentrated at reduced pressure. The residue was dissolved in diethyl ether and washed with ammonium chloride solution. After separation of the layers, the aqueous phase was re-extracted with diethyl ether. The combined organic phases were dried (MgSO4), filtered and concentrated at reduced pressure. The products were purified by silica gel chromatography eluting with 0-30% diethyl ether in iso-hexane. The first product to elute was assigned cis stereochemistry (470 mg, 40%) {when compared to the cyclohexyl analogue in Syn Comm, 2008, 38, 2799}. m/z 314 (M-tBu) +. Further elution of the column gave the trans derivative (333mg, 28%). m/z 314 (M-tBu) +.

### (iv) - Trans-1-tert-butoxycarbonyl-5-octyl-piperidine-3-carboxylic acid (relative stereochemistry)

To a solution of trans -O1-tert-butyl O3-ethyl 5-octylpiperidine-1,3-dicarboxylate (330 mg, 0.89 mmol) in dioxane (5 mL) and water (2 mL) was added lithium hydroxide monohydrate (76 mg, 1.80 mmol). The reaction mixture was stirred at room temperature for 16 hours and then treated with a further quantity of lithium hydroxide monohydrate (100 mg) for 2 days. The reaction mixture was concentrated at reduced pressure and the residue partitioned between ethyl acetate and water. The aqueous phase was acidified by the addition of 1M hydrochloric acid and the product extracted into ethyl acetate. The organic phase was passed through a hydrophobic frit and the solvent evaporated at reduced pressure to yield the title compound (275 mg, 91%). m/z 342 (MH+), C19H35NO4 exact mass 341.257.

*2-(2-Tert-butoxycarbonylamino-ethyl)-undecanoic acid*

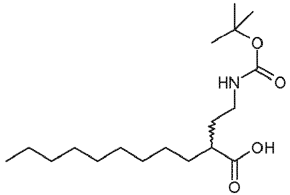

### (i) - 2-Cyanomethyl - undecanoic acid

THF (30 mL) was added to a stirred solution of n-butyl lithium (2.5 M in hexane, 9.4 mL, 23.5 mmol) at -78°C. To this solution was added diethylamine (0.5 mL, 4.8 mmol) over 2 minutes. The solution was warmed to 0°C for 15 minutes then recooled to -78°C. A solution of undecanoic acid (2.0 g, 10.7 mmol) in THF 20ml) was added over 20 minutes. The resulting mixture was warmed to 0°C for 30 minutes to give a solution. The solution was recooled to -78°C and then to this stirred mixture was added a solution of bromoacetonitrile (0.75 mL, 10.7 mmol) in THF (10 mL) over 25 minutes. The mixture was stirred at -78°C for 30 minutes then warmed to ambient temperature and left to stir for 21h. After this time, the mixture was quenched with the slow addition of water (250 mL) to give a mixture with pH 14. The mixture was extracted with diethyl ether (2 × 150 mL), adding some brine to minimize emulsions. These extracts were discarded. The aqueous phase from the extraction was acidified to pH 1 with conc hydrochloric acid (approx. 1.5 mL) and extracted with ethyl acetate (2 × 150 mL). The emulsion formed during the extraction was clarified by filtration through Celite. The organic extracts were dried (MgSO₄) and evaporated *in vacuo* to a brown oil. The oil was purified by column chromatography over silica gel eluting with a gradient of acetone/toluene to give the title product as an off-white solid (0.36 g, 1.6 mmol, 15%), *m*/*z* 226 (MH⁺). C₁₃H₂₃NO₂ exact mass 225.17.

### (ii) - 2-(2-Tert-butoxycarbonylamino-ethyl)-undecanoic acid

Nickel chloride hexahydrate (609 mg, 2.6 mmol) was added to a stirred solution of 2-cyanomethyl - undecanoic acid (577 mg, 2.6 mmol) in methanol (50 mL) at ambient temperature to give a solution. To this solution under N₂, cooled in an ice bath, was added sodium borohydride (688 mg, 18.2 mmol) in portions over 10 minutes. The black mixture was stirred at ambient temperature for 16h then filtered under suction through Celite and washed through with methanol (2 × 15mL). The filtrate and washings were evaporated to a white solid (2.6 g) which was stirred for 10 minutes with a mixture of dichloromethane/methanol (3:1) (25 mL) and re-filtered. The filtrate was evaporated to give a white solid which was dried under vacuum for 1h (1.89 g). This solid was re-dissolved in methanol (30 ml) and to the stirred solution was added triethylamine (0.67 mL, 4.8 mmol) and then a solution of di-tert butyl dicarbonate (590 mg, 2.7 mmol) in methanol (5 mL) and the whole mixture was stirred for 16h. After this time the mixture was evaporated and the residue re-dissolved in water (40ml) to give a solution with pH 11. The solution was extracted with diethyl ether (30ml) and the extract discarded. The aqueous phase was acidified to pH 4 by addition of 1M hydrochloric acid (approx.. 9.5 mL) and extracted with ethyl acetate (2 × 30 mL) and dichloromethane (30ml). The combined extracts were dried (Na₂SO₄) and evaporated to an oil. This material was purified by column chromatography over silica gel eluting with a gradient of diethyl ether/ iso-hexane to give the title compound as a colourless oil (309mg, 0.94 mmol, 36%), *m*/*z* 330 (MH⁺). C₁₈H₃₅NO₄ exact mass 329.26.

### (S)-4-Octyl-piperazine-1,3-dicarboxylic acid 1-tert-butyl ester

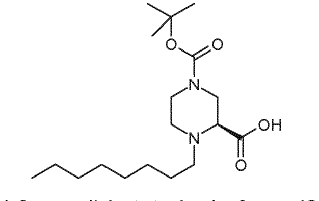

A solution of octanal (0.68 mL, 4.3 mmol) in tetrahydrofuran (2 mL) was added to a stirred solution of (S)-piperazine-1,3-dicarboxylic acid 1-tert-butyl ester (1.0 g, 4.3 mmol) in tetrahydrofuran (115 mL). Acetic acid (0.9 mL, 15.8 mmol) was added to the solution. After stirring the reaction mixture for 30 minutes, sodium triacetoxyborohydride (1.37 g, 6.5 mmol) was added in portions over 5 minutes and the mixture stirred for 18h. The mixture was then filtered through Celite and the filtrate evaporated to an oil (1.47 g). The oil was stirred with diethyl ether (20 mL) and the insoluble solid was filtered off and purified by column chromatography over silica gel, eluting with a gradient of methanol/ethyl acetate to give the title compound as a white solid (287 mg, 0.84 mmol, 19%), *m*/*z* 343 (MH⁺). C₁₈H₃₄N₂O₄ exact mass 342.25.

### Racemic trans 4-octyl-pyrrolidine-1,3-dicarboxylic acid 1-tert-butyl ester

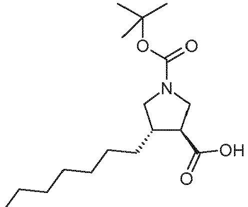

### (i) - Racemic trans 1-benzyl-4-octyl-pyrrolidine-3-carboxylic acid ethyl ester

Benzyl-methoxymethyl-trimethylsilanylmethyl-amine (0.53 mL, 2.1 mmol) was added to a stirred solution of (E)-Undec-2-enoic acid ethyl ester (see J. Org. Chem., 2007, 72(17), 6628-30 for synthesis) (400 mg, 1.9 mmol) in dry toluene (4 mL) under N₂. To this stirred solution was added trifluoroacetic acid (70 µL, 0.95 mmol) and the stirred solution was heated at 50°C for 17 h under N₂. After this time, the reaction mixture was poured into saturated sodium hydrogen carbonate solution (50 mL) and extracted with ethyl acetate (2 × 40 mL). The combined extracts were dried (Na₂SO₄) and evaporated to an orange oil. The oil was purified by column chromatography over silica gel eluting with a gradient of diethyl ether/iso-hexane to give the title compound as a colourless oil (300 mg, 0.87 mmol, 46%), *m*/*z* 346 (MH⁺). C₂₂H₃₅NO₂ exact mass 345.27.

### (ii) - Racemic trans 4-octyl-pyrrolidine-3-carboxylic acid ethyl ester

A suspension of 30% Pd/C (330 mg) in a solution of racemic *trans* 1-benzyl-4-octyl-pyrrolidine-3-carboxylic acid ethyl ester (330 mg, 1.0 mmol) in ethanol (4.9 mL) and formic acid (2.1 mL) was stirred under N₂ for 22h at ambient temperature. After this time the mixture was filtered through Celite and the filtrate evaporated to a residue. This was re-dissolved in ethyl acetate (20 mL) and washed with a saturated solution of sodium hydrogen carbonate (20 mL). The latter was re-extracted with ethyl acetate (2 × 20mL) and the combined extracts were dried (Na₂SO₄) and evaporated to a give the title compound as a colourless oil (174 mg, 0.68 mmol, 68%), *m*/*z* 256 (MH⁺). C₁₅H₂₉NO₂ exact mass 255.22.

### (iii) - Racemic trans 4-octyl-pyrrolidine-1,3-dicarboxylic acid 1-tert-butyl ester 3-ethyl ester

Triethylamine (114 µL, 0.82 mmol) and a solution of di-*tert* butyl dicarbonate (178 mg, 0.82 mmol) in dichloromethane (2 mL), were successively added to a stirred solution of racemic *trans* 4-octyl-pyrrolidine-3-carboxylic acid ethyl ester (174 mg, 0.68 mmol) in dichloromethane (8 mL). After stirring the solution for 20h it was diluted with diethyl ether (20 mL) and the solution washed with saturated ammonium chloride solution (30 mL). The latter was re-extracted with diethyl ether (20ml) and the combined extracts dried (Na₂SO₄) and evaporated to a colourless oil. The oil was purified by column chromatography over silica gel eluting with a gradient of ethyl acetate/iso-hexane to afford the title compound as an oil (196 mg, 0.55 mmol, 81%), *m*/*z* 356 (MH⁺). C₂₀H₃₇NO₄ exact mass 355.27.

### (iv) - Racemic trans 4-octyl-pyrrolidine-1,3-dicarboxylic acid 1-tert-butyl ester

A solution of lithium hydroxide monohydrate (81 mg, 1.9 mmol) in water (1.5 mL) was added to a stirred solution of racemic *trans* 4-octyl-pyrrolidine-1,3-dicarboxylic acid 1-tert-butyl ester 3-ethyl ester (228 mg, 0.64 mmol) in methanol (6 mL) at ambient temperature. The solution was stirred for 17 h, then diluted with water (20 mL) and adjusted to pH 2 with 1 M hydrochloric acid (1.85 mL). The resulting milky mixture was extracted with ethyl acetate (3 × 15 mL) and the combined extracts were dried (Na₂SO₄) and evaporated to give the title compound as a colourless oil (193 mg, 0.59 mmol, 92%), *m*/*z* 328 (MH⁺). C₁₈H₃₃NO₄ requires 327.24.

*Tert-butoxycarbonyl-3-aminomethyl undecanoic acid*

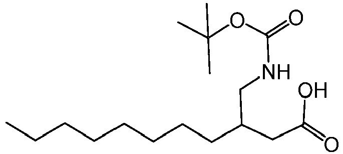

### (i) - 3-Nitromethyl undecanoic acid ethyl ester

To a solution of (E)-Undec-2-enoic acid ethyl ester (1.46 g, 6.8 mmol) in acetonitrile (5.84 mL)were added nitromethane (1.86 mL, 34.3 mmol) and DBU (1.05 mL, 7.0 mmol) at room temperature. The reaction mixture was heated at 65 °C for 4h, cooled and concentrated. The residue was partitioned between ethyl acetate (20 mL) and water (20 mL). The aqueous layer was extracted twice with ethyl acetate (20 mL). The combined organic extracts were washed further with 0.1M HCl (30 mL), water (30 mL) and brine (30 mL). The organic extract was dried over magnesium sulphate, filtered and concentrated. The crude material was purified by automated flash column chromatography eluting with 0-80% ethyl acetate in hexane. Fractions were isolated and concentrated to leave a colourless oil. Yield 0.48 g, 26%. ¹H NMR (400 MHz, CDCl₃) δ (ppm): 0.82 (t, 3H, CH₃), 1.25 (m, 16H, CH₂), 1.42 (m, 3H, CH₃), 2.42 (m, 2H, CH₂), 2.62 (m, 1H, CH), 4.19 (m, 2H, CH₂), 4.46 (m, 2H, CH₂).

### (ii) - 3-Aminomethyl undecanoic acid ethyl ester

To a solution of 3-nitromethyl undecanoic acid ethyl ester (200 mg, 0.73 mmol) in acetic acid (5 mL) were added at room temperature THF (10.52 mL), water (2.1 mL), concentrated HCl (820 uL), and portionwise Zn dust (600 mg, 9.2 mmol). The reaction mixture was vigorously stirred for 2.5 h. The suspension was then filtered and the filtrate was concentrated *in vacuo.* The residue was diluted with DCM (20 mL) washed with water (20 mL). The organic layer was isolated and dried over magnesium sulphate, filtered and the filtrate was concentrated in vacuo to leave the acetate salt. Yield 150 mg, 88%. *m*/*z* 244.2 (MH⁺). C14H29NO2 requires 243.22.

### (iii) - Tert-butoxycarbonyl-3-aminomethyl undecanoic acid ethyl ester

To a solution of 3-aminomethyl undecanoic acid ethyl ester (150 mg, 0.6 mmol) in THF (20 mL), triethyl amine (170 µL, 1.2 mmol) was added. After 10 min di-*tert*-butyl dicarbonate (147 mg, 0.67 mmol) was added and the reaction mixture was stirred for 6 h at room temperature. The reaction mixture was evaporated to dryness; the crude material was re-dissolved in water (20 mL) and extracted with DCM (20 mL). The organic layer was isolated, dried over magnesium sulphate, filtered and concentrated *in vacuo.* The crude material was purified by automated flash column chromatography eluting with 0-80% ethyl acetate in hexane. Fractions were isolated and evaporated to leave a colourless oil. Yield 70 mg, 33%. *m*/*z* (MH⁺). C19H37NO4₂ requires 343.27.

### (iv) - Tert-butoxycarbonyl-3-aminomethyl undecanoic acid

Tert-butoxycarbonyl-3-Aminomethyl undecanoic acid ethyl ester (70mg, 0.2 mmol) was dissolved in THF (600 µL). To the solution was added 1M NaOH (4 mL) and the reaction was stirred at room temperature for 16 h. The reaction mixture was then concentrated, re-dissolved in water (20 mL) and extracted with DCM (20 mL). The aqueous layer was isolated, acidified to pH 1 using 1M HCl and extracted with DCM (20 mL). The organic layer was isolated, dried over magnesium sulphate, filtered and concentrated to dryness to leave a yellow oil. Yield 40 mg, 63%.*m*/*z* 316.63 (MH⁺). C17H33NO4 requires 315.24.

*(S)-4-Isobutyl-piperazine-1,3-dicarboxylic acid 1-tert-butyl ester*

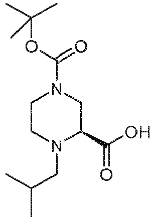

The title compound was prepared in 43% yield from iso-butyraldehyde and (S)-piperazine-1,3-dicarboxylic acid 1-tert-butyl ester using the method described for S)-4-Octyl-piperazine-1,3-dicarboxylic acid 1-tert-butyl ester. *m*/*z 287* (MH⁺), C₁₄H₂₆N₂O₄ requires 286.19.

*Racemic trans-4-isobutyl-pyrrolidine-1,3-dicarboxylic acid 1-tert-butyl ester*

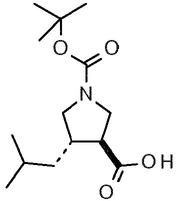

The title compound was prepared from (E)-5-methyl-hex-2-enoic acid ethyl ester (see Synthesis 2009, 15, 2634-45 for a preparation) by an analogous sequence of reactions to that described for Racemic *trans* 4-octyl-pyrrolidine-1,3-dicarboxylic acid 1-tert-butyl ester. For the title compound *m*/*z* 272 (MH⁺), C₁₄H₂₅NO₄ requires 271.18.

*1-Tert-butoxycarbonyl-4-octyl-piperidine-3-carboxylic acid*

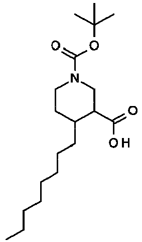

### (i) - 1-Tert-butyl 5-ethyl 4-oct-1-ynyl-3,6-dihydro-2H-pyridine-1,5-dicarboxylate

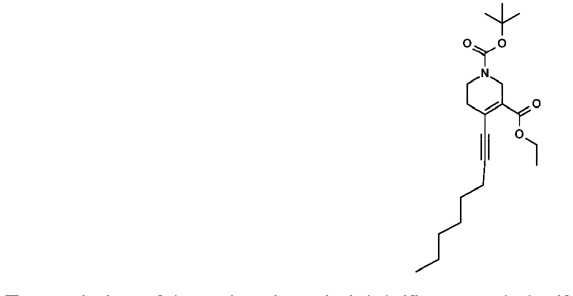

To a solution of 1-tert-butyl 5-ethyl 4-(trifluoromethylsulfonyloxy)-3,6-dihydro-2H-pyridine-1,5-dicarboxylate (US 2011/0190278) (620 mg, 1.53 mmol) in ethyl acetate (15 mL)was added 1-octyne (340 µL, 2.31 mmol), triethylamine (320 µL, 2.30 mmol), bis(triphenylphosphine)palladium (II) dichloride (54 mg, 0.077 mmol) and copper iodide (3 mg, 0.015 mmol). The reaction mixture was stirred at 50°C for 4 hours, then filtered under suction through Celite and washed through with ethyl acetate. The filtrate was evaporated at reduced pressure. The residue was purified by silica gel chromatography eluting with 0 - 30% ethyl acetate in iso-hexane to yield the title compound (364 mg, 66%). m/z 364 (MH+), C21H33NO4 exact mass 363.241.

### (ii) - 1 -Tert-butyl 3-ethyl 4-octylpiperidine-1,3-dicarboxylate

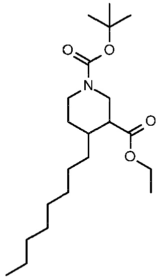

To a solution of 1-tert-butyl 5-ethyl 4-oct-1-ynyl-3,6-dihydro-2H-pyridine-1,5-dicarboxylate (360 mg, 0.99 mmol) in acetic acid (25 mL) was added platinum oxide (40 mg). The reaction mixture was hydrogenated for 16 hours and then a further quantity of platinum oxide (50 mg) was added and hydrogenated for a further 4 hours. The reaction mixture was then filtered under suction through Celite and washed through with ethyl acetate. The filtrate was evaporated at reduced pressure and then the residue was partitioned between ethyl acetate and water. The pH of the aqueous layer was adjusted to pH 10 by the addition of 880 ammonia. After separation of the layers, the aqueous phase was re-extracted with ethyl acetate and then the combined organic layers were passed through a hydrophobic frit. The solvent was evaporated at reduced pressure and the residue purified by silica gel chromatography eluting with 0 - 30% ethyl acetate in iso-hexane to yield the title compound {relative stereochemistry, presumed cis} (133mg, 36%). m/z 370 (MH+), C21H39NO4 exact mass 369.288.

### (iii) - 1-Tert-butoxycarbonyl-4-octyl-piperidine-3-carboxylic acid

To a solution of O1-tert-butyl O3-ethyl 4-octylpiperidine-1,3-dicarboxylate (110 mg, 0.30mmol) in tetrahydrofuran (2 mL) and methanol (2 mL) was added 2 M sodium hydroxide solution (2 mL). The reaction mixture was stirred at room temperature for 6 hours and then concentrated at reduced pressure. The residue was partitioned between ethyl acetate and water and the pH adjusted to 2 by the addition of 1M hydrochloric acid. After separation of the layers, the organic phase as washed with water, dried over magnesium sulphate, filtered and concentrated to give the title compound (102 mg, 100%). m/z 342 (MH+), C19H35NO4 exact mass 341.257.

### Racemic trans-1-(tert-butoxycarbonyl)-5-isobutylpiperidine-3-carboxylic acid

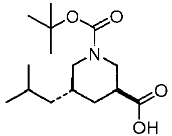

### (i) - 5-Isobutyl-nicotinic acid ethyl ester

Isobutylmagnesium chloride (2 M solution in THF) (9.25 mL, 18.52 mmol) was added dropwise to a solution of zinc bromide (4.17 g, 18.52 mmol) in THF (30 mL) at 0°C under N₂ and the resulting suspension was stirred for 30 minutes. The mixture was cooled to -78°C and [1,1'-Bis(diphenylphosphino)ferrocene]dichloropalladium(II), complex with dichloromethane (252 mg, 0.309 mmol) was added followed by a solution of the 5-bromo-nicotinic acid ethyl ester (1.42 g, 6.17 mmol) in THF (5 mL). After 5 minutes the reaction was allowed to warm to room temperature and stirring was continued for 3 hours. The reaction mixture was diluted with water and ethyl acetate and the phases were separated. The aqueous layer was extracted with ethyl acetate and the combined organic layers were dried (MgSO₄) and the solvent evaporated to afford a crude oil. This was purified by Biotage SP4, 50 g SNAP cartridge eluting with 0 to 50% EtOAc / i-hexane to afford the 5-iosbutyl-nicotinic acid isobutyl ester (365 mg) followed by the 5-isobutyl-nicotinic acid ethyl ester (576mg).

5-isobutyl-nicotinic acid isobutyl ester: ¹H NMR (CDCl₃) 9.10 (1H, s), 8.60 (1H, s), 8.10 (1H, s), 4.15 (2H, d), 2.55 (2H, d), 2.10 (1H, sept), 1.95 (1H, sept), 1.05 (6H, d), 0.95 (6H, d). 5-isobutyl-nictinic acid ethyl ester: ¹H NMR (CDCl₃) 9.05 (1H, s), 8.55 (1H, s), 8.10 (1H, s), 4.45 (2H, q), 2.55 (2H, d), 1.90 (1H, sept), 1.45 (3H, t), 0.95 (6H, d).

### (ii) - Racemic trans-1-(tert-butoxycarbonyl)-5-isobutylpiperidine-3-carboxylic acid

The title compound was prepared from 5-isobutyl-nicotinic acid ethyl ester under the same conditions as described for trans-1-(tert-butoxycarbonyl)-5-octyl 3-carboxylic acid. m/z (ES⁻) 284 (M-H). C15H27NO4 requires: 285.19.

The *trans* geometry of the compound was confirmed from NMR analysis of the deprotected form of the title compound.

### 1-tert-Butoxycarbonyl-5-isobutoxy-piperidine-3-carboxylic acid

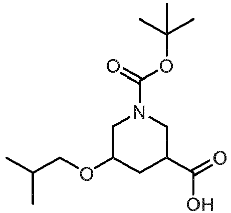

### (i) - Methyl 5-isobutoxypyridine-3-carboxylate

To a solution of methyl 5-hydroxynicotinate (1.5 g, 9.80mmol) in dimethylformamide (20 ml) was added potassium carbonate (2.7 g, 19.6 mmol) and 1-bromo-2-methylpropane (1.2 mL, 11.0 mmol). The reaction mixture was stirred at 80°C for 5 hours and then allowed to cool to room temperature. The mixture was concentrated at reduced pressure and the residue partitioned between ethyl acetate and water. After separation of the layers, the aqueous phase was re-extracted with ethyl acetate. The combined organic layers were dried over magnesium sulphate, filtered and the solvent evaporated at reduced pressure. The residue was purified by silica gel chromatography eluting with 0 - 100% ethyl acetate in iso-hexane to yield the title compound (1.29g, 63%). m/z 210 (MH+), C₁₁H₁₅NO₃ exact mass 209.105.

### (ii) - Methyl 5-isobutoxypiperidine-3-carboxylate

To a solution of methyl 5-isobutoxypyridine-3-carboxylate (1.5 g, 7.18 mmol) in acetic acid (50 mL) was added platinum oxide (100 mg). The reaction mixture was hydrogenated for 3 days and then a further quantity of platinum oxide (50 mg) was added and the mixture hydrogenated for a further 5 hours. The reaction mixture was then filtered under suction through Celite and washed through with ethyl acetate. The filtrate was evaporated at reduced pressure and then the residue was partitioned between ethyl acetate and water. The pH of the aqueous layer was adjusted to pH 10 by the addition of .880 ammonia. After separation of the layers, the aqueous phase was re-extracted with ethyl acetate and then the combined organic layers were dried over magnesium sulphate and then filtered. The solvent was evaporated at reduced pressure to yield the crude title compound (961 mg). m/z 216 (MH+), C₁₁H₂₁NO₃ exact mass 215.152.

### (iii) 1-(tert-butyl) 3-methyl 5-isobutoxypiperidine-1,3-dicarboxylate

To a solution of methyl 5-isobutoxypiperidine-3-carboxylate (960 mg, 4.49 mmol) in dichloromethane (50 mL) was added di-*tert*-butyl dicarbonate (1.1 g, 5.04 mmol). The reaction mixture was stirred at room temperature for 16 hours and then concentrated at reduced pressure. The residue was purified by silica gel chromatography eluting with 0 - 100% diethyl ether in iso-hexane to yield the title compound (665mg, 47%). m/z 260 (MH⁺-tBu), C₁₂H₂₁NO₅ exact mass 259.142.

### (iv) - 1-tert-butoxycarbonyl-5-isobutoxy-piperidine-3-carboxylic acid

To a solution of 1-(tert-butyl) 3-methyl 5-isobutoxypiperidine-1,3-dicarboxylate (660 mg, 2.1 mmol) in dioxane (5 mL) and water (2.5 mL) was added lithium hydroxide monohydrate (266 mg, 6.33 mmol). The reaction mixture was stirred at room temperature for 16 hours and then partially concentrated at reduced pressure. The residue was partitioned between ethyl acetate and water and the pH of the aqueous layer adjusted to pH 1 by the addition of 1 M hydrochloric acid. After separation of the layers, the aqueous phase was re-extracted with ethyl acetate. The combined organic layers were dried over magnesium sulphate, filtered and concentrated to yield the title compound (348mg, 55%). m/z 302 (MH⁺), C₁₅H₂₇NO₅ exact mass 301.189.

*(3RS,5SR)-1-(tert-butoxycarbonyl)-5-(isobutylcarbamoyl) piperidine-3-carboxylic acid*

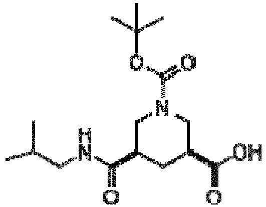

To a solution of 2, 4-dioxo-3-oxa-7-aza-bicyclo[3.3.1] nonane-7-carboxylic acid tert-butyl ester (for a synthesis see US2008/0319018 A1), (70 mg, 0.27 mmol) in THF (5 mL) was added isobutylamine (27 µL, 0.27 mmol). The reaction mixture was left to stir at rt 18 h. The reaction mixture was then evaporated to dryness to leave a pale yellow solid (yield 90 mg, quantitative) m/z 329.0 (MH+), C16H28N2O5 requires 328.20.

*(S)-4-((Tert-butoxycarbonyl)amino-2-(((S)-6-methyloctyl)amino)butanoic acid*

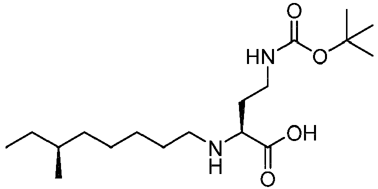

### (i) (S)-6 methyloctanal

Under nitrogen gas atmosphere, (S)-(+)-6-methyl-1-octanol (197 mg, 1.36 mmol) [TCI Ltd] in DCM (3 mL) was added to 0.3M Dess Martin periodinane solution (5 mL, 1.5 mmol) dropwise at room temperature. The reaction mixture was stirred for 1.5h at ambient temperature. The reaction mixture was evaporated and re-suspended in ether (50 mL) and filtered. The filtrate was evaporated to leave a colourless oil. The oil was purified by column chromatography over silica gel, eluting with a gradient of hexane/ethyl acetate to give the title compound as a colourless oil (80 mg, 41%).

### (ii) Methyl (S)-4-(tert-butoxycarbonyl)amino)-2-((S)-6-methyloctyl)amino) butanoate

(S)-6 methyloctanal was reacted with methyl (*S*)-4-(*tert*-butoxycarbonyl)amino)-2-aminobutanoate using the reductive alkylation method as described for (*S*)-4-octyl-piperazine-1.3-dicarboxylic acid 1-tert butyl ester. The title compound was obtained as a white solid (80 mg, 42%), m/z 359.2 (MH⁺). C₁₉H₃₈N₂O₄ exact mass 358.28.

### (S)-4-((tert-butoxycarbonyl)amino-2-((S)-6-methyloctyl)amino)butanoic acid

To a solution of methyl (*S*)-4-(*tert*-butoxycarbonyl)amino)-2-((*S*)-6-methyloctyl)amino) butanoate (80 mg, 0.22 mmol) in methanol (5 mL) was added lithium hydroxide (11 mg, 0.44mmol) in water (1 mL). The reaction mixture was stirred at room temperature for 16 hours. The reaction mixture was concentrated at reduced pressure and the residue was partitioned between ethyl acetate and water. The aqueous phase was acidified by the addition of 1M hydrochloric acid and the product was extracted into ethyl acetate. The organic phase was dried over magnesium sulphate, filtered and the filtrate was evaporated at reduced pressure to yield the title compound (50 mg, 66%), m/z 345.2 (MH+), C₁₈H₃₆N₂O₄ exact mass 344.27.

4-((*tert*-Butoxycarbonyl)amino)-2-cyclohexylbutanoic acid

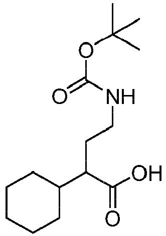

A suspension of platinum oxide (80 mg, 0.28 mmol) in acetic acid (2 mL) was added to a stirred solution of 4-Boc-amino-2-phenyl-butyric acid (300 mg, 1.07 mmol) in acetic acid (20 mL). The reaction was hydrogenated for 24h at ambient temperature and atmospheric pressure. The reaction mixture was filtered through Celite and washed with acetic acid (20 mL). The filtrate was evaporated at reduced pressure to leave a yellow oil (300 mg, 98%). m/z 285.9(M⁺), C₁₅H₂₇NO₄ exact mass 285.19.

### 2-[(tert-Butoxycarbonylamino)methyl]octanoic acid

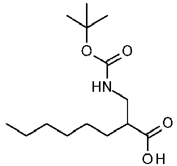

### (i) Ethyl 2-cyanooctanoate

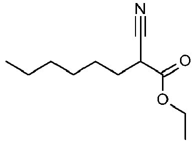

A mixture of ethyl cyanoacetate (2 mL, 19.0 mmol), 1-iodohexane (3.0 mL, 20.0 mmol) and potassium carbonate (2.76 g, 20.0 mmol) in acetone (100 mL) was heated to reflux for 4 h. After cooling to room temperature, the reaction mixture was filtered through Celite and washed with acetone. The filtrate was evaporated at reduced pressure and then partitioned between dichloromethane and saturated ammonium chloride solution. After separation of the layers, the organic phase was washed with brine, dried over magnesium sulphate, filtered and concentrated at reduced pressure. The product was purified by silica gel chromatography eluting with 0 - 30% ethyl acetate in iso-hexane to yield the title compound as a colourless oil (2.29 g, 61%). m/z 198 (MH⁺), C₁₁H₁₉NO₂ exact mass 197.14.

### (ii) Ethyl 2-(aminomethyl)octanoate

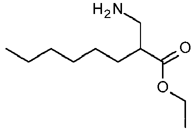

To a solution of ethyl 2-cyanooctanoate (880 mg, 4.47 mmol) in methanol (40 mL) was added cobalt chloride (1.14 g, 8.76 mmol). The mixture was cooled to 0°C and then treated portion-wise with sodium borohydride (1.67 g, 44 mmol). After the addition was complete, the reaction mixture was warmed to room temperature and stirred for a further 30 minutes. The mixture was quenched with 1 M hydrochloric acid and stirred for 2 minutes. The mixture was then basified to ∼ pH 11 by the addition of .880 ammonia and the product extracted into dichloromethane (× 4). The combined organic phases were dried over magnesium sulphate, filtered and concentrated to give the crude title compound as a pale brown oil (775 mg, 86%). m/z 202 (MH⁺), C₁₁H₂₃NO₂ exact mass 201.17.

### (iii) Ethyl 2-[(tert-butoxycarbonylamino)methyl]octanoate

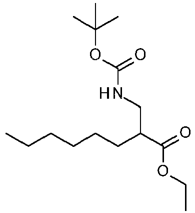

To a solution of ethyl 2-(aminomethyl)octanoate (775 mg, 3.86 mmol) in dichloromethane (50 ml) was added di-*tert*-butyl dicarbonate (1.0 g, 4.58mmol). The reaction mixture was stirred at room temperature for 16h and then concentrated at reduced pressure. The product was purified by silica gel chromatography eluting with 0 - 20% ethyl acetate in *iso-*hexane to yield the title compound as a colourless oil (818 mg, 71%). m/z 246 (M-BOC)⁺, C₁₆H₃₁NO₄ exact mass 301.22.

### (iv) 2-[(tert-Butoxycarbonylamino)methyl]octanoic acid

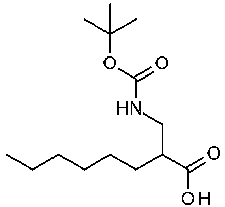

To a solution of ethyl 2-[(*tert*-butoxycarbonylamino)methyl]octanoate (816 mg, 2.71 mmol) in dioxane (12 mL) and water (6 mL) was added lithium hydroxide (350 mg, 8.33 mmol). The reaction mixture was stirred at room temperature for 16 h and then partially concentrated at reduced pressure. The residue was partitioned between diethyl ether and water and the pH adjusted to 1 by the addition of 1 M hydrochloric acid. After separation of the layers, the aqueous phase was re-extracted with diethyl ether (× 2). The combined organic layers were dried over magnesium sulphate, filtered and concentrated to give a colourless oil (715 mg, 97%). m/z 272 (M-H)⁻, C₁₄H₂₇NO₄ exact mass 273.19.

### Synthesis Examples

Table 4A includes examples of the invention.

All compounds were isolated as TFA salts unless otherwise specified. Compounds D9, D11, D15 and D25 were also prepared as acetate salts according to the general method described herein. The structures in the table depict the N-terminal group (-R) and side chain on the Polymyxin B heptapeptide scaffold (PMBH, below). Relative stereochemistry is depicted by heavy or dashed lines. Absolute stereochemistry is depicted by heavy or hashed wedged bonds. The PMBH scaffold:

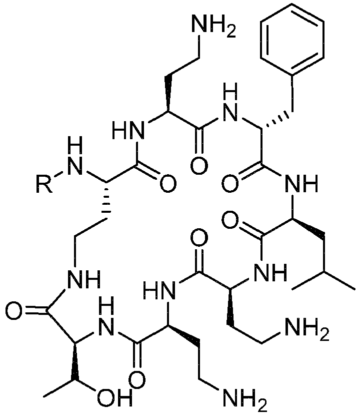

**Table 4A - Example Compounds**

| **Ex.** | **R** | **Formula** | **Mass** | **Method of Preparation** | **sm** | **Name** | **HPLC Retention time (min.)** | **m/z** |
|---|---|---|---|---|---|---|---|---|
| D1 | 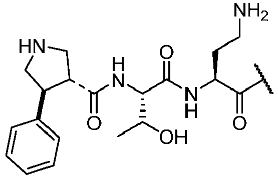 | C54H85 N15O12 | 1135.7 | 2A | Int. 7 | *trans*-4-Phenylpyrrolidine-3-carbonyl polymyxin B nonapeptide. Isomer 1 | 5.13 | 1136 [MH+] |
| D2 | 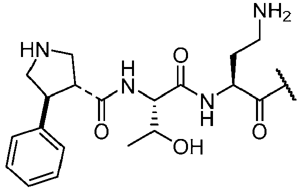 | C54H85 N15O12 | 1135.7 | 2A | Int. 7 | *trans*-4-Phenylpyrrolidine-3-carbonyl polymyxin B nonapeptide. Isomer 2 | 5.23 | 1137 [MH+] |
| D3 | 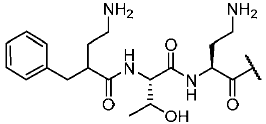 | C54H87 N15O12 | 1137.7 | 2A | Int. 7 | 4-Amino-2-benzyl butanoylpolymyxin B nonapeptide. Isomer 1 | 5.17 | 1139 [MH+] 570 [M+2H]²⁺ |
| D4 | 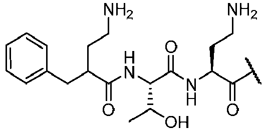 | C54H87 N15O12 | 1137.7 | 2A | Int. 7 | 4-Amino-2-benzyl butanoyl polymyxin B nonapeptide. Isomer 2 | 5.44 | 1139 [MH+] 570 [M+2H]²⁺ |
| D5 | 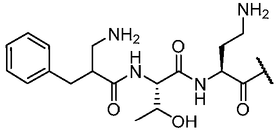 | C53H85 N15O12 | 1123.7 | 2A | Int. 7 | 3-Amino-2-benzylpropanoylpolym yxin B nonapeptide. Isomer 1 | 5.57 | 1124 [MH+] 563 [M+2H]²⁺ |
| D6 | 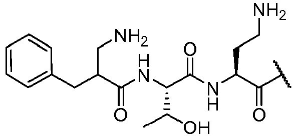 | C53H85 N15O12 | 1123.7 | 2A | Int. 7 | 3-Amino-2-benzylpropanoylpolym yxin B nonapeptide. Isomer 2 | 5.83 | 1124 [MH+] 563 [M+2H]²⁺ |
| D7 | 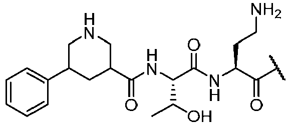 | C55H87 N15O12 | 1149.7 | 2A | Int. 7 | (5-Phenyl-piperidine)-3-carbonyl polymyxin B nonapeptide. Isomer 1 | 5.66 | 1151 [MH+] |
| D8 | 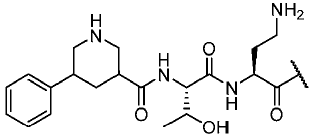 | C55H87 N15O12 | 1149.7 | 2A | Int. 7 | (5-Phenyl-piperidine)-3-carbonyl polymyxin B nonapeptide. Isomer 2 | 5.93 | 1151 [MH+] 577 [M+2H]²⁺ |
| D9 | 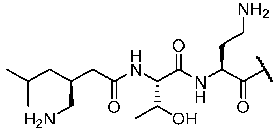 | C51H89 N15O12 | 1103.7 | 2A | Int. 7 | 3(S)-3-aminomethyl-5-methyl hexanoyl polymyxin B nonapeptide | 5.29 | 1104 [MH+] |
| D10 | 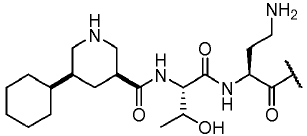 | C55H93 N15O12 | 1155.7 | 2A | Int. 7 | (*cis*-5-cyclohexyl-piperidine)-3-carbonyl polymyxin B nonapeptide. Isomer 1 | 5.37 | 1157 [MH+] 579 [M+2H]²⁺ |
| D11 | 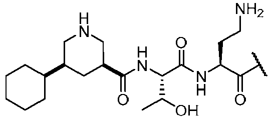 | C55H93 N15O12 | 1155.7 | 2A | Int. 7 | (*cis*-5-cyclohexylpiperidine)-3-carbonyl polymyxin B nonapeptide. Isomer 2 | 5.79 | 1157 [MH+] 579 [M+2H]²⁺ |
| D12 | 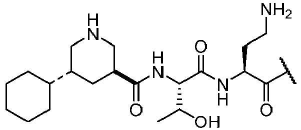 | C55H93 N15O12 | 1155.7 | 2A | Int. 7 | (*trans*-5-cyclohexylpiperidine)-3-carbonyl polymyxin B nonapeptide. Isomer 1 | 5.31 | 1157 [MH+] 579 [M+2H]²⁺ |
| D13 | 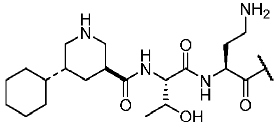 | C55H93 N15O12 | 1155.7 | 2A | Int. 7 | (*trans*-5-cyclohexylpiperidine)-3-carbonyl polymyxin B nonapeptide. Isomer 2 | 5.70 | 1157 [MH+] 579 [M+2H]²⁺ |
| D14 | 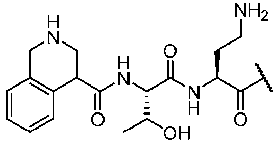 | C53H83 N15O12 | 1121.6 | 2A | Int. 7 | 1,2,3,4-tetrahydroisoquinoline -4-carbonyl polymyxin B nonapeptide. Isomer 1 | 4.90 | 1123 [MH+] 562 [M+2H]²⁺ |
| D15 | 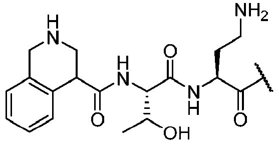 | C53H83 N15O12 | 1121.6 | 2A | Int. 7 | 1,2,3,4-tetrahydroisoquinoline -4-carbonyl polymyxin B nonapeptide. Isomer 2 | 5.34 | 1122 [MH+] 561 [M+2H]²⁺ |
| D16 | 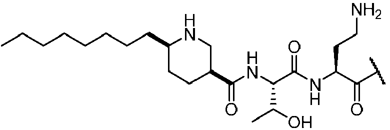 | C57H99 N15O12 | 1185.8 | 2A | Int. 7 | (*cis*-6-octylpiperidi ne)-3-carbonyl polymyxin B nonapeptide. Isomer 1 | 6.20 | 594 [M+2H]²⁺ |
| D17 | 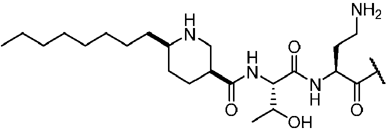 | C57H99 N15O12 | 1185.8 | 2A | Int. 7 | (*cis*-6-octylpiperidine)-3-carbonyl polymyxin B nonapeptide. Isomer 2 | 6.60 | 1186 [MH+] 594 [M+2H]²⁺ |
| D18 | 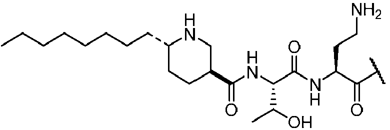 | C57H99 N15O12 | 1185.8 | 2A | Int. 7 | (*trans-*6-octylpiperidine)-3-carbonyl polymyxin B nonapeptide. Isomer 1 | 6.37 | 594 [M+2H]²⁺ |
| D19 | 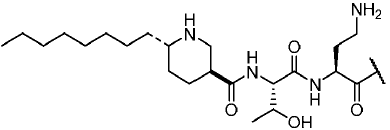 | C57H99 N15O12 | 1185.8 | 2A | Int. 7 | (*trans*-6-octylpiperidine)-3-carbonyl polymyxin B nonapeptide. Isomer 2 | 6.64 | 1186 [MH+] 594 [M+2H]²⁺ |
| D20 | 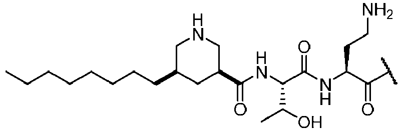 | C57H99 N15O12 | 1185.8 | 2A | Int. 7 | (*cis*-5-octylpiperidine)-3-carbonyl polymyxin B nonapeptide. Isomer 1 | 6.29 | 594 [M+2H]²⁺ |
| D21 | 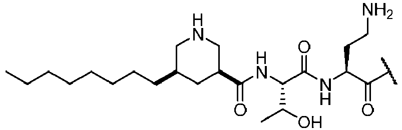 | C57H99 N15O12 | 1185.8 | 2A | Int. 7 | (*cis*-5-octylpiperidine)-3-carbonyl polymyxin B nonapeptide. Isomer 2 | 6.75 | 1187 [MH+] |
| D22 | 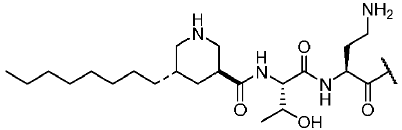 | C57H99 N15O12 | 1185.8 | 2A | Int. 7 | (*trans*-5-octylpiperidine)-3-carbonyl polymyxin B nonapeptide. Isomer 1 | 6.36 | 1187 [MH+] 594 [M+2H]²⁺ |
| D23 | 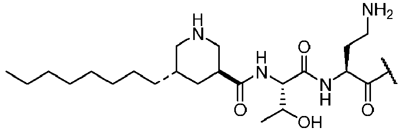 | C57H99 N15O12 | 1185.8 | 2A | Int. 7 | (*trans*-5-octylpiperidine)-3-carbonyl polymyxin B nonapeptide. Isomer 2 | 6.81 | 1186 [MH+] 594 [M+2H]²⁺ |
| D24 | 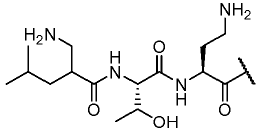 | C50H87 N15O12 | 1089.7 | 2A | Int. 7 | 2-aminomethyl-4-methyl pentanoyl polymyxin B nonapeptide. Isomer 1 | 5.14 | 1090 [MH+] 546 [M+2H]²⁺ |
| D25 | 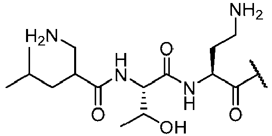 | C50H87 N15O12 | 1089.7 | 2A | Int. 7 | 2-aminomethyl-4-methyl pentanoyl polymyxin B nonapeptide. Isomer 2 | 5.21 | 1090 [MH+] 546 [M+2H]²⁺ |
| D26 | 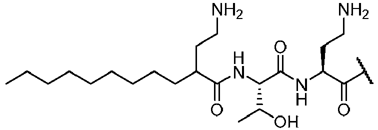 | C56H99 N15O12 | 1173.8 | 2A | Int. 7 | 2-(2-aminoethyl)-undecanoyl polymyxin B nonapeptide. Isomer 1 | 6.62 | 1174 [MH+] 588 [M+2H]²⁺ |
| D27 | 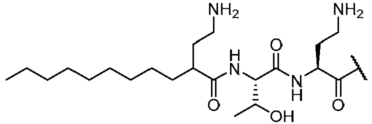 | C56H99 N15O12 | 1173.8 | 2A | Int. 7 | 2-(2-aminoethyl)-undecanoyl polymyxin B nonapeptide. Isomer 2 | 6.14 | 1175[MH +] 588 [M+2H]²⁺ |
| D28 | 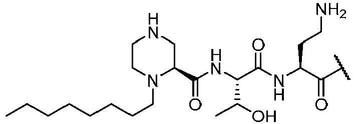 | C56H98 N16O12 | 1186.8 | 2A | Int. 7 | (*S*)-1-octyl-piperazine-2-carbonyl polymyxin B nonapeptide. | 5.99 | 1187 [MH+] 594 [M+2H]²⁺ |
| D29 | 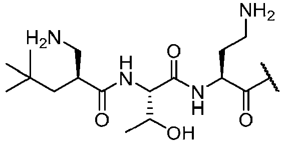 | C51H89 N15O12 | 1103.7 | 2A | Int. 7 | (S)-2-aminomethyl-4-dimethyl pentanoyl polymyxin B nonapeptide | 5.36 | 1105 [MH+] 553 [M+2H]²⁺ |
| D30 | 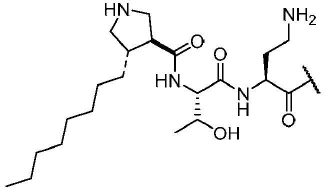 | C56H97 N15O12 | 1171.7 | 2A | Int. 7 | *trans*-4-octylpyrrolidine-3-carbonyl polymyxin B nonapeptide. Isomer 1 | 6.51 | 1173 [MH+] 587 [M+2H]²⁺ |
| D31 | 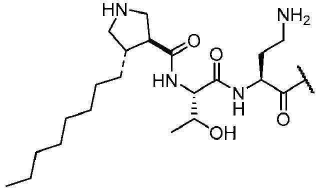 | C56H97 N15O12 | 1171.7 | 2A | Int. 7 | *trans*-4-octylpyrrolidine-3-carbonyl polymyxin B nonapeptide. Isomer 2 | 6.10 | 1173 [MH+] 587 [M+2H]²⁺ |
| D32 | 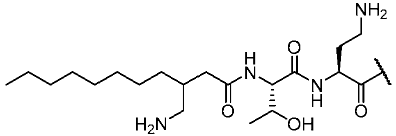 | C55H97 N15O12 | 1159.7 | 2A | Int. 7 | 3-aminomethyl-undecanoyl polymyxin B nonapeptide. Isomer 1 | 6.31 | 1161 [MH+] 581 [M+2H]²⁺ |
| D33 | 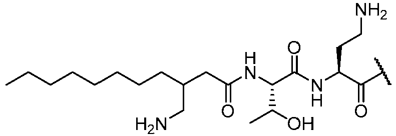 | C55H97 N15O12 | 1159.7 | 2A | Int. 7 | 3-aminomethyl-undecanoyl polymyxin B nonapeptide. Isomer 2 | 6.41 | 1161 [MH+] 581 [M+2H]²⁺ |
| D34 | 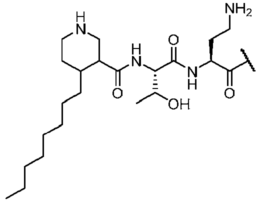 | C57H99 N15O12 | 1185.8 | 2A | Int. 7 | 4-octylpiperidine-3-carbonyl polymyxin B nonapeptide. Isomer 1 | 6.19 | 1187 [MH+] 594 [M+2H]²⁺ |
| D35 | 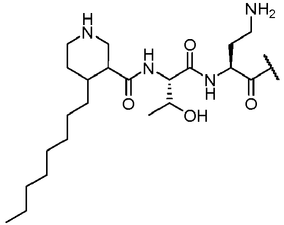 | C57H99 N15O12 | 1185.8 | 2A | Int. 7 | 4-octylpiperidine-3-carbonyl polymyxin B nonapeptide. Isomer 2 | 6.76 | 1187 [MH+] 594 [M+2H]²⁺ |
| D36 | 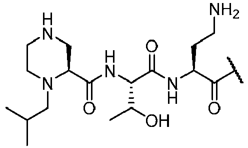 | C52H90 N16O12 | 1130.7 | 2A | Int. 7 | (S)-1-(2-methylpropyl)-piperazine-2-carbonyl polymyxin B nonapeptide | 5.28 | 1132 [MH+] 567 [M+2H]²⁺ |
| D37 | 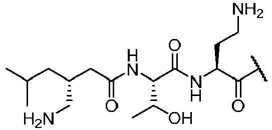 | C51H89 N15O12 | 1103.7 | 2A | Int. 7 | 3(R)-3-ami nomethyl-5-methyl hexanoyl polymyxin B nonapeptide | 5.28 | 553 [M+2H]²⁺ |
| D38 | 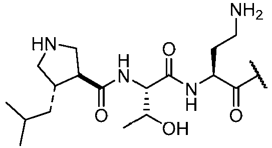 | C52H89 N15O12 | 1115.7 | 2A | Int. 7 | *trans-*4-(2-methyl propyl) pyrrolidine-3-carbonyl polymyxin B nonapeptide. Isomer 1 | 5.18 | 1117 [MH+] 559 [M+2H]²⁺ |
| D39 | 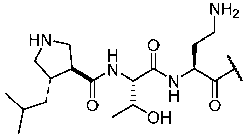 | C52H89 N15O12 | 1115.7 | 2A | Int. 7 | *trans*-4-(2-methyl propyl) pyrrolidine-3-carbonyl polymyxin B nonapeptide. Isomer 2 | 5.43 | 1117 [MH+] 559 [M+2H]²⁺ |
| D40 | 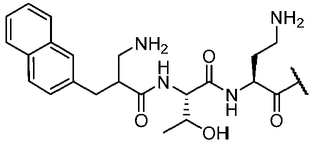 | C57H87 N15O12 | 1173.7 | 2A | Int. 7 | 2-aminomethyl-3-naphthalen-2-yl-propanoyl polymyxin B nonapeptide. Isomer 1 | 5.60 | 1175[MH ⁺] |
| D41 | 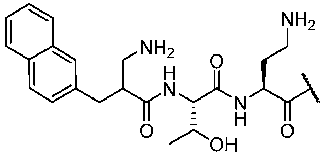 | C57H87 N15O12 | 1173.7 | 2A | Int. 7 | 2-aminomethyl-3-naphthalen-2-yl-propanoyl polymyxin B nonapeptide. Isomer 2 | 5.94 | 1175[MH ⁺] |
| D42 | 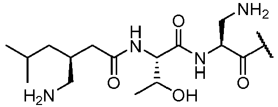 | C50H87 N15O12 | 1089.6 | 3 | Int 5 | 3(S)-3-aminomethyl-5-methyl hexanoyl L-Thr-L-Dap-polymyxin B heptapeptide | 5.41 | 1090 [MH+] 546 [M+2H]²⁺ |
| D43 | 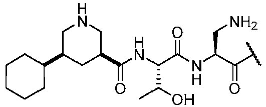 | C54H91 N15O12 | 1141.7 | 3 | Int 5 | (*cis-*5-cyclohexylpiperidine)-3-carbonyl L-Thr-L-Dap-polymyxin B heptapeptide isomer 1 | 5.61 | 1143 [MH+] 572 [M+2H]²⁺ |
| D44 | 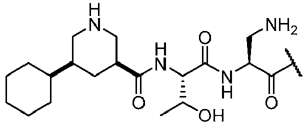 | C54H91 N15O12 | 1141.7 | 3 | Int 5 | (*cis*-5-cyclohexylpiperidine)-3-carbonyl L-Thr-L-Dap-polymyxin B heptapeptide isomer 1 | 5.94 | 1143 [MH+] 572 [M+2H]²⁺ |
| D45 | 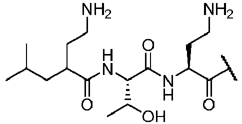 | C51H89 N15O12 | 1103.7 | 2A | Int 7 | 2-(2-aminoethyl)-4-methyl pentanoyl polymyxin B nonapeptide isomer 1 | 5.18 | 1104.6 [MH+] |
| D46 | 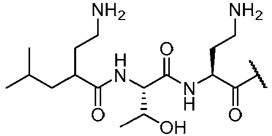 | C51H89 N15O12 | 1103.7 | 2A | Int 7 | 2-(2-aminoethyl)-4-methyl pentanoyl polymyxin B nonapeptide isomer 2 | 5.40 | 1104.6 [MH+] |
| D47 | 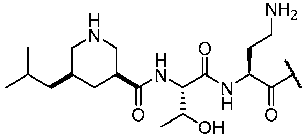 | C53H91 N15O12 | 1129.7 | 2A | Int 7 | (*cis*-5-(2-methylpropyl) piperidine)-3-carbonyl polymyxin B nonapeptide isomer 1 | 5.33 | 1130 [MH+] 566 [M+2H]²⁺ |
| D48 | 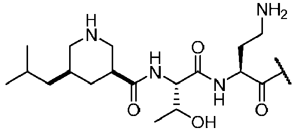 | C53H91 N15O12 | 1129.7 | 2A | Int 7 | (*cis*-5-(2-methylpropyl) piperidine)-3-carbonyl polymyxin B nonapeptide isomer 2 | 5.61 | 1130 [MH+] 566 [M+2H]²⁺ |
| D49 | 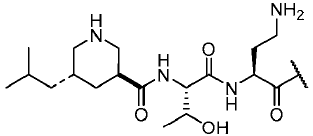 | C53H91 N15O12 | 1129.7 | 2A | Int 7 | (*trans*-5-(2-methyl propyl) piperidine)-3-carbonyl polymyxin B nonapeptide isomer 1 | 5.25 | 1130 [MH+] 566 [M+2H]²⁺ |
| D50 | 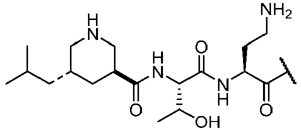 | C53H91 N15O12 | 1129.7 | 2A | Int 7 | (*trans-*5*-*(2-methyl propyl) piperidine)-3-carbonyl polymyxin B nonapeptide isomer 2 | 5.58 | 1130 [MH+] 566 [M+2H]²⁺ |
| D51 | 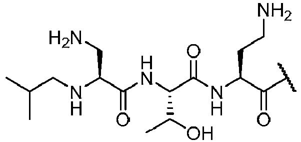 | C50H88 N16O12 | 1104.7 | 2A | Int 7 | (S)-2-(2-methylpropylamino) 3-aminopropanoyl polymyxin B nonapeptide | 5.23 | 1105 [MH+] 552 [M+2H]²⁺ |
| D52 | 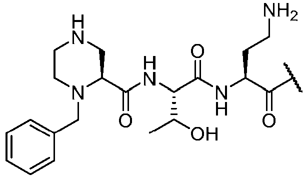 | C55H88 N16O12 | 1164.7 | 2A | Int 7 | (S)-1-benzyl piperazine-2-carbonyl polymyxin B nonapeptide. | 5.45 | 1166 [MH+] 583 [M+2H]²⁺ |

The comparator compounds were Polymyxin B (PMB), C1 (NAB-739), and C2 (CB-182,804).

CB-182,804 (C2) is a polymyxin decapeptide derivative with an aryl urea substituent at the N-terminus, which has been claimed to have lower toxicity than Polymyxin B (shown as compound 5 in WO 2010/075416), and was prepared by the present inventors. C1 was also prepared in-house, and corresponds to NAB-739 (as described by Vaara in, for example, WO 2008/01773. Other comparator compounds were prepared in-house as shown in Table 4B.

**Table 4B - Further Comparator Compounds**

| **Ex.** | **-R** | **Formula** | **Mass** | **Method of Preparation** | **sm** | **Name** | **HPLC Retentio n time (min.)** | **m/z** |
|---|---|---|---|---|---|---|---|---|
| CC3 | 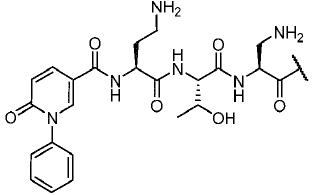 | C58H87 N17O14 | 1245.7 | 3B | Int 5 | Compound 5x in T.M.Magee at al, J. Med. Chem., 2013, 5079 | 5.24 | 1247 [MH+] |
| CC4 | 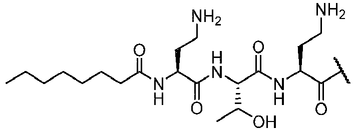 | C55H96 N16O13 | 1189.5 | 3B | Int 7 | Octanoyl polymyxin B decapeptide | 5.97 | 1190 [MH+] 595 [M+2H]²⁺ 397 [M+3H]³⁺ |
| CC5 | 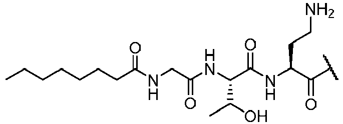 | C53H91 N15O13 | 1146.4 | 3B | Int 7 | Octanoyl-Gly-Polymyxin B nonapeptide | 6.17 | 1147 [MH+] |
| CC6 | 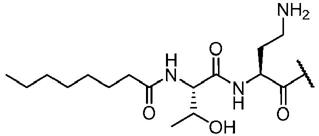 | C51H88 H14O12 | 1088.7 | 1 | Int 2 | Octanoyl polymyxin B nonapeptide, sulphate salt | 6.29 | 1089.6 [MH+] |
| CC7 | 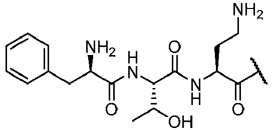 | C52H83 N15O12 | 1109.6 | 2A | Int 7 | D-Phe polymyxin B nonapeptide | 5.43 | 1110 [MH+] 555 [M+2H]²⁺ |
| CC8 | 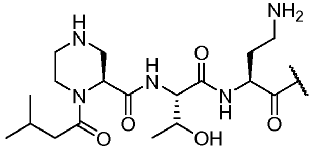 | C53H90 N16O13 | 1158.7 | 2A | Int.7 | (S)-1-(3-methylbutanoyl)pipera zine-2-carbonyl polymyxin B nonapeptide. | 5.30 | 1159 [MH+] 580 [M+2H]²⁺ |

### Further Synthesis Examples

Table 4C includes additional compounds of the invention. All compounds were prepared using general method 2A, as described above, apart from example D93 which used dicyclohexyl carbodiimide in the final coupling step. All compounds were isolated as TFA salts. Compounds D65, D67, D69, D76, D77, D78, D81 and D86 were also prepared as acetate salts.

Structures depict the N-terminal group and side chain on the Polymyxin B or Polymyxin E heptapeptide scaffold (PMBH or PMEH, below). Relative stereochemistry is depicted by heavy or dashed lines. Absolute stereochemistry is depicted by heavy or hashed wedged bonds.

In Table 4C, Scaffold refers to polymyxin B ("B") and polymyxin E ("E") scaffolds, as shown below. R- is the N-terminal and side-chain on the heptapeptide.

Similarly, in Table 4D, Scaffold refers to polymyxin B ("B") and polymyxin E ("E") scaffolds, as shown below. R- is the N-terminal and side-chain on the heptapeptide.

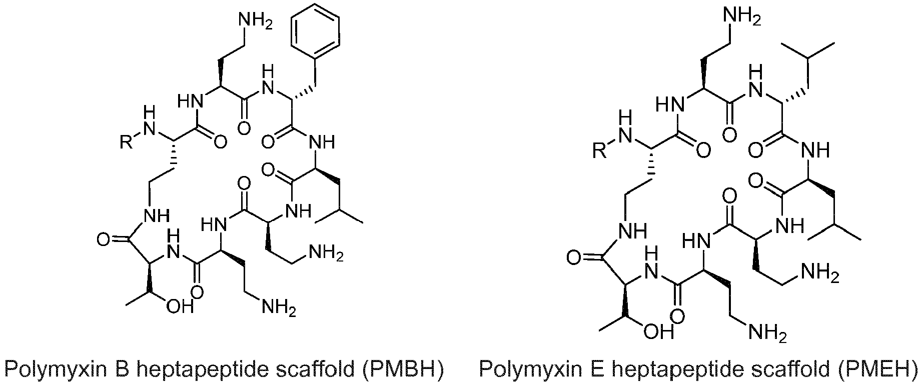

Table 4D: All compounds were isolated as TFA salts. Compounds D95, D100, D101, D102, D103, D104, D105, D106, D107, and D114 were also prepared as acetate salts.

**Table 4C- Further Example Compounds**

| Ex | R- | Scaffold | Formula | mass | Starting material | name | HPLC Retention time (min) | m/z |
|---|---|---|---|---|---|---|---|---|
| D53 | 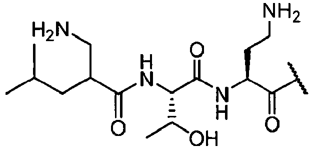 | E | C47H89 N15O12 | 1055.7 | Int. 10 | 2-aminomethyl-4-methyl pentanoyl polymyxin E nonapeptide. | 5.27 | 1056[MH+] 529[M+2H]²⁺ |
| D54 | 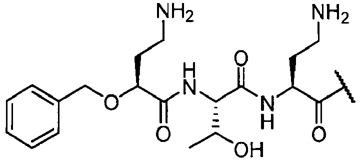 | B | C54H87 N15O13 | 1153.7 | Int 7 | (S)-4-amino-2-(benzyloxy)butan oyl Polymyxin B nonapeptide | 5.47 | 1154[MH+] 578[M+2H]²⁺ |
| D55 | 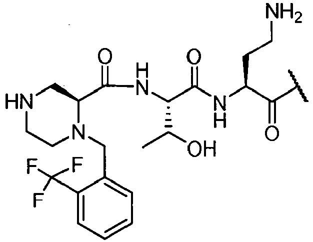 | B | C56H87 F3N16O 12 | 1232.7 | Int 7 | (S)-1-(2-trifluoromethilbenzil)piperazine-2-carbonyl Polymixin B non apeptide | 5.88 | 1234[MH+] 617[M+2H]²⁺ |
| D56 | 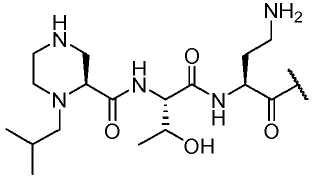 | E | C49H92 N16O12 | 1096.7 | Int 10 | (S)-1-(2-methylpropyl)-piperazine-2-carbonyl polymyxin E nonapeptide | 5.07 | 578[M+2H]²⁺ |
| D57 | 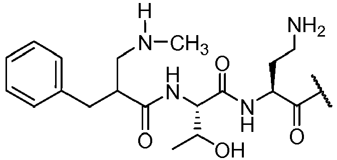 | B | C54H87 N15O12 | 1137.7 | Int7 | 2-benzyl-3-(methylamino)pr opanoyl polymyxin B nonapeptide | 5.49 | 1138.6[MH+] |
| D58 | 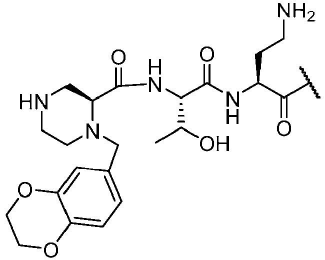 | B | C57H90 N16O14 | 1222.7 | Int7 | (S)-1-((2,3-dihydrobenzo[b][ 1,4]dioxin-6-yl)methyl)pi peraz ine-2-carbonyl polymyxin B nonapeptide | 5.38 | 1224[MH+] 613[M+2H]²⁺ |
| D59 | 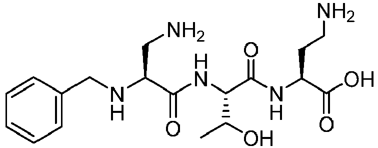 | B | C53H86 N16O12 | 1138.7 | Int 7 | (S)-3-amino-2-(benzylamino)pr opanoyl Polymyxin B nonapeptide | 5.27 | 1140[MH+] 570[M+2H]²⁺ |
| D60 | 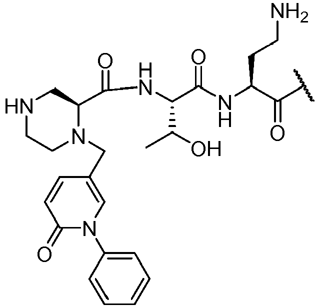 | B | C60H91 N17O13 | 1257.7 | Int 7 | (S)-1-((6-oxo-1-phenyl-1,6-dihydropyridin-3-yl)methyl)pi peraz ine-2-carbonyl Polymyxin B nonapeptide | 5.19 | 1259[MH+] 630[M+2H]²⁺ |
| D61 | 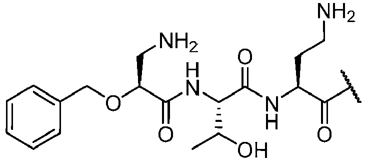 | B | C53H85 N15O13 | 1139.6 | Int 7 | (S)-3-amino-2-(benzyloxy)propa noyl Polymyxin B nonapeptide | 5.10 | 1141[MH+] 571[M+2H]²⁺ |
| D62 | 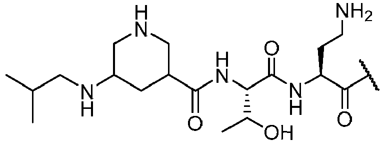 | B | C53H92 N16O12 | 1144.7 | Int 7 | 5-(isobutylamino)pi peridine-3-carbonyl Polymyxin B nonapeptide | 4.96 | 594[M+2H]²⁺ |
| D63 | 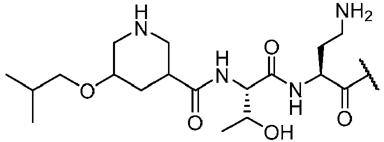 | B | C53H91 N15O13 | 1145.7 | Int 7 | 5-(isobutoxy)piperi dine-3-carbonyl Polymyxin B nonapeptide | 5.37 | 1146[MH+] 574[M+2H]²⁺ |
| D64 | 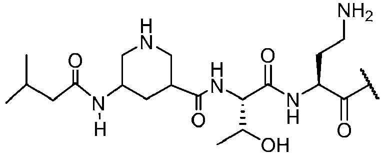 | B | C54H92 N16O13 | 1172.7 | Int 7 | 5-(3-methylbutanamid o)piperidine-3-carbonyl Polymyxin B nonapeptide | 5.18 | 1174[MH+] 588[M+2H]²⁺ |
| D65 | 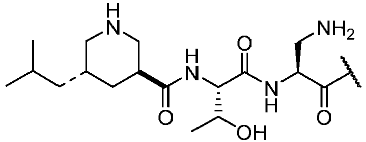 | B | C52H89 N15O12 | 1115.7 | Int 11 | (*trans*-5-(isobutyl -piperidine)-3-carbonyl L-Thr-L-Dap-polymyxin B heptapeptide | 5.45 | 1117[MH+] 559[M+2H]²⁺ |
| D66 | 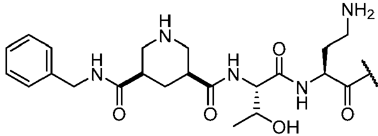 | B | C57H90 N16O13 | 1206.7 | Int 7 | *cis*-5-(benzylcarbamoy l)piperidine-3-carbonyl Polymyxin B nonapeptide | 5.45 | 1207.7 [MH+] |
| D67 | 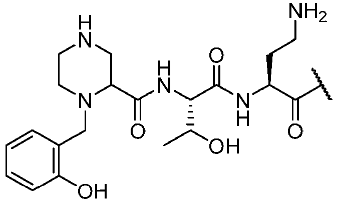 | B | C55H88 N16O13 | 1180.7 | Int7 | 1-(2-hydroxybenzyl)pi perazine-2-carbonyl Polymyxin B nonapeptide | 5.83 | 1181[MH+] 591[M+2H]²⁺ |
| D68 | 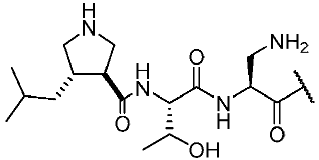 | B | C51H87 N15O12 | 1101.7 | Int 11 | *trans*-4-(2-methyl propyl) pyrrolidine-3-carbonyl -L-Thr-L-Dap-polymyxin B heptapeptide | 5.34 | 1103[MH+] 552[M+2H]²⁺ |
| D69 | 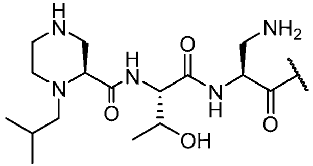 | B | C51H88 N16O12 | 1116.7 | Int11 | (S)-1-isobutyl piperazine-2-carbonyl -L-Thr-L-Dap-polymyxin B heptapeptide | 5.20 | 1118[MH+] 559[M+2H]²⁺ |
| D70 | 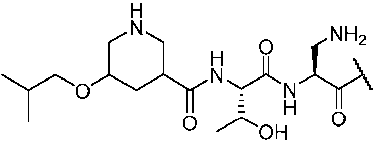 | B | C52H89 N15O13 | 1131.7 | Int11 | 5-isobutoxypiperidi ne-3-carbonyl L-Thr-L-Dap-polymyxin B heptapeptide | 5.47 | 1133 [MH+] 567 [M+2H]²⁺ |
| D71 | 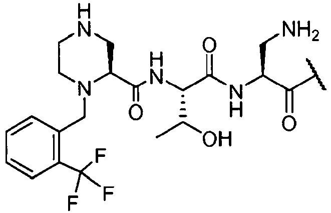 | B | C55H85 F3N16O 12 | 1218.6 | Int 11 | (S)-1-(2-trifluoromethylbenzyl)piperazine-2-carbonyl L-Thr-L-Dap-polymixin B heptapeptide | 5.85 | 1219[MH+] 610[M+2H]²⁺ 407[M+3H]³⁺ |
| D72 | 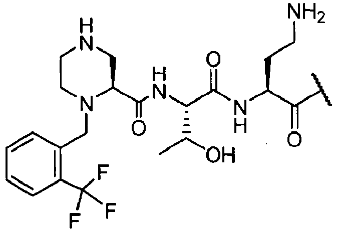 | E | C53H89 F3N16O 12 | 1198.7 | Int 10 | (S)-1-(2-trifluoromethylbenzyl)piperazine-2-carbonyl polymixin E nonapeptide | 5.70 | 1200[MH+] 600[M+2H]²⁺ |
| D73 | 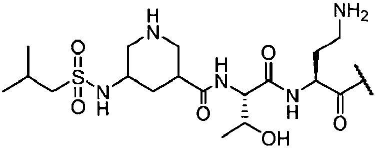 | B | C53H92 N16O14 S | 1208.7 | Int 7 | 5-((2-methylpropyl)sulf onamido)piperidi ne-3-carbonyl-polymyxin B nonapeptide | 5.42 | 1210[MH+] 609[M+2H]²⁺ |
| D74 | 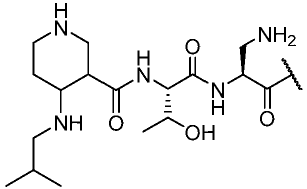 | B | C52H90 N16O12 | 1130.7 | Int 11 | 4-(isobutylamino)pi peridine-3-carbonyl L-Thr-L-Dap-polymyxin B heptapeptide | 5.14 | 566[M+2H]²⁺ |
| D75 | 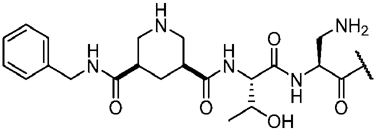 | B | C56H88 N16O13 | 1192.7 | Int 11 | *cis*-5-(benzylcarbamoy l)piperidine-3-carbonyl L-Thr-L-Dap-polymyxin B heptapeptide | 5.58 | 1193.8 [MH+] |
| D76 | 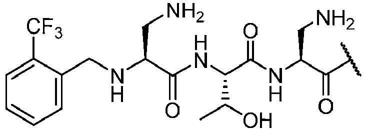 | B | C53H83 F3N16O 12 | 1192.6 | Int 11 | (S)-3-amino-2-((2-trifluoromethybe nzyl)amino)prop anoyl L-Thr-L-Dap-polymyxin B heptapeptide | 5.77 | 597[M+2H]²⁺ |
| D77 | 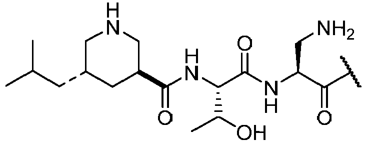 | E | C49H91 N15O12 | 1081.7 | Int 14 | (*trans*-5-(isobutyl -piperidine)-3-carbonyl L-Thr-L-Dap-polymyxin E heptapeptide | 5.52 | 1083[MH+] 542[M+2H]²⁺ |
| D78 | 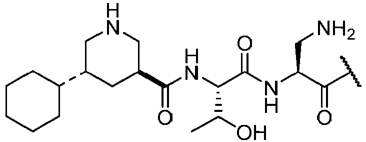 | B | C54H91 N15O12 | 1141.7 | Int 11 | (*trans*-5-(cyclohexyl - piperidine)-3-carbonyl L-Thr-L-Dap-polymyxin B heptapeptide | 5.92 | 1143[MH+] 572[M+2H]²⁺ |
| D79 | 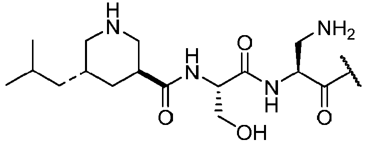 | B | C51H87 N15O12 | 1101.7 | Int 12 | (*trans*-5-(isobutyl -piperidine)-3-carbonyl L-Ser-L-Dap-polymyxin B heptapeptide | 5.61 | 552[M+2H]²⁺ |
| D80 | 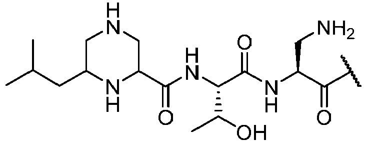 | B | C51H88 N16O12 | 1116.7 | Int 11 | 6-isobutylpiperazin e-2-carbonyl L-Thr-L-Dap-polymyxin B heptapeptide | 5.45 | 1118[MH+] 559[M+2H]²⁺ |
| D81 | 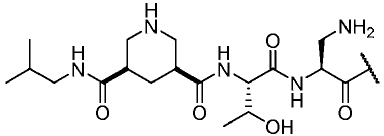 | B | C53H90 N16O13 | 1158.7 | Int 11 | *cis*-5-(isobutylcarbamo yl)piperidine-3-carbonyl L-Thr-L-Dap-polymyxin B heptapeptide | 5.54 | 1159.9 [MH+] |
| D82 | 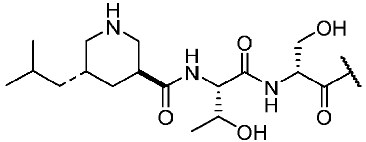 | B | C52H88 N14O13 | 1116.7 | Int 13 | (*trans*-5-(isobutyl -piperidine)-3-carbonyl L-Thr-D-Ser-polymyxin B heptapeptide | 5.49 | 1117[MH+] 559[M+2H]²⁺ |
| D83 | 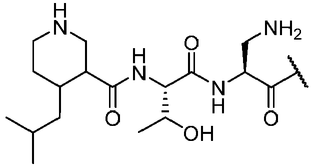 | B | C52H89 N15O12 | 1115.7 | Int 7 | 4-(isobutyl - piperidine)-3-carbonyl L-Thr-L-Dap -polymyxin B heptapeptide | 5.32 | 1116.7[MH+] 559[M+2H]²⁺ |
| D84 | 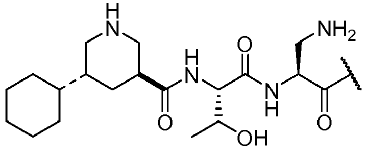 | B | C54H85 N15O12 | 1135.7 | Int 7 | *trans*-5-phenyl - piperidine-3-carbonyl L-Thr-L-Dap-polymyxin B heptapeptide | 5.38 | 1136.6 [MH+] |
| D85 | 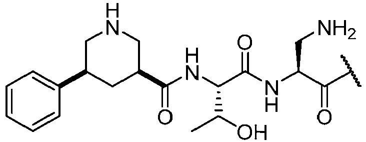 | B | C54H85 N15O12 | 1135.7 | Int 7 | cis-5-cyclohexyl - piperidine-3-carbonyl L-Thr-L-Dap-polymyxin B heptapeptide | 5.55 | 1136.7 [MH+] |
| D86 | 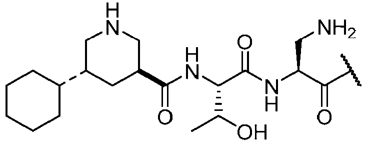 | E | C51H93 N15O12 | 1107.7 | Int 14 | *trans*-5-cyclohexyl - piperidine-3-carbonyl L-Thr-L-Dap-polymyxin E heptapeptide | 5.69 | 1109[MH+] 555[M+2H]²⁺ |
| D87 | 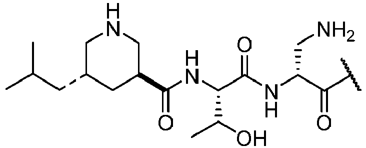 | B | C52H89 N15O12 | 1115.7 | Int 15 | (*trans*-5-(isobutyl -piperidine)-3-carbonyl L-Thr-D-Dap-polymyxin B heptapeptide | 5.82 | 1117[MH+] 559[M+2H]²⁺ |
| D88 | 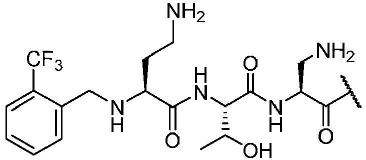 | B | C54H85 F3N16O 12 | 1206.7 | Int 11 | (S)-4-amino-2-((2-trifluoromethybe nzyl)amino)butan oyl L-Thr-L-Dap-polymyxin B heptapeptide | 5.57 | 1207.7[MH+] 605[M+2H]²⁺ |
| D89 | 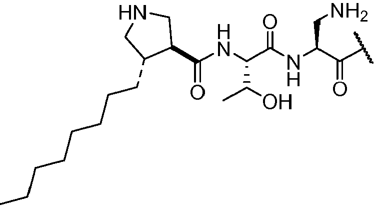 | B | C55H95 N15O12 | 1157.7 | Int 11 | *trans*-4-octylpyrrolidine-3-carbonyl L-Thr-L-Dap-polymyxin B heptapeptide | 6.55 | 1159[MH+] |
| D90 | 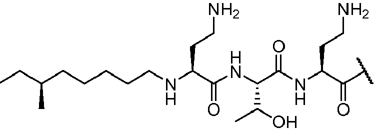 | B | C56H10 0N16O1 2 | 1188.7 | Int 7 | (S)-4-amino-2-(((S)-6-methyloctyl)amin o)butanoyl polymyxin B nonapeptide | 6.34 | 1189.7[MH+] |
| D91 | 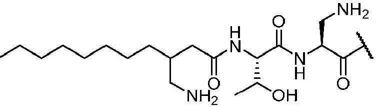 | B | C54H95 N15O12 | 1145.7 | Int 11 | 3-aminomethyl-undecanoyl L-Thr-L-Dap-polymyxin B heptapeptide Isomer 1 | 6.47 | 1146.8[MH+] |
| D92 | 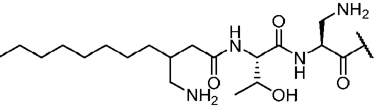 | B | C54H95 N15O12 | 1145.7 | Int 11 | 3-aminomethyl-undecanoyl L-Thr-L-Dap-polymyxin B heptapeptide Isomer 2 | 6.53 | 1146.7[MH+] |
| D93 | 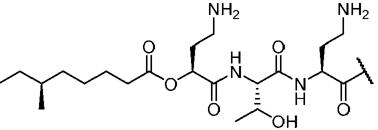 | B | C56H97 N15O14 | 1203.7 | Int 7 | (S)-4-amino-2-(((S)-6-methyloctanoyl)o xy)butanoyl polymyxin B nonapeptide | 6.46 | 1204.8[MH+] 603[M+2H]²⁺ |
| D94 | 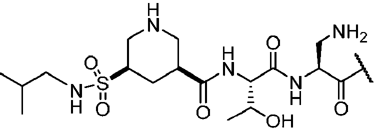 | B | C52H90 N16O14 S | 1194.7 | Int 11 | Cis-5-(N-isobutylsu lfamoyl ) piperidine-3-carbonyl L-Thr-L-Dap-polymyxin B heptapeptide | 5.38 | 1195.8[MH+] |

**Table 4D - Additional Further Example Compounds**

| **Ex**. | **R-** | **Scaffold** | **Formula** | **Mass** | Starting material | **Name** | **HPLC Retention time (min.)** | **m/z** |
|---|---|---|---|---|---|---|---|---|
| D95 | 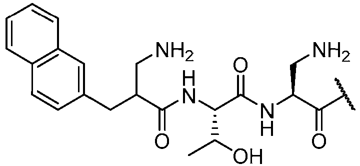 | B | C56H85 N15O12 | 1159.7 | Int 11 | 2-aminomethyl-3-naphthalen-2-yl-propanoyl L-Thr-L-Dap-polymyxin B heptapeptide | 6.04 | 1160.6 [MH⁺] |
| D96 | 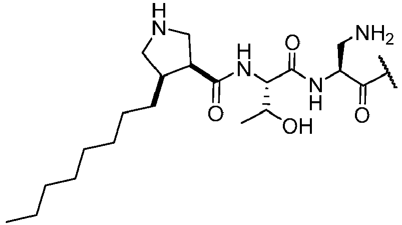 | B | C55H95 N15O12 | 1157.7 | Int 11 | *cis*-4-octylpyrrolidi ne-3-carbonyl L-Thr-L-Dap-polymyxin B heptapeptide | 6.79 | 1158.8 [MH⁺] 579.9 [M+2H]²⁺ |
| D97 | 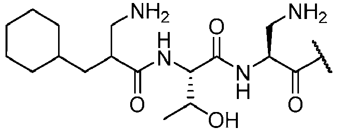 | B | C52H89 N15O12 | 1115.7 | Int 11 | 3-amino-2-(cyclohexylmethyl)pro panoyl L-Thr-L-Dap-polymyxin B heptapeptide | 5.93 | 1116.8 [MH⁺] |
| D98 | 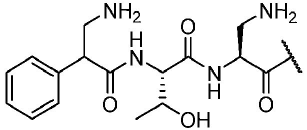 | B | C51H81 N15O12 | 1095.6 | Int 11 | 3-amino-2-phenylpropanoyl L-Thr-L-Dap-polymyxin B heptapeptide | 5.44 | 549[M+2H]²⁺ |
| D99 | 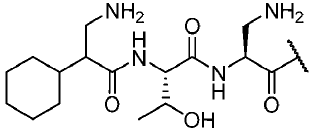 | B | C51H87 N15O12 | 1101.7 | Int 11 | 3-amino-2-cyclohexylpropanoyl L-Thr-L-Dap-polymyxin B heptapeptide | 5.69 | 552 [M+2H]²⁺ |
| D100 | 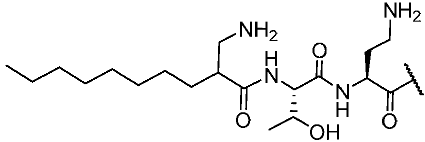 | B | C54H95 N15O12 | 1145.7 | Int 7 | 2-(aminomethyl)-decanoyl polymyxin B nonapeptide | 6.7 | 1146.5 [MH⁺] 573.9 [M+2H]²⁺ |
| D101 | 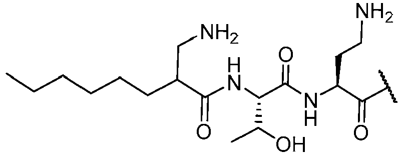 | B | C52H91 N15O12 | 1117.7 | Int 7 | 2-(aminomethyl)-octanoyl polymyxin B nonapeptide | 6.16 | 1119 [MH⁺] |
| D102 | 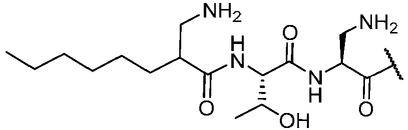 | B | C51H89 N15O12 | 1103.7 | Int 11 | 2-(aminomethyl)-octanoyl L-Thr-L-Dap-polymyxin B heptapeptide | 6.16 | 1105 [MH⁺] |
| D103 | 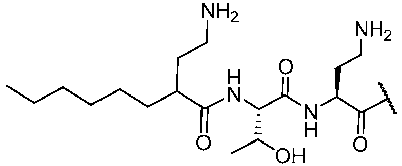 | B | C53H93 N15O12 | 1131.7 | Int 7 | 2-(2-aminoethyl)octanoyl polymyxin B nonapeptide | 6.16 | 1133 [MH⁺] |
| D104 | 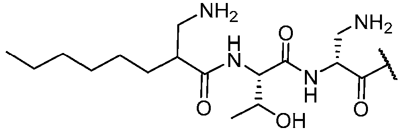 | B | C51H89 N15O12 | 1103.7 | Int 15 | 2-(aminomethyl) octanoyl L-Thr-D-Dap-polymyxin B heptapeptide | 6.40 | 1105 [MH⁺] |
| D105 | 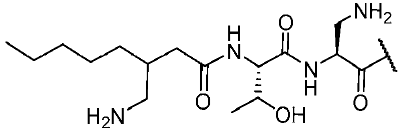 | B | C51H89 N15O12 | 1103.7 | Int 11 | 3-(aminomethyl) octanoyl L-Thr-L-Dap-polymyxin B heptapeptide | 5.89 | 1104.8 [MH⁺] |
| D106 | 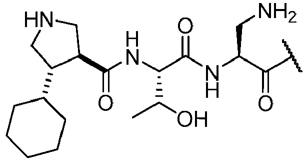 | B | C53H89 N15O12 | 1127.7 | Int 11 | *trans*-4-cyclohexylpyrrolidine-3-carbonyl L-Thr-L-Dap-polymyxin B heptapeptide | 5.9 | 1128 [MH]+ 564 [M+2H]²⁺ |
| D107 | 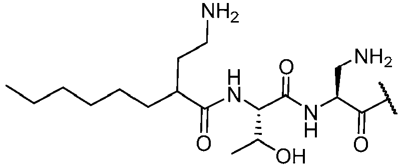 | B | C52H91 N15O12 | 1117.7 | Int 11 | 2-(2-aminoethyl)octanoyl L-Thr-L-Dap-polymyxin B heptapeptide | 5.84 | 1119 [MH⁺] |
| D108 | 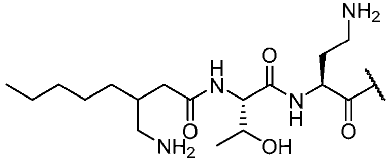 | B | C52H91 N15O12 | 1117.7 | Int 7 | 3-(aminomethyl) octanoyl polymyxin B nonapeptide | 5.45 | 1116.6 [M]⁺ |
| D109 | 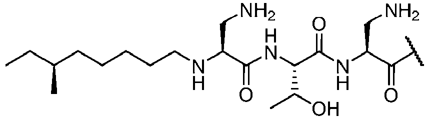 | B | C54H96 N16O12 | 1160.7 | Int 11 | (S)-3-amino-2-(((S )-6-methyloctyl)amino) propanoyl L-Thr-L-Dap-polymyxin B heptapeptide | 6.30 | 1162 [MH]+ 582 [M+2H]²⁺ |
| D110 | 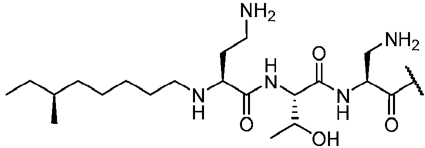 | B | C55H98 N16O12 | 1174.8 | Int 11 | (S)-4-amino-2-(((S )-6-methyloctyl)amino) butanoyl L-Thr-L-Dap-polymyxin B heptapeptide | 6.18 | 1176 [MH]+ 589 [M+2H]²⁺ |
| D111 | 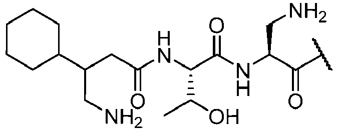 | B | C52H89 N15O12 | 1115.7 | Int 11 | 4-amino-3-cyclohexylbutanoyl L-Thr-L-Dap-polymyxin B heptapeptide isomer 1 | 5.34 | 1116.7 [MH⁺] |
| D112 | 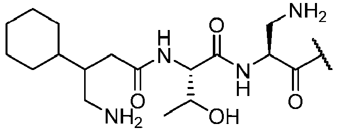 | B | C52H89 N15O12 | 1115.7 | Int 11 | 4-amino-3-cyclohexylbutanoyl L-Thr-L-Dap-polymyxin B heptapeptide isomer 2 | 5.42 | 1116.6 [MH⁺] |
| D113 | 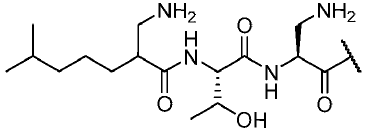 | B | C51H89 N15O12 | 1103.7 | Int 11 | 2-(aminomethyl )-6-methylheptanoyl L-Thr-L-Dap-polymyxin B heptapeptide | 5.75 | 553 [M+2H]²⁺ |
| D114 | 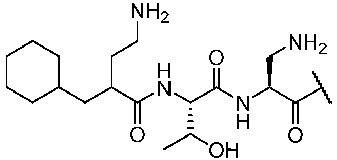 | B | C53H91 N15O12 | 1129.7 | Int 11 | 4-amino-2-(cyclohexyl methyl) butanoyl L-Thr-L-Dap-polymyxin B heptapeptide | 5.73 | 1130.7 [MH⁺] |
| D115 | 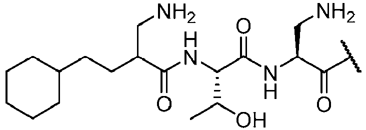 | B | C54H95 N15O12 | 1130.4 | Int 11 | 2-(aminomethyl )-4-cyclohexylbutanoyl L-Thr-L-Dap-polymyxin B heptapeptide | 6.16 | 1131 [MH]⁺, 566[M+2H]²⁺ |
| D116 | 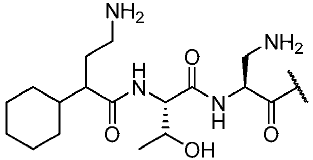 | B | C52H89 N15O12 | 1115.7 | Int 11 | 4-amino-2-cyclohexylbutanoyl L-Thr-L-Dap-polymyxin B heptapeptide | 5.61 | 1116.7 [MH]⁺ 559[M+2H]²⁺ |
| D117 | 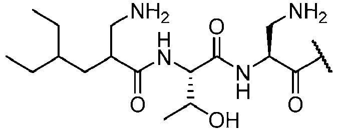 | B | C51H89 N15O12 | 1103.7 | Int 11 | 2-(aminomethyl )-4-ethylhexanoyl L-Thr-L-Dap-polymyxin B heptapeptide | 5.80 | 1105[MH]⁺ |

### Biological Activity

To evaluate the potency and spectrum of the compounds both alone and in combination with another agent, susceptibility testing was performed against at least two strains of each of the four gram negative pathogens, *Escherichia coli, Pseudomonas aeruginosa, Klebsiella pneumoniae* and *Acinetobacter baumannii.*

### MIC Determination

The inoculum was prepared by making a direct suspension of isolated colonies (selected from an 18-24 hour Mueller-Hinton agar plate) adjusted to the 0.5 McFarland standard.

MIC testing was performed by two-fold serial antibiotic dilutions in cation-adjusted Mueller-Hinton Broth in sterile 96-well microtitre plates in a total volume of 170 µL (150 µL broth containing the antimicrobial agent, 20 µL inoculum). The assays were performed in duplicate. Plates were incubated aerobically without shaking for 18-20 hours at 35°C with the MIC defined as the lowest concentration of drug that prevented visible growth.

In cases where the duplicate values varied by less than 2-fold, the lower of the two values is reported. If a variation of greater than 2-fold was observed, the assay was considered non-valid. Several of the compounds were subjected to multiple tests, and where this is the case, the MIC reflects the modal value obtained.

### Combination Activity

Table 5C below shows the activity, shown by way of MIC values, of Example Compounds in the presence of Rifampicin. MIC values were determined against *E. coli* and *K. pneumoniae* in the presence of a fixed concentration (2 µg/mL) of Rifampicin, and against *A. baumannii* in the presence of 0.25 µg/mL of rifampicin for the example compounds and the comparator compounds C1 and C2 and CC3.

CB-182,804 (C2) is a polymyxin decapeptide derivative with an aryl urea substituent at the N-terminus, which has been claimed to have lower toxicity than Polymyxin B (shown as compound 5 in WO 2010/075416), and was prepared by the present inventors. C1 was also prepared in-house, and corresponds to NAB-739 (as described by Vaara in, for example, WO 2008/017734). CC3 corresponds to compound 5x in Magee et al., J. Med. Chem. 2013, 56, 5079.

**Table 5C - MIC Values (micrograms/mL) for Example Compounds in combination with Rifampicin (2 µg/mL or 0.25 µg/mL).**

| | ***E. coli*** | | ***K. pneumoniae*** | | ***A. baumannii*** | |
|---|---|---|---|---|---|---|
| | *NCTC 13441* | *ATCC* 25922 | *ATCC BAA-*2146 | *ATCC 4352* | NCTC 13424 | ATCC BAA-747 |
| PMB | 0.03 | ≤0.015 | 0.25 | 0.06 | ND | 0.03 |
| C1 | 1 | 1 | 2 | 1 | ND | 0.25 |
| C2 | 0.25 | ≤0.015 | ND | 0.06 | 1 | 0.125 |
| CC3 | 0.06 | 0.06 | 0.25 | 0.06 | 0.03 | ND |
| D9 | ≤0.015 | ≤0.015 | 0.125 | 0.03 | ND | ≤0.015 |
| D11 | 0.06 | ≤0.015 | 1 | 0.06 | 0.5 | 0.06 |
| D15 | 0.03 | ≤0.015 | 0.125 | 0.03 | ≤0.015 | ≤0.015 |
| D25 | 0.03 | ≤0.015 | 0.06 | ≤0.015 | ND | ≤0.015 |

Table 5D shows the MIC values for Further Example Compounds in the presence of Rifampicin. MIC values were determined against *E. coli* and *K. pneumoniae* in the presence of a fixed concentration (2 µg/mL) of Rifampicin, and against *A. baumannii* in the presence of 0.25 µg/mL of rifampicin.

**Table 5D - MIC Values (micrograms/mL) for Further Example Compounds in combination with Rifampicin (2 µg/mL or 0.25 µg/mL).**

| | *E. coli* | | *K. pneumoniae* | | *A. baumannii* | |
|---|---|---|---|---|---|---|
| Example | *NCTC 13441* | *ATCC* 25922 | *ATCC BAA-*2146 | *ATCC* 4352 | NCTC 13424 | ATCC BAA-747 |
| D2 | 0.03 | ≤0.015 | 0.125 | 0.03 | ≤0.015 | 0.03 |
| D5 | 0.06 | 0.03 | 0.25 | 0.03 | ≤0.015 | 0.25 |
| D6 | 0.03 | ≤0.015 | 0.06 | ND | ≤0.015 | 0.06 |
| D7 | 0.5 | 0.06 | 0.5 | 0.125 | 0.03 | 1 |
| D8 | ND | ≤0.015 | 0.06 | 0.03 | ≤0.015 | 0.03 |
| D9 | 0.06 | ≤0.015 | 0.06 | 0.03 | 0.03 | ≤0.015 |
| D10 | 0.5 | ND | 0.25 | 0.125 | ≤0.015 | 0.5 |
| D11 | 0.06 | 0.125 | 0.5 | 0.125 | 0.25 | 0.125 |
| D12 | 0.25 | 0.06 | 0.25 | 0.125 | 0.03 | 0.5 |
| D13 | 0.125 | 0.06 | 0.125 | 0.125 | 0.06 | 0.125 |
| D15 | 0.03 | ≤0.015 | 0.125 | ≤0.015 | ≤0.015 | ≤0.015 |
| D25 | 0.03 | ≤0.015 | 0.06 | ≤0.015 | ND | ≤0.015 |
| D26 | 1 | 0.25 | 4 | 0.5 | 1 | 2 |
| D27 | ND | 0.125 | 4 | 0.5 | 1 | 1 |
| D28 | 0.5 | 1 | 2 | 0.5 | 1 | 1 |
| D29 | ≤0.015 | ≤0.015 | 0.125 | 0.03 | 0.06 | 0.03 |

Table 5E shows the MIC values (micrograms/mL) recorded for Further Examples (D50 and D65) and polymyxin each tested in a 1:1 (wt:wt) combination with Rifampicin.

**Table 5E - MIC values (microgram/mL) for test compounds in 1:1 combination with rifampicin**

| | *E. coli* | | | | *K. pneumoniae* | | | *P*. *aeruginosa* | *A. baumannii* | |
|---|---|---|---|---|---|---|---|---|---|---|
| | 058 | 059 | 060 | ATCC 25922 | CCUG 59348 | 062 | 063 | 068 | 053 | 056 |
| PMB | 1 | ≤0.5 | 1 | ≤0.5 | 1 | 1 | 16 | 1 | ≤0.5 | ≤0.5 |
| D50 | ND | ≤0.5 | ≤0.5 | ≤0.5 | ≤0.5 | 1 | 32 | 1 | ≤0.5 | ND |
| D65 | ≤0.5 | ≤0.5 | ≤0.5 | ≤0.5 | 1 | 1 | 16 | 1 | ≤0.5 | ≤0.5 |

MIC values (microgram/mL) refer to the total drug concentration (i.e. test agent plus Rifampicin).

### In Vitro Renal Cell Toxicity Assay

The renal cell toxicity of the compounds was assessed in an *in vitro* assay using the HK-2 cell line, an immortalized proximal tubule cell line derived from a normal human kidney. The endpoint to describe the toxicity of the compounds was the reduction of resazurin correlating with the metabolic activity of the cells. Cells were cultured in 150 cm² flasks in 25 mL supplemented KSF (with 5 ng/mL EGF and 50 µg/mL BPE). Cells were maintained at 70% confluence with a maximum of 25 passages.

Day 1: Media was removed and cells were washed with 10ml DPBS. Six ml of a 0.25% trypsin solution with EDTA was then added to the flask and the cells returned to the incubator. After 1 to 2 minutes incubation, 14 mL media was added to the flask to inactivate the trypsin. The cell suspension was transferred to a centrifuge tube and the cells pelleted at 1000 rpm for 6 minutes. The cell pellet was then resuspended in fresh media supplemented with EGF and BPE. The cell number was counted and cells were diluted to 46875 cells/mL in fresh medium supplemented with epidermal growth factor (EGF) and bovine pituitary extract (BPE).. 7500 cells were dispensed in each well in a volume of 160µl and incubated at 37°C for 24 h.

Day 2: Test compounds were prepared directly into the media, or from stock solutions to result in no more than 0.5% DMSO, or 5% water in the final assay. Nine point concentrations were prepared from 1000 µg/mL to 1.95 µg/mL in two-fold dilutions in fresh medium. The microtiter plates were removed from the incubator and the media replaced with 100 µL of the dilutions of the compound solution. Every set of concentration was done in triplicate, and positive and negative controls were added to each plate. The plates were then incubated for 24h at 37°C with 5% CO₂ in a humidified atmosphere.

Day 3: The reagent containing the resazurin (CellTiter-Blue, Promega) was diluted in PBS (1:4) and added at 20% (v/v) to each well. The plates were then incubated at 37°C for 2h before the fluorescent reduction product was detected.

Media only background values were subtracted before the data was analysed using GraphPad Prism. Compound concentration values were plotted as log values to enable a dose-response curve to be fitted and IC₅₀ values determined.

The data is shown in Table 6, expressed relative to polymyxin B.

### Sole Activity

Table 6B below shows the activity, shown by way of MIC values, of Example Compounds in the absence of a second agent together with the toxicity against the HK-2 cell line (measured in relation to IC₅₀ values and expressed relative to the value for polymyxin B)

MICs were determined as described herein. HK-2 cell IC₅₀ values were determined as described herein. Values are reported relative to Polymyxin B.

The MIC values were compared to compounds known from the literature. The data is in table 6B. C2 (CB-182,804) is a polymyxin decapeptide derivative described by Cubist (shown as compound 5 in WO 2010/075416), C1 is NAB-739 (as described by Vaara in, for example, WO 2008/01773). CC3 corresponds to Compound 5x Magee et al, J. Med. Chem., 2013, 56, 5079.

Table 6B - MIC Values (micrograms/mL) and HK-2 cell toxicity for Example Compounds (measured in relation to IC₅₀ values and expressed relative to the value for polymyxin B)

| Ex. | *E. coli* | | *K. pneumoniae* | | *P. aeruginosa* | | *A. baumannii* | | HK-2 IC₅₀ rel to PMB |
|---|---|---|---|---|---|---|---|---|---|
| | NCTC 13441 | ATCC 25922 | ATCC BAA-2146 | ATCC 4352 | CCUG 59347 | ATCC 27853 | NCTC 13424 | ATCC BAA-747 | |
| PMB | 0.25 | 0.25 | 0.5 | 0.25 | 0.5 | 0.5 | 0.5 | 0.25 | 1 |
| C1 | 1 | 2 | 2 | ND | >8 | 8 | 2 | ND | 17 |
| C2 | 2 | 1 | 2 | 1 | 1 | 0.5 | 0.5 | 0.5 | 2 |
| CC3 | 1 | 2 | ND | 0.5 | 1 | 1 | 1 | 0.5 | 17 |
| D1 | 4 | 4 | 1 | 2 | 2 | 0.5 | 4 | 4 | 5.7 |
| D2 | 0.5 | 1 | 0.25 | ND | 0.5 | 0.25 | 1 | 2 | 7.6 |
| D3 | 0.5 | 0.25 | 2 | 1 | 1 | 0.5 | >8 | >8 | ND |
| D4 | 1 | 0.5 | ND | ND | 0.5 | 0.25 | 2 | 4 | 7.5 |
| D5 | >8 | 8 | >8 | 8 | >8 | 1 | >8 | >8 | 15.3 |
| D6 | 0.25 | 0.25 | 0.5 | 0.25 | 0.5 | 0.25 | 1 | 1 | 3.7 |
| D7 | >8 | >8 | >8 | 8 | >8 | 2 | >8 | >8 | ND |
| D8 | 0.25 | 0.125 | 0.5 | 0.125 | 1 | 0.5 | 0.5 | 0.25 | ND |
| D9 | 0.25 | 0.25 | 0.5 | 0.25 | 1 | 0.25 | 0.5 | 1 | 11.7 |
| D10 | 4 | 4 | >8 | 8 | 2 | 1 | 8 | >8 | 4.4 |
| D11 | 0.25 | 0.25 | 0.25 | 0.25 | 0.5 | 0.5 | 0.5 | 1 | 2.5 |
| D12 | >8 | >8 | >8 | >8 | 4 | 1 | >8 | >8 | ND |
| D13 | 0.125 | 0.25, 1 | 0.25 | 0.125 | 0.5 | 0.5 | 0.125 | 0.5 | 4.1 |
| D14 | >8 | >8 | >8 | >8 | 4 | 1 | >8 | >8 | ND |
| D15 | 0.5 | 0.25 | ND | ND | 0.5 | 0.25 | 2 | 2 | 17 |
| D16 | >8 | >8 | >8 | >8 | 1 | 8 | >8 | >8 | ND |
| D17 | 2 | 2 | 2 | 2 | 0.5 | 0.5 | 4 | 2 | ND |
| D18 | 4 | 4 | >8 | 8 | 2 | 4 | >8 | >8 | ND |
| D19 | 2 | 2 | 4 | 2 | 1 | 0.5 | ND | 2 | ND |
| D20 | 4 | 4 | >8 | 8 | 1 | 0.5 | >8 | >8 | ND |
| D21 | 0.5 | 0.5 | 1 | 0.25 | 0.5 | 0.5 | 2 | 0.5 | ND |
| D22 | 4 | 4 | >8 | ND | 1 | 2 | >8 | >8 | ND |
| D23 | 1 | ND | 1 | ND | 0.5 | 0.5 | 0.25 | 0.25 | 0.8 |
| D24 | >8 | >8 | >8 | >8 | >8 | 1 | >8 | >8 | ND |
| D25 | 0.5 | 0.25 | 0.5 | 0.25 | 0.5 | 0.25 | 1 | 0.5 | 8 |
| D26 | 2 | 0.5 | 2 | 1 | 0.5 | 0.5 | 2 | 0.5 | ND |
| D27 | 0.06 | 0.125 | 0.5 | 0.25 | 0.5 | 0.5 | 1 | 0.25 | 1 |
| D28 | 0.125 | 0.125 | 0.5 | 0.25 | 1 | 0.5 | 1 | 0.25 | 1.3 |
| D29 | 1 | 1 | 0.5 | 0.5 | 1 | 0.5 | 0.5 | 0.5 | ND |
| D30 | 2 | 2 | ND | 1 | 1 | 1 | 2 | 1 | ND |
| D31 | 1 | 2 | ND | 0.5 | 1 | 0.5 | 2 | 1 | 1.8 |
| D32 | 1 | 1 | ND | 1 | 1 | 0.5 | 1 | 2 | ND |
| D33 | 2 | 1 | ND | 2 | 1 | 0.5 | 2 | 1 | ND |
| D34 | 1 | 1 | 2 | 2 | 2 | 1 | 2 | 2 | ND |
| D35 | 1 | 1 | 2 | 2 | 1 | 1 | 1 | 2 | ND |
| D36 | 0.25 | 0.06 | ND | 0.25 | 1 | 0.25 | 0.25 | 0.5 | 4.5 |
| D37 | 2 | 0.5 | 1 | 0.5 | 1 | 0.5 | 1 | 1 | ND |
| D38 | 8 | 8 | 8 | 4 | 2 | 1 | 2 | >8 | 6.3 |
| D39 | 0.5 | 0.125 | 0.25 | 0.125 | 1 | 0.25 | 0.25 | 0.5 | 5.4 |
| D40 | 2 | 2 | 4 | 2 | 1 | 0.5 | 8 | >8 | ND |
| D41 | 1 | 1 | ND | 2 | 1 | 0.5 | 1 | 2 | ND |
| D42 | 0.25 | 0.25 | 0.5 | 0.125 | 0.5 | 0.25 | 0.5 | 0.25 | 6.6 |
| D43 | 4 | 2 | 4 | 2 | 2 | 1 | 4 | 4 | ND |
| D44 | 0.5 | 1 | 0.5 | ND | 0.5 | 0.25 | ND | 0.25 | 1.3 |
| D45 | 2 | 0.5 | 2 | 0.5 | 4 | 1 | 4 | 8 | ND |
| D46 | 1 | 1 | 1 | 0.5 | 1 | 0.5 | 2 | 1 | ND |
| D47 | 8 | 4 | 8 | 4 | >8 | 2 | >8 | >8 | ND |
| D48 | 0.125 | 0.125 | 0.25 | 0.125 | 1 | 0.25 | 0.5 | 0.5 | ND |
| D49 | 8 | 8 | 8 | 4 | 4 | 2 | >8 | >8 | ND |
| D50 | 0.06 | 0.06 | 0.125 | 0.06 | 1 | 0.5 | 0.25 | 0.25 | 7.4 |
| D51 | 0.5 | 0.5 | 0.5 | 0.125 | 1 | 0.5 | 1 | 1 | 34 |
| D52 | 0.125 | 0.125 | 0.25 | 0.125 | 0.5 | 0.5 | 0.25 | 0.5 | ND |

### Further Sole Activity

The Further Synthesis Examples were tested against a range of Gram negative bacteria, and tested for HK-2 cell toxicity. The data is contained in Table 6C. All the worked examples, including the Synthesis and Further Synthesis Examples, were also tested against a range of Gram negative bacteria that are resistant to Polymyxin B (i.e. those strains where Polymyxin B has an elevated MIC value). The data is contained in Table 6D.

In the tables ND is not determined.

**Table 6C - MIC Values (micrograms/mL) and HK-2 cell toxicity (measured in relation to IC₅₀ values and expressed relative to the value for polymyxin B) for additional Example Compounds.**

| Ex. | *E. coli* | | *K. pneumoniae* | | *P. aeruginosa* | | *A. baumannii* | | HK-2 IC₅₀ rel to PMB* |
|---|---|---|---|---|---|---|---|---|---|
| | NCTC 13441 | ATCC 25922 | ATCC BAA-2146 | ATCC 4352 | CCUG 59347 | ATCC 27853 | NCTC 13424 | ATCC BAA-747 | |
| D53 | 1 | 1 | 1 | 0.25 | 1 | 0.5 | 2 | 1 | 18.2 |
| D54 | 0.5 | 0.25 | 0.25 | 0.125 | 1 | 0.5 | 4 | 1 | 5.8 |
| D55 | 0.25 | 0.25 | 0.5 | 0.25 | 1 | 0.5 | 0.5 | 0.25 | ND |
| D56 | 0.25 | 0.25 | 0.25 | 0.125 | 1 | 0.5 | 0.5 | 0.5 | 22 |
| D57 | 0.5 | 0.25 | 0.25 | 0.25 | 1 | 0.5 | 2 | 4 | ND |
| D58 | 0.25 | 0.25 | 0.25 | 0.25 | 1 | 0.5 | 0.5 | 1 | ND |
| D59 | 0.5 | 0.5 | 0.5 | 0.5 | 1 | 0.5 | 1 | 2 | 6.6 |
| D60 | 0.5 | 0.25 | 0.5 | 0.25 | 2 | 1 | 2 | 4 | ND |
| D61 | 0.5 | 0.5 | 0.25 | 0.25 | 1 | 0.5 | 1 | 2 | ND |
| D62 | 1 | 0.5 | 1 | 0.5 | 2 | 1 | 4 | 4 | ND |
| D63 | 0.125 | 0.06 | 0.125 | 0.125 | 0.5 | 0.5 | 0.25 | 0.25 | 6.3 |
| D64 | 0.25 | 0.25 | 0.25 | 0.25 | 1 | 1 | 2 | 2 | 11 |
| D65 | 0.06 | 0.03 | 0.125 | 0.03 | 0.25 | 0.25 | 0.125 | 0.06 | 8.2 |
| D66 | 0.125 | 0.125 | 0.06 | 0.125 | 0.5 | 0.5 | 0.25 | 0.25 | 4.6 |
| D67 | 0.125 | 0.06 | 0.06 | 0.03 | 0.5 | 0.25 | 0.25 | 0.5 | 5.1 |
| D68 | 0.25 | 0.25 | ND | 0.125 | 0.5 | 0.5 | 0.5 | 0.25 | 5.3 |
| D69 | 0.125 | 0.25 | ND | 0.125 | 0.5 | 0.5 | 0.5 | 0.25 | 16.6 |
| D70 | 0.125 | 0.125 | ND | 0.06 | 0.5 | 0.25 | 0.125 | 0.06 | 6.3 |
| D71 | 0.25 | 0.25 | 0.5 | 0.25 | 1 | 0.5 | 0.25 | 0.5 | 2.9 |
| D72 | 0.25 | 0.25 | 0.25 | 0.125 | 1 | 0.5 | ND | 0.125 | 2.2 |
| D73 | 0.5 | 0.25 | 0.5 | 0.5 | 2 | 1 | 2 | 4 | 11.3 |
| D74 | 0.25 | 0.25 | 0.5 | 0.125 | 0.5 | 0.25 | 2 | 2 | 29.6 |
| D75 | 0.125 | 0.06 | 0.125 | 0.06 | 0.5 | 0.5 | 0.125 | 0.06 | 10.1 |
| D76 | 0.25 | 0.125 | 0.125 | 0.06 | 0.25 | 0.25 | 0.25 | 0.06 | 3.1 |
| D77 | 0.06 | 0.06 | 0.125 | 0.125 | 1 | 0.5 | 0.06 | 0.06 | 13.3 |
| D78 | 0.125 | 0.125 | 0.125 | 0.06 | 0.5 | 0.5 | 0.06 | 0.06 | 5.2 |
| D79 | 0.125 | 0.125 | 0.125 | 0.06 | 0.25 | 0.25 | 0.125 | 0.06 | 7.5 |
| D80 | ND | 0.5 | ND | 0.25 | 0.5 | 0.5 | 0.5 | 0.5 | ND |
| D81 | ND | 0.06 | 0.06 | 0.06 | 0.5 | 0.25 | 0.06 | 0.03 | ND |
| D82 | ND | 0.125 | ND | 0.06 | 4 | 2 | 0.125 | 0.06 | ND |
| D83 | 0.06 | 0.125 | ND | 0.06 | 0.5 | 0.25 | 0.125 | 0.06 | 7.1 |
| D84 | 0.06 | 0.25 | ND | 0.125 | 0.5 | 0.5 | 0.25 | 0.06 | ND |
| D85 | 0.125 | 0.06 | ND | 0.06 | 0.25 | 0.125 | 0.06 | ≤0.015 | 6.6 |
| D86 | ND | 0.125 | ND | 0.03 | 0.5 | 0.25 | 0.06 | 0.03 | ND |
| D87 | ND | 0.03 | ND | 0.06 | 1 | 0.25 | 0.03 | 0.03 | 18.9 |
| D88 | ND | 0.06 | ND | 0.06 | 0.5 | 0.125 | 0.06 | 0.06 | ND |
| D89 | 0.125 | 0.5 | 0.5 | ND | 1 | 0.5 | 0.5 | 0.5 | 2.6 |
| D90 | ND | 0.25 | ND | 0.25 | 0.25 | 0.5 | 0.25 | 0.25 | 0.7 |
| D91 | ND | 0.5 | ND | 0.25 | 0.5 | 0.5 | 0.5 | 0.25 | ND |
| D92 | ND | 0.5 | ND | 0.25 | 0.5 | 0.25 | 0.25 | 0.5 | 2.7 |
| D93 | ND | 0.25 | ND | 0.25 | 1 | 0.25 | 0.5 | 0.25 | ND |
| D94 | ND | 0.03 | ND | ND | 1 | 0.5 | 0.06 | 0.06 | ND |

| | | | | | | | | | |
|---|---|---|---|---|---|---|---|---|---|
| * Figures represent the mean value obtained from up to three independent studies. | | | | | | | | | |

**Table 6D - MIC Values (micrograms/mL) for Example Compounds and Comparator compounds polymyxin B, C1, C2, and CC3 against strains of E. coli. K. pneumoniae, P. aeruginosa and A. baumannii with elevated MICs to Polymyxin B**

| Ex. | *E. coli* | | | | *K. pneumoniae* | | | | | | | *P. aeruginosa* | | *A. baumannii* | |
|---|---|---|---|---|---|---|---|---|---|---|---|---|---|---|---|
| | 058 | 059 | 060 | 061 | 655 | 062 | 063 | 064 | 065 | 066 | 067 | 068 | 070 | 053 | 056 |
| PMB | 4 | 4 | 4 | 16 | 32 | 128 | 32 | 8 | 4 | 8 | 128 | 8 | 32 | 128 | 32 |
| C1 | ND | 32 | 32 | 32 | 64 | 16 | 64 | 8 | ND | 8 | >64 | 64 | 32 | >64 | >64 |
| C2 | 16 | 32 | 16 | 64 | 64 | >128 | 64 | 32 | ND | 16 | >128 | 32 | 128 | 64 | 32 |
| CC3 | 32 | 32 | 64 | 32 | 32 | >256 | 64 | 8 | 16 | 16 | >128 | 8 | 16 | 64 | 8 |
| D20 | ND | 4 | 16 | 32 | >64 | >64 | >64 | >64 | ND | 64 | >64 | 32 | 64 | >64 | >64 |
| D21 | 1 | 0.5 | 1 | 2 | 32 | 2 | 32 | 1 | ND | 2 | 4 | 1 | 2 | 8 | 4 |
| D22 | ND | 8 | 16 | 64 | >256 | >64 | >64 | >64 | ND | >64 | >64 | 4 | 4 | >64 | >64 |
| D23 | 0.5 | 0.25 | 1 | 1 | 4 | 4 | 4 | 1 | 0.5 | 1 | 8 | 1 | 1 | 2 | 0.5 |
| D25 | 16 | 64 | 16 | 64 | 128 | 256 | 128 | 32 | ND | 64 | >128 | 64 | 128 | 128 | 128 |
| D26 | ND | 0.5 | 2 | 4 | 4 | 2 | 4 | 0.5 | ND | 1 | 8 | 2 | 2 | 8 | 4 |
| D27 | 2 | 2 | 4 | 16 | 64 | 16 | 64 | 16 | ND | 16 | >64 | 2 | 4 | 64 | 32 |
| D28 | 0.5 | 0.5 | 2 | 4 | 16 | 8 | 16 | 2 | 0.5 | 4 | 32 | 4 | 4 | 32 | 16 |
| D30 | 1 | 1 | 1 | 4 | 2 | ND | 4 | 1 | 0.5 | 0.5 | 16 | 2 | 1 | 16 | 8 |
| D31 | 2 | 8 | 4 | 16 | >256 | 64 | >128 | 64 | 16 | 32 | >64 | 4 | 4 | >128 | 64 |
| D32 | ND | 1 | 2 | 8 | 32 | 8 | 32 | 2 | ND | 4 | 32 | 4 | 8 | 32 | 8 |
| D33 | ND | 2 | 2 | 8 | 32 | 2 | 16 | 2 | ND | 4 | 32 | 4 | 4 | 32 | 16 |
| D35 | 1 | 0.5 | 2 | 4 | 4 | 1 | 2 | 1 | 0.25 | 1 | 4 | 1 | 1 | 32 | 8 |
| D36 | 16 | 16 | 16 | 32 | 128 | 256 | 128 | 32 | 16 | 32 | >256 | 64 | >128 | >256 | >256 |
| D42 | 8 | 16 | 16 | 32 | 64 | >256 | 64 | 16 | 4 | 32 | >256 | 16 | 128 | >256 | 256 |
| D44 | 2 | ND | ND | ND | 32 | ND | ND | 2 | 1 | 0.5 | 64 | ND | ND | ND | ND |
| D50 | 4 | 8 | 4 | 8 | 16 | 64 | 16 | 4 | 6 | 4 | >256 | 8 | 16 | 128 | 32 |
| D63 | 16 | ND | ND | 32 | ND | 128 | ND | 4 | 4 | 32 | >256 | ND | ND | ND | 128 |
| D64 | 64 | 128 | 64 | 64 | ND | >256 | >256 | 128 | 64 | 64 | >256 | 128 | >256 | >256 | 256 |
| D65 | 1 | 2 | 1 | 2 | 4 | 32 | 8 | 2 | 2 | 1 | 64 | 2 | 4 | 16 | 1 |
| D66 | 8 | 16 | 8 | 32 | ND | 128 | 64 | 32 | 16 | 32 | >256 | 32 | ND | >256 | 128 |
| D67 | 8 | 64 | 16 | ND | ND | ND | 128 | ND | 4 | ND | ND | 64 | ND | >256 | ND |
| D68 | 8 | 16 | 8 | 32 | ND | 64 | 64 | 16 | 16 | 16 | >256 | 16 | ND | >256 | 128 |
| D69 | 8 | 16 | 8 | 32 | ND | ND | 128 | 32 | 16 | 32 | >256 | 32 | ND | >256 | 256 |
| D70 | 4 | 8 | 4 | 16 | 32 | 64 | 64 | 6 | 4 | 16 | >256 | 8 | 32 | >256 | 16 |
| D71 | 1 | 8 | 4 | 8 | 32 | 32 | 16 | 4 | 0.5 | 1 | 16 | 4 | 8 | 64 | 16 |
| D72 | ND | 16 | 8 | 16 | 64 | 64 | 32 | ND | ND | ND | ND | 64 | 128 | >128 | 128 |
| D75 | 4 | 8 | 4 | 8 | 64 | 128 | 64 | 8 | 4 | 8 | ND | 16 | 32 | 64 | 8 |
| D76 | 4 | 6 | 2 | 8 | 16 | 64 | 16 | 2 | 2 | 2 | 64 | 8 | 16 | 64 | 16 |
| D77 | 2 | 4 | 1 | 4 | 16 | 32 | 16 | 4 | 4 | 2 | 128 | 4 | 4 | 64 | ND |
| D78 | 0.5 | 0.5 | 1 | 1 | 4 | ND | 4 | 1 | 1 | 0.5 | 32 | 1 | 1 | 8 | ≤0.25 |
| D79 | 8 | 8 | 4 | 8 | 128 | 64 | >128 | 8 | 16 | 8 | >128 | 8 | 16 | 128 | 32 |
| D81 | 8 | 16 | 8 | 32 | 64 | ND | 64 | 16 | 2 | 16 | >128 | 32 | 32 | 128 | 32 |
| D82 | 4 | 4 | 4 | 8 | 8 | 32 | 16 | 1 | 0.5 | ND | 64 | 32 | 32 | 16 | 8 |
| D83 | 4 | 8 | 2 | 8 | 16 | 32 | 16 | 2 | 2 | 2 | 32 | 8 | 16 | >64 | 32 |
| D84 | 4 | 8 | 4 | 8 | 16 | >64 | 32 | 8 | 8 | 2 | >64 | 8 | 16 | >64 | 32 |
| D85 | 2 | 16 | 4 | 16 | 32 | ND | 64 | 16 | 4 | 16 | >128 | 8 | 8 | 64 | 4 |
| D86 | 0.5 | 1 | 1 | 4 | 4 | 1 | 8 | 1 | 0.5 | 0.25 | 32 | 1 | 2 | 16 | ≤0.25 |
| D87 | 1 | 1 | 1 | 4 | 4 | 8 | 8 | 0.5 | 0.5 | 0.25 | 64 | 2 | 2 | 32 | 2 |
| D88 | 2 | 8 | 4 | 16 | 16 | 32 | 16 | 1 | 0.25 | 1 | >64 | 8 | 16 | >64 | 32 |
| D89 | 1 | 1 | 0.5 | 2 | 8 | 8 | 4 | 1 | 0.5 | 1 | 16 | 0.5 | 0.5 | 8 | 4 |
| D90 | 2 | 2 | 2 | 8 | 16 | 16 | 32 | 4 | 0.25 | 8 | 32 | 2 | 2 | 32 | 8 |
| D91 | 1 | 0.5 | 2 | 4 | 8 | 8 | 16 | 4 | ND | 2 | 16 | 2 | 1 | 8 | 2 |
| D92 | 0.5 | 0.5 | 1 | 4 | 4 | 2 | 8 | 2 | 0.5 | 1 | 8 | 1 | 1 | 8 | 4 |
| D93 | 8 | ND | ND | ND | ND | ND | ND | ND | 2 | ND | ND | ND | ND | ND | ND |
| D94 | 16 | ND | ND | ND | ND | ND | ND | ND | 32 | ND | ND | ND | ND | ND | ND |

**Table 6E - MIC Values (micrograms/mL) and HK-2 cell toxicity relative to PMB for additional example compounds. PMB is included as a comparator.**

| | *E.coli* | | *K. pneumoniae* | | *P. aeruginosa* | | *A. baumannii* | | HK-2 rel to PMB |
|---|---|---|---|---|---|---|---|---|---|
| **Ex.** | CCUG 59342 | ATCC 25922 | ATCC BAA-2146 | ATCC 4352 | CCUG 59347 | ATCC 27853 | NCTC 13424 | ATCC BAA-747 | |
| PMB | 0.125 | 0.25 | 0.5 | 0.25 | 0.5 | 0.5 | 0.5 | 0.25 | 1 |
| 95 | ND | 0.125 | ND | 0.06 | 0.5 | 0.25 | 0.25 | 0.06 | >3.3 |
| 96 | 0.25 | 0.5 | 0.5 | 0.5 | 1 | 0.5 | 0.5 | 0.5 | 2.3 |
| 97 | ND | 0.25 | ND | 0.125 | 0.5 | 0.25 | 0.125 | ND | 13.3 |
| 98 | ND | 0.25 | ND | 0.125 | 0.25 | 0.125 | 0.5 | 0.5 | ND |
| 99 | ND | ND | ND | 0.03 | 0.25 | 0.25 | 0.125 | 0.06 | ND |
| 100 | 0.25 | 0.25 | 0.5 | 0.25 | 0.5 | 0.25 | 0.25 | 0.25 | 1.6 |
| 101 | 0.06 | ND | 0.06 | 0.06 | 0.25 | 0.125 | 0.125 | 0.06 | 4.2 |
| 102 | 0.125 | 0.06 | 0.06 | 0.125 | 0.25 | 0.125 | 0.06 | 0.06 | 7.2 |
| 103 | 0.125 | 0.06 | 0.125 | 0.06 | 0.5 | 0.25 | 0.25 | 0.125 | 3 |
| 104 | 0.125 | 0.06 | 0.125 | 0.125 | 0.25 | 0.25 | 0.125 | 0.25 | 8.3 |
| 105 | 0.125 | 0.06 | 0.06 | 0.06 | 0.25 | 0.125 | 0.06 | 0.06 | 6.1 |
| 106 | 0.06 | 0.06 | 0.06 | ND | 0.5 | 0.25 | 0.125 | 0.25 | ND |
| 107 | 0.125 | 0.06 | 0.125 | ND | 0.25 | 0.125 | 0.03 | 0.06 | ND |
| 108 | ND | 0.125 | ND | ND | 0.25 | 0.125 | 0.125 | 0.25 | ND |
| 109 | ND | 1 | ND | 0.5 | 2 | 0.5 | 1 | 0.5 | ND |
| 110 | ND | 0.5 | ND | 0.5 | 1 | 0.5 | 0.5 | 0.5 | ND |
| 111 | ND | 0.125 | ND | 0.125 | 0.5 | 0.06 | 0.25 | 0.125 | ND |
| 112 | ND | 0.25 | ND | 0.25 | 0.5 | 0.125 | 0.25 | 0.25 | ND |
| 113 | ND | 0.03 | ND | ND | 0.25 | 0.125 | 0.03 | ND | ND |
| 114 | ND | 0.06 | ND | ND | 0.5 | 0.25 | 0.125 | ND | ND |
| 115 | ND | ND | ND | ND | 0.25 | 0.25 | ND | ND | ND |
| 116 | ND | ND | ND | ND | 0.25 | 0.25 | ≤0.015 | ND | ND |
| 117 | ND | ND | ND | ND | 0.125 | 0.125 | ND | ND | ND |

**Table 6F - MIC values (micrograms /mL) for additional example compounds against strains of E. coli. K. pneumoniae, P. aeruginosa and A. baumannii with elevated MICs to Polymyxin B. PMB is included as a comparator.**

| Ex | *E. coli* | | | | *K. pneumoniae* | | | | | | | *P. aeruginosa* | | *A. baumannii* | |
|---|---|---|---|---|---|---|---|---|---|---|---|---|---|---|---|
| | 058 | 059 | 060 | 061 | 655 | 062 | 063 | 064 | 065 | 066 | 067 | 068 | 070 | 053 | 056 |
| PMB | 4 | 4 | 4 | 16 | 32 | 128 | 32 | 8 | 4 | 8 | 128 | 8 | 32 | 128 | 32 |
| 95 | 2 | 2 | 2 | 8 | 8 | 16 | 16 | 2 | 1 | 2 | >64 | 2 | 2 | 32 | 16 |
| 96 | 1 | 1 | 1 | 2 | 4 | 4 | 4 | 1 | 0.5 | 1 | 16 | 1 | 1 | 8 | 2 |
| 97 | 4 | 4 | 4 | 16 | 32 | 8 | 32 | 4 | 2 | ND | ND | 4 | 16 | >64 | 32 |
| 98 | 16 | 16 | 16 | 32 | 64 | >64 | >64 | 32 | 16 | 32 | >64 | 16 | 64 | 32 | 16 |
| 99 | 4 | 8 | 4 | 8 | 32 | 32 | 64 | 2 | 8 | 8 | >64 | 8 | 16 | 64 | 32 |
| 100 | 1 | 2 | 2 | 4 | 16 | 4 | 16 | 2 | 1 | 4 | 32 | 2 | 4 | 16 | ND |
| 101 | 2 | 8 | 2 | 8 | 16 | 16 | 32 | 2 | 1 | 2 | >64 | 4 | 16 | 32 | 16 |
| 102 | 1 | 2 | 1 | 4 | 8 | 4 | 16 | 2 | 0.5 | ND | 64 | 2 | 8 | 16 | 4 |
| 103 | 4 | 8 | 4 | 16 | 16 | 4 | 8 | 1 | 1 | 1 | 64 | 8 | 32 | ND | 16 |
| 104 | 2 | 2 | 2 | 8 | 4 | 1 | 8 | 0.5 | 0.25 | 0.25 | 8 | 4 | 4 | 16 | 8 |
| 105 | 4 | 8 | 4 | 16 | 32 | ND | 32 | 4 | 2 | ND | >64 | 8 | 16 | ND | 16 |
| 106 | 4 | 8 | 4 | 16 | 16 | >64 | 16 | 4 | 1 | 4 | >64 | 8 | 16 | >64 | >64 |
| 107 | 1 | ND | ND | ND | 8 | 32 | 8 | 2 | 0.25 | 2 | 64 | 2 | 8 | >64 | 32 |
| 108 | 8 | 16 | 8 | 32 | 32 | >64 | 64 | 16 | 2 | 8 | >64 | 32 | 64 | >64 | >64 |
| 109 | 2 | 1 | 2 | 8 | 16 | 8 | 16 | 4 | 2 | 4 | 32 | 2 | 2 | 32 | 16 |
| 110 | 2 | 2 | 2 | 8 | 32 | 16 | 16 | 8 | 1 | 8 | 32 | 2 | 2 | 32 | 16 |
| 111 | 8 | 8 | 8 | 16 | 32 | >64 | 32 | 16 | 4 | 8 | >64 | 8 | 16 | >64 | >64 |
| 112 | 8 | ND | ND | ND | ND | ND | ND | ND | 4 | ND | ND | ND | ND | ND | ND |
| 113 | 2 | 2 | 1 | 8 | 16 | ND | 32 | 4 | 4 | 4 | >64 | 2 | 8 | >64 | 32 |
| 114 | 4 | 8 | 4 | 16 | 32 | ND | 32 | 2 | 2 | 2 | >64 | 4 | 16 | >64 | >64 |
| | | | | | | | | | | | | | | | |

| Ex | *E. coli* | | | | *K. pneumoniae* | | | | | | | *P. aeruginosa* | | *A. baumannii* | |
|---|---|---|---|---|---|---|---|---|---|---|---|---|---|---|---|
| | 058 | 059 | 060 | 061 | 655 | 062 | 063 | 064 | 065 | 066 | 067 | 068 | 070 | 053 | 056 |
| 115 | 1 | ND | ND | ND | ND | ND | ND | ND | 1 | ND | ND | ND | ND | ND | ND |
| 116 | 4 | ND | ND | ND | ND | ND | ND | ND | 1 | ND | ND | ND | ND | ND | ND |
| 117 | 4 | ND | ND | ND | ND | ND | ND | ND | ND | ND | ND | ND | ND | ND | ND |

### In vivo efficacy against P. aeruginosa ATCC 27853 thigh infection in mice

In this study, 4 male mice were used in each compound treatment group and 6 for vehicle control. Mice were rendered temporarily neutropenic by immunosuppression with cyclophosphamide at 150mg/kg 4 days before infection and 100 mg/kg 1 day before infection by intraperitoneal injection. 24 hours post the second round of immunosuppression, mice were infected with *P. aeruginosa* ATCC27853 intramuscularly into both lateral thigh muscles under inhaled anaesthesia using ~ 2.5 to 5 × 10⁵ CFU/mouse thigh. Compounds were administered in solution by intravenous (IV) bolus injection into the lateral tail vein. This was performed three times at 1, 3.5 and 6 hours post infection at a dose volume of 10 mL/kg (0.25 mL/25g mouse). The vehicle control group was treated with 0.9% saline for injection also at 10 mL/kg IV thrice at 1, 3.5 and 6 hours post infection.

At 1 hour post infection, 4 animals were humanely euthanized using pentabarbitone overdose to provide a pre-treatment control group. At 9 hours post infection, the clinical condition of all animals was assessed prior to them being humanely euthanized by pentabarbitone overdose. Animal weight was determined before both thighs were removed and weighed individually. Individual thigh tissue samples were homogenized in ice cold sterile phosphate buffered saline. Thigh homogenates were then quantitatively cultured onto cystine lactose electrolyte deficient (CLED) agar and incubated at 37°C for 24 hours before colonies were counted.

An *in vivo* mouse thigh efficacy study compared D9, D11, D15 and D25 at 1.7 mg/Kg and 3.4 mg/kg free base equivalent with Polymyxin sulphate at 0.85, 1.7 and 3.4 mg/kg free base equivalent The infection level was ~ 1 × 10⁴ CFU/mouse thigh. Results are given in Table 17.

Table 17 - Mean log₁₀ reductions in *P. aeruginosa* ATCC 27853 burden recovered from mouse thighs following IV administration of compounds at 1, 3.5 and 6 h post-infection in a 9h infection model.

| **Treatment** | **Dose (mg/kg)** | **Log reduction from vehicle (CFU/g)** |
|---|---|---|
| Pre-treatment | ND | 1.89 |
| Vehicle | ND | 0.00 |
| D9 | 1.7 | 0.67 |
| | 3.4 | 3.54 |
| D11 | 1.7 | 3.42 |
| | 3.4 | 3.86 |
| D15 | 1.7 | 0.19 |
| | 3.4 | 3.89 |
| D25 | 1.7 | 3.10 |
| | 3.4 | 3.90 |
| Polymyxin B | 0.85 | 1.19 |
| | 1.7 | 4.04 |
| | 3.4 | 4.23 |

The doses in the table are indicated in mg drug base/kg.

All compounds gave > 3-log reduction in bacterial counts at a dose of 3.4 mg/kg free base equivalent. Examples D11 and D25 gave a > 3-log reduction in bacterial counts at dose of 1.7 mg/kg free base equivalent.

### Further In vivo efficacy against Escherichia coli ATCC 25922 thigh infection in mice

The *in vivo* efficacy of 5 compounds of the invention (D25, D31, D36, D50 and D52) was evaluated in a 9 h mouse thigh infection model of *E. coli.* The model was set up as for the Pseudomonas infection as described herein using an inoculum of 3.3 × 10⁵ CFU of *Escherichia coli* isolate ATCC 25922 delivered into each thigh. The results are summarized in Table 18.

Test compounds were administered at two concentrations (equivalent to 0.43 and 1.7 mg/kg free base). Three dose levels of polymyxin B sulphate were used as comparator (equivalent to 0.43, 1.7 and 3.4 mg/kg free base). The Results are given in Table 18.

Table 18 - Mean log₁₀ reductions in *E. coli ATCC* 25922 burden recovered from mouse thighs following IV administration of compounds at 1, 3.5 and 6 h post-infection in a 9h infection model

| Example | Dose (mg/kg/ dose) free base | Mean log₁₀ CFU reduction vs. control |
|---|---|---|
| Polymyxin B | 0.43 | 3.72 |
| | 1.7 | 4.08 |
| | 3.4 | 4.32 |
| D25 | 0.43 | 3.31 |
| | 1.7 | 4.17 |
| D31 | 0.43 | 4.16 |
| | 1.7 | 4.37 |
| D36 | 0.43 | 4.50 |
| | 1.7 | 4.13 |
| D50 | 0.43 | 4.05 |
| | 1.7 | 4.33 |
| D52 | 0.43 | 3.57 |
| | 1.47 | 4.04 |

All compounds gave a reduction in bacterial counts similar to or greater than that achieved by Polymyxin B at the same dose.

### Further In vivo efficacy against Acinetobacter baumannii NCTC 13301 - thigh infection in mice

The *in vivo* efficacy of 3 compounds of the invention (D36, D50 and D65) was evaluated in a mouse thigh infection model of *A. baumannii.* The model was setup as described above, with the exception that compounds were delivered SC at 2, 6 and 10 h post-infection with thighs harvested at 16 h. post-infection. An inoculum of approx. 1.4 × 10⁵ CFU of *A*. *baumannii* isolate NCTC 13301 was delivered into each mouse thigh. Test compounds and the comparator polymyxin B were administered at five concentrations (equivalent to 0.215, 0.86, 2.6, 8.6 and 17.2 mg/kg free base). The results are summarized in Table 19.

**Table 19 - Mean log₁₀ reductions in A. baumannii NCTC 13301 burden recovered from mouse thighs following SC administration of compounds at 2, 6 and 10 h post-infection in a 16h infection model.**

| Example | Dose (mg/kg/dose) free base | Mean log₁₀ CFU reduction vs. control |
|---|---|---|
| Polymyxin B | 0.215 | 0.17 |
| | 0.86 | 1.64 |
| | 2.6 | 5.44 |
| | 8.6 | 6.15 |
| | 17.2 | 6.53 |
| D36 | 0.215 | 0.51 |
| | 0.86 | 1.60 |
| | 2.6 | 4.57 |
| | 8.6 | 5.92 |
| | 17.2 | 6.07 |
| D50 | 0.215 | 0.69 |
| | 0.86 | 4.74 |
| | 2.6 | 5.05 |
| | 8.6 | 5.79 |
| | 17.2 | 5.69 |
| D65 | 0.215 | 0.71 |
| | 0.86 | 2.40 |
| | 2.6 | 5.42 |
| | 8.6 | 6.05 |
| | 17.2 | 6.59 |

All test compounds showed a good dose response and similar or greater efficacy than seen with the equivalent dose of polymyxin B.

### Further in vivo efficacy against Acinetobacter baumannii NCTC 13301 - lung infection in mice

The *in vivo* efficacy of 2 compounds of the invention (D36, D65) was evaluated in a mouse lung infection model of *A*. *baumannii NCTC 13301.* The results are summarized in Table 20. Groups of 7 male neutropenic specific-pathogen-free CD-1 mice weighing 21.5-27.5g were used. On Day 0, animals were infected with 0.04 mL inoculum by intranasal instillation into mouse nostrils and were kept in an upright position on a string rack for -10 min post-infection. The inoculum concentration was approx. 8 × 10⁸ CFU/ml of *A. baumannii* isolate NCTC 13301 (3.5 × 10⁷ CFU/lung). At 2 h, the CFU count was determined from 3 mice and the remaining mice (7 per test compound groups) were treated with a subcutaneous injection of the drug at + 2, 6 and 10 hr post-infection. Test compounds and the comparator polymyxin B were administered at four concentrations (2.6, 8.6, 17.2 and 25.8 mg/kg free base). The vehicle control group (7 mice) was treated with 0.9% saline for injection at 2, 6 and 10h post-infection.

Sixteen hours after infection, the mice were euthanized humanely. The lungs of each animal were harvested, homogenized and quantitatively cultured onto CLED agar for CFU determination. Decrease of the total CFU compared to control counts at 16h post-infection was determined for each dose group.

**Table 20 - Mean log₁₀ reductions in A. baumannii NCTC 13301 burden recovered from mouse lungs following SC administration of compounds at 2, 6 and 10 h post-infection in a 16h infection model.**

| Example | Dose (mg/kg/ dose) free base | Mean log₁₀ CFU reduction vs. control |
|---|---|---|
| Polymyxin B | 2.6 | 0.79 |
| | 8.6 | 0.58 |
| | 17.2 | 0.54 |
| | 25.8 | 1.10 |
| D36 | 2.6 | 0.95 |
| | 8.6 | 1.37 |
| | 17.2 | 2.56 |
| | 25.8 | 3.74 |
| D65 | 2.6 | 1.07 |
| | 8.6 | 1.91 |
| | 17.2 | 2.89 |
| | 25.8 | 8.15 |

Example D36 and D65 at all doses showed greater efficacy than observed with the equivalent dose of polymyxin B.

### Further in vivo efficacy against Pseudomonas aeruginosa ATCC 27853 - lung infection in mice

The *in vivo* efficacy of 4 compounds of the invention (D25, D36, D42 and D50) was evaluated in a mouse lung infection model of *P. aeruginosa ATCC* 27853. The model was setup as described above with the exception that groups of up to 8 mice were inoculated through intranasal instillation of ~2.5 × 10⁶ CFUs of *P. aeruginosa* isolate ATCC 27853 (1.0 × 10⁵ CFU/lung). Test compounds were administered at two concentrations (equivalent to 4.3 and 17.2 mg/kg free base). Three dose levels of polymyxin B sulfate were used as comparator (equivalent to 4.3, 8.6 and 17.2 mg/kg free base). The results are summarized in Table 21.

**Table 21 - Mean log₁₀ reductions in P. aeruginosa ATCC 27853 burden recovered from mouse lungs following SC administration of compounds at 2, 6 and 10 h post-infection in a 16h infection model.**

| Example No | Dose (mg/kg/ dose) free base | Mean log₁₀ CFU reduction vs. control |
|---|---|---|
| Polymyxin B | 4.3 | 0.36 |
| | 8.6 | 1.46 |
| | 17.2 | 2.87 |
| D25 | 4.3 | 3.68 |
| | 17.2 | 5.55 |
| D36 | 4.3 | 1.19 |
| | 17.2 | 5.30 |
| D42 | 4.3 | 1.23 |
| | 17.2 | 5.34 |
| D50 | 4.3 | 1.44 |
| | 17.2 | 5.29 |

All test compounds showed greater efficacy than seen with the equivalent dose of polymyxin B with many samples at or below the limit of detection of the model when the higher 17.2 mg/kg/dose was administered.

### References

de Visser et al, J. Peptide Res, 61, 2003, 298-306 GCC 2012/22819
Handbook of Pharmaceutical Excipients, 5th edition, 2005
Katsuma et al. Chem. Pharm. Bull. 2009; 57, 332-336 WO 2013/072695
Petrosillo et al. Clin. Microbiol. Infect. 2008; 14, 816-827
Quale et al. Microb. Drug Resist. 2012; 18, 132-136
Remington's Pharmaceutical Sciences, 18th edition, Mack Publishing Company, Easton, Pa., 1990
Sato et a/. Chem. Pharm. Bull. 2011; 59, 597-602
TW 101142961
US 8,415,307
Vaara et al, Antimicrob. Agents and Chemotherapy, 52, 2008. 3229-3236 Vaara et al. Microbiol. Rev. 1992; 56, 395-411
WO 1988/00950
WO 2008/017734
WO 2010/075416
WO 2012/168820
Yamada et al, J. Peptide Res. 64, 2004, 43-50
Yousef et al., Antimicrob. Agents Chemother. 2011, 55, 4044-4049

## Claims

1. A compound of formula (III): 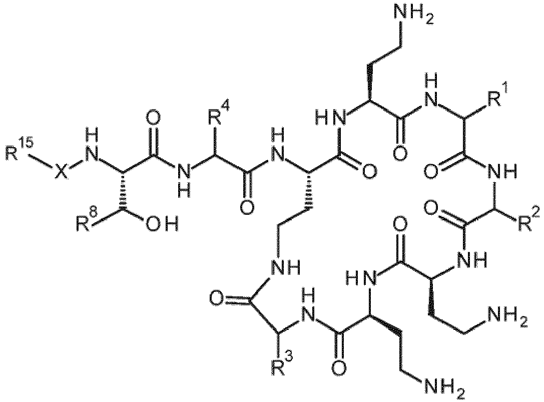 and pharmaceutically acceptable salts, protected forms, solvates and hydrates thereof, such as pharmaceutically acceptable salts, and hydrates thereof.
wherein:
-X- represents -C(O)-, -NHC(O)-, -OC(O)-, -CH₂- or -SO₂-;
-R¹ together with the carbonyl group and nitrogen alpha to the carbon to which it is attached, is a phenylalanine, leucine or valine residue;
-R² together with the carbonyl group and nitrogen alpha to the carbon to which it is attached, is a leucine, iso-leucine, phenylalanine, threonine, valine or nor-valine residue;
-R³ together with the carbonyl group and nitrogen alpha to the carbon to which it is attached, is a threonine or leucine residue;
-R⁴ is C₁₋₆ alkyl substituted with one hydroxyl group or one amino group;
-R¹⁵ is an amino-containing group: 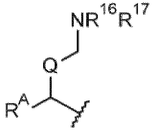
where:
-R^{A} is -L^{A}-R^{AA} or hydrogen;
-Q- is a covalent bond or -CH(R^{B})-;
-R^{B} is hydrogen or -L^{B}-R^{BB};
or, where -Q- is -CH(R^{B})-, -R^{A} and -R^{B} together form a 5- to 10-membered monocyclic or bicyclic carbocycle, or -R^{A} and -R^{B} together form a 5- to 10-monocyclic or bicyclic heterocycle;
and, where -Q- is a covalent bond, -R^{A} is -L^{A}-R^{AA}, and where -Q- is -CH(R^{B})-one or both of -R^{A} and -R^{B} is not hydrogen;
-R¹⁶ is independently hydrogen or C₁₋₄ alkyl;
-R¹⁷ is independently hydrogen or C₁₋₄ alkyl;
or -NR¹⁶R¹⁷ is a guanidine group;
or -R¹⁷ and -R^{A} together form a 5- to 10-membered nitrogen-containing monocyclic or bicyclic heterocycle;
or, where -Q- is -CH(R^{B})-, -R¹⁷ and -R^{B} together form a 5- to 10-membered nitrogen-containing monocyclic or bicyclic heterocycle;
and where -R¹⁷ and -R^{A} together form a monocyclic nitrogen-containing heterocycle, each ring carbon atom in -R¹⁷ and -R^{A} is optionally mono- or di-substituted with -R^{C}, and the monocyclic heterocycle is substituted with at least one group selected from -R^{C}, -R^{N}, -R^{NA} and -L^{B}-R^{BB}, where present,
and where -R¹⁷ and -R^{B} together form a monocyclic nitrogen-containing heterocycle, each ring carbon atom in -R¹⁷ and -R^{B} is optionally mono- or di-substituted with -R^{C}, and the monocyclic heterocycle is substituted with at least one group selected from -R^{C}, and -R^{N}, where present, or the monocyclic heterocycle is optionally substituted when -R^{A} is -L^{A}-R^{AA},
and a monocyclic nitrogen-containing heterocycle optionally contains one further nitrogen, oxygen or sulfur ring atom, and where a further nitrogen ring atom is present it is optionally substituted with -R^{N}, with the exception of a further nitrogen ring atom that is connected to the carbon that is α to the group -X-, which nitrogen ring atom is optionally substituted with -R^{NA};
where -R¹⁷ and -R^{A} or -R¹⁷ and -R^{B} together form a bicyclic nitrogen-containing heterocycle, each ring carbon atom in -R¹⁷ and -R^{A} or -R¹⁷ and -R^{B} is optionally mono- or di-substituted with -R^{D};
and the bicyclic nitrogen-containing ring atom heterocycle optionally contains one, two or three further heteroatoms, where each heteroatom is independently selected from the group consisting of nitrogen, oxygen and sulfur, and where further nitrogen ring atoms are present, each further nitrogen ring atom is optionally substituted with -R^{N}, with the exception of a nitrogen ring atom that is connected to the carbon that is α to the group -X-, which nitrogen ring atom is optionally substituted with -R^{NA};
where -R^{A} and -R^{B} together form a 5- to 10-membered monocyclic carbocycle or heterocycle, each ring carbon atom in -R^{A} and -R^{B} is optionally mono- or di-substituted with -R^{C}, and a nitrogen ring atom, where present in the monocyclic heterocycle, is optionally substituted with -R^{N}, with the exception of a nitrogen ring atom that is connected to the carbon that is α to the group -X-, which nitrogen ring atom is optionally substituted with -R^{NA};
where -R^{A} and -R^{B} together form a 5- to 10-membered bicyclic carbocycle or heterocycle, each ring carbon atom in -R^{A} and -R^{B} is optionally mono- or di-substituted with -R^{D}, and a nitrogen ring atom, where present in the bicyclic heterocycle, is optionally substituted with -R^{N}, with the exception of a nitrogen ring atom that is connected to the carbon that is α to the group -X-, which nitrogen ring atom is optionally substituted with -R^{NA};
and where R¹⁷ and -R^{A} or -R¹⁷ and -R^{B} together form a 5- to 10-membered nitrogen-containing monocyclic or bicyclic heterocycle, or where -R^{A} and -R^{B} together form a 5- to 10-membered monocyclic or bicyclic carbocycle, or together form a 5- to 10-membered monocyclic or bicyclic heterocycle, a carbon ring atom in -R¹⁷ and -R^{A}, -R¹⁷ and -R^{B}, or -R^{A} and -R^{B} is optionally alternatively substituted with oxo (=O);
each -R^{C} is independently -L^{C}-R^{CC};
each -R^{D} is independently selected from -R^{C}, halo, -NO₂, -OH, and -NH₂;
each -R^{N} is independently -L^{N}-R^{NN};
each -R^{NA} is independently -R^{L}-R^{NN} or -R^{NN};
-R^{AA}, -R^{BB}, and each -R^{CC} and -R^{NN} where present, is independently selected from C₁₋₁₂ alkyl, C₃₋₁₀ cycloalkyl, C₄₋₁₀ heterocyclyl, and C₅₋₁₂ aryl;
each -L^{A}- is independently a covalent bond or a linking group selected from -R^{L}-*, -O-L^{AA}-*, -OC(O)-L^{AA}-*, -N(R¹¹)-L^{AA}-*, and -C(O)-L^{AA}-*, where the asterisk indicates the point of attachment of the group -L^{A}- to -R^{AA};
each -L^{B}- and -L^{C}- is independently a covalent bond or a linking group selected from -RL-*, -O-L^{AA}-*, -OC(O)-L^{AA}-*,-N(R¹¹)-L^{AA}-*, -N(R¹¹)C(O)-L^{AA}-*, -C(O)-L^{AA}-*, -C(O)O-L^{AA}-*, and -C(O)N(R¹¹)- L^{AA}-*, and optionally further selected from -N(R¹¹)S(O)-L^{AA}-*, -N(R¹¹)S(O)₂-L^{AA}-*, -S(O)N(R¹¹)-L^{AA}-*, and -S(O)₂N(R¹¹)-L^{AA}-*where the asterisk indicates the point of attachment of the group -L^{B}- to -R^{BB} or the group -L^{C}- to -R^{CC};
each -L^{N}- is independently a covalent bond or a group selected from -S(O)-L^{AA}-*, -S(O)₂₋L^{AA}-*, -C(O)-L^{AA}-* and -C(O)N(R¹¹)-L^{AA}-*, where the asterisk indicates the point of attachment of the group -L^{N}- to -R^{NN};
and each -L^{AA}- is independently a covalent bond or -R^{L}-;
and each -R^{L}- is independently selected from C₁₋₁₂ alkylene, C₂₋₁₂ heteroalkylene, C₃₋₁₀ cycloalkylene and C₅₋₁₀ heterocyclylene, and where -L^{AA}- is connected to a group G₁₋₁₂ alkyl, -R^{L}- is not C₁₋₁₂ alkylene;
and each C₁₋₁₂ alkyl, C₃₋₁₀ cycloalkyl, C₄₋₁₀ heterocyclyl, C₅₋₁₂ aryl, C₁₋₁₂ alkylene, C₂₋₁₂ heteroalkylene, C₃₋₁₀ cycloalkylene and C₅₋₁₀ heterocyclylene group is optionally substituted, where -R^{S} is an optional substituent to carbon and -R¹² is an optional substituent to nitrogen;
each -R^{S} is independently selected from -OH, -OR¹², -OC(O)R¹², halo, -R¹², -NHR¹², -NR¹²R¹³, -NHC(O)R¹², -N(R¹²)C(O)R¹², -SH, -SR¹², -C(O)R¹², -C(O)OH, -C(O)OR¹², -C(O)NH₂, -C(O)NHR¹² and C(O)NR¹²R¹³, such as wherein each -R^{S} is independently selected from -OR¹², halo, and -R¹²; except that -R¹² is not a substituent to a C₁₋₁₂ alkyl group; or where a carbon atom is di-substituted with -R^{S}, these groups may together with the carbon to which they are attached form a C₃₋₆ carbocycle or a C₅₋₆ heterocycle, where the carbocycle and the heterocycle are optionally substituted with one or more groups -R¹²;
each -R¹² is independently C₁₋₆ alkyl, C₁₋₆ haloalkyl, phenyl or benzyl;
each -R¹³ is independently C₁₋₆ alkyl, C₁₋₆ haloalkyl, phenyl or benzyl;
or -R¹² and -R¹³, where attached to N, may together form a 5- or 6-membered heterocyclic ring, which is optionally substituted with C₁₋₆ alkyl, C₁₋₆ haloalkyl, phenyl or benzyl;
each -R¹¹ is independently hydrogen or C₁₋₄ alkyl; and -R⁸ is hydrogen or methyl.

2. The compound of claim 1, wherein:
(i) -X- is -C(O)-; and/or
(ii) -R⁸ is methyl; and/or
(iii) -R¹ together with the carbonyl group and nitrogen alpha to the carbon to which it is attached is a phenylalanine residue or a leucine residue; and/or
(iv) -R² together with the carbonyl group and nitrogen alpha to the carbon to which it is attached is a leucine residue; and/or
(v) -R³ together with the carbonyl group and nitrogen alpha to the carbon to which it is attached is a threonine residue; and/or
(vi) -R⁴ together with the carbonyl group and nitrogen alpha to the carbon to which it is attached, is α,γ-diaminobutyric acid (Dab), a serine residue, a threonine residue, a lysine residue, an ornithine residue, or α,β-diaminopropionic acid (Dap), such as wherein -R⁴ together with the carbonyl group and nitrogen alpha to the carbon to which it is attached is α,γ-diaminobutyric acid (Dab) or α,β-diaminopropionic (Dap), such as L-Dab or L-Dap; and/or
(vii) -R¹⁶ is hydrogen; and/or diaminopropionic
(viii) -R¹⁷ is hydrogen.

3. The compound of claim 1 or claim 2, wherein -R¹⁵ is: 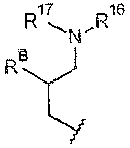 where -R^{B} is -L^{B}-R^{BB}.

4. The compound of claim 3, wherein -L^{B}- is a covalent bond.

5. The compound of claim 3, wherein -L^{B}- is C₁₋₁₂ alkylene.

6. The compound of any one of claims 3 to 5, wherein -R^{BB} is independently a group selected from C₁₋₁₂ alkyl, C₄₋₁₀ heterocyclyl, and C₅₋₁₂ aryl, and each group is optionally substituted with one or more groups -R^{S} or one or more groups -R^{N}.

7. The compound of claim 6, wherein -R^{BB} is C₁₋₁₂ alkyl, optionally substituted with one or more groups -R^{S}, such as wherein -R^{BB} is C₁₋₁₂ alkyl.

8. The compound of claim 6, wherein -R^{BB} is C₅₋₁₂ aryl, optionally substituted with one or more groups -R^{S} or one or more groups -R^{N}, such as wherein -R^{BB} is phenyl or naphthyl, optionally substituted with one or more groups -R^{S}.

9. The compound of claim 1 or claim 2, wherein -R¹⁵ is: 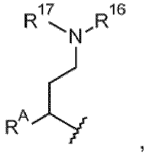 such as 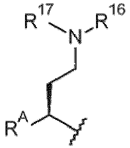 where -R^{A} is -L^{A}-R^{AA}.

10. The compound of claim 9, wherein -L^{A}- is -R^{L}-* or -O-L^{AA}-*.

11. The compound of claim 10, wherein -L^{AA}- is C₁₋₁₂ alkylene, such as -CH₂-.

12. The compound of any one of claims 9 to 11, wherein -R^{AA} is C₅₋₁₂ aryl, optionally substituted with one or more groups -R^{S} or one or more groups -R^{N}, such as wherein -R^{AA} is phenyl optionally substituted with one or more groups -R^{S}.

13. A pharmaceutical composition comprising a compound of any one of claims 1 to 12 and a biologically acceptable excipient, optionally together with a second active agent.

14. A compound of any one of claims 1 to 12 or a pharmaceutical composition of claim 14 for use in a method of treatment.

15. A compound of any one of paragraphs 1 to 12 or a pharmaceutical composition of claim 14 for use in a method of treating a microbial infection, such as a method of treating a Gram-negative bacterial infection, such as wherein the Gram-negative bacteria is selected from the group consisting of *Escherichia* spp., *Klebsiella* spp., *Enterobacter* spp., *Salmonella* spp., *Shigella* spp., *Citrobacter* spp., *Morganella morganii, Yersinia pseudotuberculosis* and other Enterobacteriaceae, *Pseudomonas* spp., *Acinetobacter* spp., *Moraxella, Helicobacter, Stenotrophomonas, Bdellovibrio,* acetic acid bacteria, *Legionella* and alpha-proteobacteria.

## Patentansprüche

1. Verbindung der Formel (III): 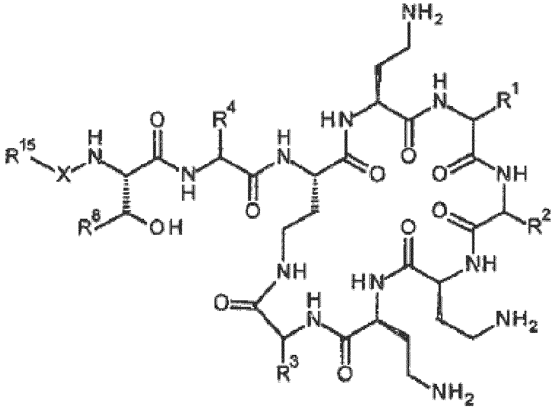 und pharmazeutisch annehmbare Salze, geschützte Formen, Solvate und Hydrate davon, wie z.B. pharmazeutisch annehmbare Salze und Hydrate davon,
worin:
- X- für -C(O)-, -NHC(O)-, -OC(O)-, -CH₂- oder -SO₂- steht;
- R¹ zusammen mit der Carbonylgruppe und dem Stickstoff in alpha-Stellung zu dem Kohlenstoff, an den er gebunden ist, ein Phenylalanin-, Leucin- oder Valinrest ist;
- R² zusammen mit der Carbonylgruppe und dem Stickstoff in alpha-Stellung zu dem Kohlenstoff, an den er gebunden ist, ein Leucin-, Isoleucin-, Phenylalanin-, Threonin-, Valin- oder Norvalinrest ist;
- R³ zusammen mit der Carbonylgruppe und dem Stickstoff in alpha-Stellung zu dem Kohlenstoff, an den er gebunden ist, ein Threonin- oder Leucinrest ist;
- R⁴ C₁₋₆-Alkyl ist, das mit einer Hydroxylgruppe oder einer Aminogruppe substituiert ist;
- R¹⁵ eine Amino enthaltende Gruppe ist: 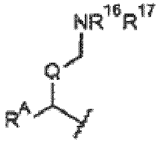
worin:
-R^{A} -L^{A}-R^{AA} oder Wasserstoff ist;
-Q- eine kovalente Bindung oder -CH(R⁸)- ist;
-R^{B} Wasserstoff oder -L^{B}-R^{BB} ist;
oder, wenn -Q- -CH(R^{B})- ist, -R^{A} und -R^{B} zusammen einen 5- bis 10-gliedrigen monozyklischen oder bizyklischen Carbozyklus bilden oder -R^{A} und -R^{B} zusammen einen 5- bis 10-gliedrigen monozyklischen oder bizyklischen Heterozyklus bilden;
und, wenn -Q- eine kovalente Bindung ist, -R^{A} -L^{A}-R^{AA} ist, und wenn -Q- -CH(R^{B})- ist, einer oder beide von -R^{A} und -R^{B} nicht Wasserstoff sind;
-R¹⁶ unabhängig Wasserstoff oder C₁₋₄-Alkyl ist;
-R¹⁷ unabhängig Wasserstoff oder C₁₋₄-Alkyl ist;
oder -NR¹⁶R¹⁷ eine Guanidingruppe ist;
oder -R¹⁷ und -R^{A} zusammen einen 5- bis 10-gliedrigen, Stickstoff-hältigen, monozyklischen oder bizyklischen Heterozyklus bilden;
oder, wenn -Q- -CH(R^{B})- ist, -R¹⁷ und -R^{B} zusammen einen 5- bis 10-gliedrigen, Stickstoff-hältigen, monozyklischen oder bizyklischen Heterozyklus bilden;
und, wenn -R¹⁷ und -R^{A} zusammen einen monozyklischen, Stickstoff-hältigen Heterozyklus bilden, jedes Ringatom in -R¹⁷ und -R^{A} gegebenenfalls mit -R^{C} mono- oder disubstituiert ist, und der monozyklische Heterozyklus mit zumindest einer Gruppe, ausgewählt aus -R^{C}, -R^{N}, -R^{NA} und -L^{B}-R^{BB}, sofern vorhanden, substituiert ist,
und, wenn -R¹⁷ und -R^{B} zusammen einen monozyklischen, Stickstoff-hältigen Heterozyklus bilden, jedes Ringatom in -R¹⁷ und -R^{B} gegebenenfalls mit -R^{C} mono- oder disubstituiert ist, und der monozyklische Heterozyklus mit zumindest einer Gruppe, ausgewählt aus -R^{C} und -R^{N}, sofern vorhanden, substituiert ist, oder der monozyklische Heterozyklus gegebenenfalls substituiert ist, wenn -R^{A} -L^{A}-R^{AA} ist,
und wobei ein monozyklischer, Stickstoff-hältiger Heterozyklus gegebenenfalls ein weiteres Stickstoff-, Sauerstoff oder Schwefel-Ringatom enthält und, wenn ein weiteres Stickstoff-Ringatom vorhanden ist, dieses gegebenenfalls mit -R^{N} substituiert ist, ausgenommen eines weiteren Stickstoffatoms, das mit dem Kohlenstoff verbunden ist, der in α-Stellung zur Gruppe -X- liegt, wobei das Stickstoff-Ringatom gegebenenfalls mit -R^{NA} substituiert ist;
wenn -R¹⁷ und -R^{A} oder -R¹⁷ und -R^{B} zusammen einen bizyklischen, Stickstoff-hältigen Heterozyklus bilden, jedes Ringatom in -R¹⁷ und -R^{A} oder -R¹⁷ und -R^{B} gegebenenfalls mit -R^{D} mono- oder disubstituiert ist;
und wobei der bizyklische, Stickstoff-hältige Ringatom-Heterozyklus gegebenenfalls ein, zwei oder drei weitere Heteroatome enthält, wobei jedes Heteroatom unabhängig aus der aus Stickstoff, Sauerstoff und Schwefel bestehenden Gruppe ausgewählt ist, und, wenn weitere Stickstoff-Ringatome vorhanden sind, jedes weitere Stickstoff-Ringatom gegebenenfalls mit -R^{N} substituiert ist, ausgenommen eines Stickstoff-Ringatoms, das mit dem Kohlenstoff verbunden ist, der in α-Stellung zur Gruppe -X- liegt, wobei das Stickstoff-Ringatom gegebenenfalls mit -R^{NA} substituiert ist;
wenn -R^{A} und -R^{B} zusammen einen 5- bis 10-gliedrigen monozyklischen Carbozyklus oder Heterozyklus bilden, jedes Ringkohlenstoffatom in -R^{A} und -R^{B} gegebenenfalls mit -R^{C} mono- oder disubstituiert ist, und ein Stickstoffringatom, sofern in dem monozyklischen Heterozyklus vorhanden, gegebenenfalls mit -R^{N} substituiert ist, ausgenommen eines Stickstoffringatoms, das mit dem Kohlenstoff verbunden ist, der in α-Stellung zur Gruppe -X-liegt, wobei das Stickstoff-Ringatom gegebenenfalls mit -R^{NA} substituiert ist;
wenn -R^{A} und -R^{B} zusammen einen 5- bis 10-gliedrigen, bizyklischen Carbozyklus oder Heterozyklus bilden, jedes Ringkohlenstoffatom in -R^{A} und -R^{B} gegebenenfalls mit -R^{D} mono- oder disubstituiert ist, und ein Stickstoffringatom, sofern in dem bizyklischen Heterozyklus vorhanden, gegebenenfalls mit -R^{N} substituiert ist, ausgenommen eines Stickstoffringatoms, das mit dem Kohlenstoff verbunden ist, der in α-Stellung zur Gruppe -X- liegt, wobei das Stickstoff-Ringatom gegebenenfalls mit -R^{NA} substituiert ist;
und wenn R¹⁷ und -R^{A} oder -R¹⁷ und -R^{B} zusammen einen 5- bis 10-gliedrigen Stickstoff-hältigen, monozyklischen oder bizyklischen Heterozyklus bilden oder wenn -R^{A} und -R^{B} zusammen einen 5- bis 10-gliedrigen, monozyklischen oder bizyklischen Carbozyklus bilden oder zusammen einen 5- bis 10-gliedrigen, monozyklischen oder bizyklischen Heterozyklus bilden, ein Kohlenstoffringatom in -R¹⁷ und -R^{A}, -R¹⁷ und -R^{B} oder -R^{A} und -R^{B} gegebenenfalls alternativ dazu mit Oxo (=O) substituiert ist;
wobei die -R^{C} jeweils unabhängig -L^{C}-R^{CC} sind;
die -R^{D} jeweils unabhängig aus -R^{C}, Halogen, -NO₂, -OH und -NH₂ ausgewählt sind;
die -R^{N} jeweils unabhängig -L^{N}-R^{NN} sind;
die -R^{NA} jeweils unabhängig -R^{L}-R^{NN} oder -R^{NN} sind;
-R^{AA}, -R^{BB} und die -R^{CC} und -R^{NN}, sofern vorhanden, jeweils unabhängig aus C₁₋₁₂-Alkyl, C₃₋₁₀-Cycloaklyl, C₄₋₁₀-Heterocyclyl und C₅₋₁₂-Aryl ausgewählt sind;
die -L^{A}- jeweils unabhängig eine kovalente Bindung oder eine Linkergruppe sind, die aus -R^{L}-*, -O-L^{AA}-*, -OC(O)-L^{AA}-*, -N(R¹¹)-L^{AA}-* und -C(O)-L^{AA}-* ausgewählt ist, wobei der Stern die Anbindungsstelle der Gruppe -L^{A}- an -R^{AA} angibt;
die -L^{B}- und -L^{C}- jeweils unabhängig eine kovalente Bindung oder eine Linkergruppe sind, die aus -R^{L}-*, -O-L^{AA}-*, -OC(O)-L^{AA}-*, -N(R¹¹)-L^{AA}-*, -N(R¹¹)C(O)-L^{AA}-*, -C(O)-L^{AA}-*, -C(O)O-L^{AA}-* und -C(O)N(R¹¹)-L^{AA}-* ausgewählt sind und die gegebenenfalls weiters aus -N(R¹¹)S(O)-L^{AA}-*, -N(R¹¹S(O)₂-L^{AA}-*, -S(O)N(R¹¹)-L^{AA}-* und -S(O)N(R¹¹)-L^{AA}-* ausgewählt sind, wobei der Stern die Anbindungsstelle der Gruppe -L^{B} an -R^{BB} oder der Gruppe -L^{C} an -R^{CC} angibt;
die -L^{N}- jeweils unabhängig eine kovalente Bindung oder eine Gruppe sind, die aus -S(O)-L^{AA}-*, -S(O)₂-L^{AA}-*, -C(O)-L^{AA}-* und -C(O)N(R¹¹)-L^{AA}-* ausgewählt ist, wobei der Stern die Anbindungsstelle der Gruppe -L^{N}- an -R^{NN} angibt;
und die -L^{AA}- jeweils unabhängig eine kovalente Bindung oder -R^{L}- sind;
und die -R^{L}- jeweils unabhängig aus C₁₋₁₂-Alkylen, C₂₋₁₂-Heteroalkylen, C₃₋₁₀-Cycloalkylen und C₅₋₁₀-Heterocyclylen ausgewählt sind und, wenn -L^{AA}- mit einer Gruppe C₁₋₁₂-Alkyl verbunden ist, -R^{L}- nicht C₁₋₁₂-Alkylen ist;
und die Gruppen C₁₋₁₂-Alkyl, C₃₋₁₀-Cycloalkyl, C₄₋₁₀-Heterocyclyl, C₅₋₁₂-Aryl, C₁₋₁₂-Alkylen, C₂₋₁₂-Heteroalkylen, C₃₋₁₀-Cycloalkylen und C₅₋₁₀-Heterocyclylen gegebenenfalls jeweils substituiert sind, wobei -R^{S} ein optionaler Substituent für Kohlenstoff und -R¹² ein optionaler Substituent für Stickstoff ist;
die -R^{S} jeweils unabhängig aus -OH, -OR¹², -OC(O)R¹², Halogen, -R¹², -NHR¹², -NR¹²R¹³, -NHC(O)R¹², -N(R¹²)C(O)R¹², -SH, -SR¹², -C(O)R¹², -C(O)OH, -C(O)OR¹², -C(O)NH₂, -C(O)NHR¹² und C(O)NR¹²R¹³ ausgewählt sind, wobei die -R^{S} z. B. jeweils unabhängig aus -OR¹², Halogen und -R¹² ausgewählt sind; ausgenommen, dass -R¹² kein Substituent für eine C₁₋₁₂-Alkylgruppe ist; oder, wenn ein Kohlenstoffatom mit -R^{S} disubstituiert ist, diese Gruppen zusammen mit dem Kohlenstoff, an den sie gebunden sind, gegebenenfalls einen C₃₋₆-Carbozyklus oder einen C₅₋₆-Heterozyklus bilden, wobei der Carbozyklus und der Heterozyklus gegebenenfalls mit einer oder mehreren Gruppen -R¹² substituiert sind;
die -R¹² jeweils unabhängig C₁₋₆-Alkyl, C₁₋₆-Halogenalkyl, Phenyl oder Benzyl sind;
die -R¹³ jeweils unabhängig C₁₋₆-Alkyl, C₁₋₆-Halogenalkyl, Phenyl oder Benzyl sind;
oder -R¹² und -R¹³, wenn sie an N gebunden sind, gegebenenfalls zusammen einen 5- oder 6-gliedrigen heterozyklischen Ring bilden, der gegebenenfalls mit C₁₋₆-Alkyl, C₁₋₆-Halogenalkyl, Phenyl oder Benzyl substituiert ist;
die -R¹¹ jeweils unabhängig Wasserstoff oder C₁₋₄-Alkyl sind; und -R⁸ Wasserstoff oder Methyl ist.

2. Verbindung nach Anspruch 1, worin:
( i) -X- -C(O)- ist; und/oder
( ii) -R⁸ Methyl ist; und/oder
( iii) -R¹ zusammen mit der Carbonylgruppe und dem Stickstoff in alpha-Stellung zu dem Kohlenstoff, an den er gebunden ist, ein Phenylalaninrest oder ein Leucinrest ist; und/oder
( iv) -R² zusammen mit der Carbonylgruppe und dem Stickstoff in alpha-Stellung zu dem Kohlenstoff, an den er gebunden ist, ein Leucinrest ist; und/oder
( v) -R³ zusammen mit der Carbonylgruppe und dem Stickstoff in alpha-Stellung zu dem Kohlenstoff, an den er gebunden ist, ein Threoninrest ist; und/oder
( vi) -R⁴ zusammen mit der Carbonylgruppe und dem Stickstoff in alpha-Stellung zu dem Kohlenstoff, an den er gebunden ist, eine α,γ-Diaminobuttersäure (Dab), ein Serinrest, ein Threoninrest, ein Lysinrest, ein Ornithinrest oder eine α,β-Diaminopropionsäure (Dap) ist, wobei -R⁴ z. B. zusammen mit der Carbonylgruppe und dem Stickstoff in alpha-Stellung zu dem Kohlenstoff, an den er gebunden ist, α,γ-Diaminobuttersäure (Dab) oder α,β-Diaminopropionsäure (Dap), wie z. B. L-Dab oder L-Dap, ist; und/oder
( vii) -R¹⁶ Wasserstoff ist; und/oder
( viii) -R¹⁷ Wasserstoff ist.

3. Verbindung nach Anspruch 1 oder Anspruch 2, worin -R¹⁵ Folgendes ist: 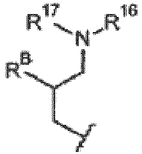 worin -R^{B} -L^{B}-R^{BB} ist.

4. Verbindung nach Anspruch 3, worin -L^{B}- eine kovalente Bindung ist.

5. Verbindung nach Anspruch 3, worin -L^{B}- C₁₋₁₂-Alkylen ist.

6. Verbindung nach einem der Ansprüche 3 bis 5, worin -R^{BB} unabhängig eine aus C₁₋₁₂-Alkyl, C₄₋₁₀-Heterocyclyl und C₅₋₁₂-Aryl ausgewählte Gruppe ist, wobei jede Gruppe gegebenenfalls mit einer oder mehreren Gruppen -R^{S} oder einer oder mehrerer Gruppen -R^{N} substituiert ist.

7. Verbindung nach Anspruch 6, worin -R^{BB} C₁₋₁₂-Alkyl ist, das gegebenenfalls mit einer oder mehreren Gruppen -R^{S} substituiert ist, z. B. worin -R^{BB} C₁₋₁₂-Alkyl ist.

8. Verbindung nach Anspruch 6, worin -R^{BB} C₅₋₁₂-Aryl ist, das gegebenenfalls mit einer oder mehreren Gruppen -R^{S} oder einer oder mehreren Gruppen -R^{N} substituiert ist, z. B. worin -R^{BB} Phenyl oder Naphthyl, gegebenenfalls substituiert mit einer oder mehreren Gruppen -R^{S}, ist.

9. Verbindung nach Anspruch 1 oder Anspruch 2, worin -R¹⁵ Folgendes ist: 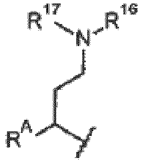 wie z. B. 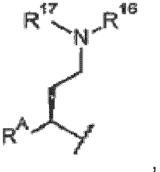 worin -RA -L^{A}-R^{AA} ist.

10. Verbindung nach Anspruch 9, worin -L^{A}- -R^{L}-* oder -O-L^{AA}-* ist.

11. Verbindung nach Anspruch 10, worin -L^{AA}- C₁₋₁₂-Alkylen, z. B. -CH₂-, ist.

12. Verbindung nach einem der Ansprüche 9 bis 11, worin -R^{AA} C₅₋₁₂-Aryl ist, das gegebenenfalls mit einer oder mehreren Gruppen -R^{S} oder einer oder mehreren Gruppen -R^{N} substituiert ist, z. B. worin -R^{AA} Phenyl ist, das gegebenenfalls mit einer oder mehreren Gruppen -R^{S} substituiert ist.

13. Pharmazeutische Zusammensetzung, die eine Verbindung nach einem der Ansprüche 1 bis 12 und einen biologisch annehmbaren Exzipienten, gegebenenfalls zusammen mit einem zweiten Wirkstoff, umfasst.

14. Verbindung nach einem der Ansprüche 1 bis 12 oder pharmazeutische Zusammensetzung nach Anspruch 13 zur Verwendung in einem Behandlungsverfahren.

15. Verbindung nach einem der Absätze 1 bis 12 oder pharmazeutische Zusammensetzung nach Anspruch 13 zur Verwendung in einem Verfahren zur Behandlung einer Mikrobeninfektion, wie z. B. ein Verfahren zur Behandlung einer gramnegativen Bakterieninfektion, z. B. wobei das gramnegative Bakterium aus der aus *Escherichia* spp., *Klebsiella* spp., *Enterobacter spp., Salmonella* spp., *Shigella* spp., *Citrobacter* spp., *Morganella morganii, Yersinia pseudotuberculosis* und anderen Enterobacteriaceae, *Pseudomonas* spp., *Acinetobacter* spp*., Moraxella, Helicobacter, Stenotrophomonas, Bdellovibrio,* Essigsäurebakterien, *Legionella* und alpha-Proteobakterien bestehenden Gruppe ausgewählt ist.

## Revendications

1. Composé de formule (III) : 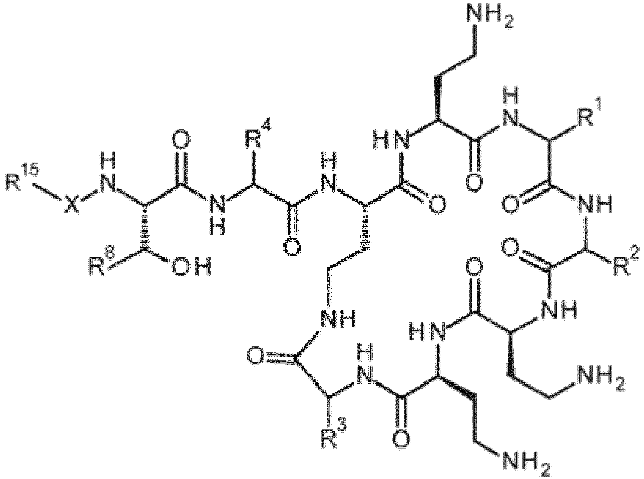 et des sels, formes protégées, solvates et hydrates pharmaceutiquement acceptables de celui-ci, tels que des sels et hydrates pharmaceutiquement acceptables de celui-ci,
dans lequel :
- X- représente -C(O)-, -NHC(O)-, -OC(O)-, -CH₂- ou -SO₂- ;
- R¹ conjointement avec le groupe carbonyle et l'azote alpha au carbone auquel il est fixé, est un résidu phénylalanine, leucine ou valine ;
- R² conjointement avec le groupe carbonyle et l'azote alpha au carbone auquel il est fixé, est un résidu leucine, iso-leucine, phénylalanine, thréonine, valine ou non-valine ;
- R³ conjointement avec le groupe carbonyle et l'azote alpha au carbone auquel il est fixé, est un résidu thréonine ou leucine ;
- R⁴ est un alkyle en C₁₋₆ substitué par un groupe hydroxyle ou un groupe amino ;
- R¹⁵ est un groupe contenant un amino : 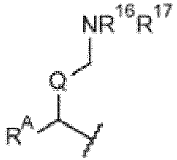
où :
-R^{A} est -L^{A}-R^{AA} ou un hydrogène ;
-Q- est une liaison covalente ou -CH(R^{B})- ;
-R^{B} est un hydrogène ou -L^{B}-R^{BB} ;
ou, lorsque -Q- est -CH(R^{B})-, -R^{A} et -R^{B} forment ensemble un carbocycle monocyclique ou bicyclique de 5 à 10 chaînons, ou -R^{A} et -R^{B} forment ensemble un hétérocycle monocyclique ou bicyclique de 5 à 10 chaînons ;
et, lorsque -Q- est une liaison covalente, -R^{A} est -L^{A}-R^{AA}, et lorsque -Q- est -CH(R^{B})- un ou plusieurs de -R^{A} et -R^{B} n'est pas un hydrogène ;
-R¹⁶ est indépendamment un hydrogène ou un alkyle en C₁₋₄ ;
-R¹⁷ est indépendamment un hydrogène ou un alkyle en C₁₋₄ ;
ou -NR¹⁶R¹⁷ est un groupe guanidine ;
ou -R¹⁷ et -R^{A} forment ensemble un hétérocycle monocyclique ou bicyclique contenant de l'azote de 5 à 10 chaînons ;
ou, lorsque -Q- est -CH(R^{B})-, -R¹⁷ et -R^{B} forment ensemble un hétérocycle monocyclique ou bicyclique contenant de l'azote de 5 à 10 chaînons ;
et lorsque -R¹⁷ et -R^{A} forment ensemble un hétérocycle monocyclique contenant de l'azote, chaque atome de carbone de noyau dans -R¹⁷ et -R^{A} est optionnellement mono- ou di-substitué par -R^{C}, et l'hétérocycle monocyclique est substitué par au moins un groupe choisi parmi -R^{C}, -R^{N}, -R^{NA} et -L^{B}-R^{BB}, le cas échéant,
et lorsque -R¹⁷ et -R^{B} forment ensemble un hétérocycle monocyclique contenant de l'azote, chaque atome de carbone de noyau dans -R¹⁷ et -R^{B} est optionnellement mono- ou di-substitué par -R^{C}, et l'hétérocycle monocyclique est substitué par au moins un groupe choisi parmi -R^{C}, et -R^{N}, le cas échéant, ou l'hétérocycle monocyclique est optionnellement substitué lorsque -R^{A} est -L^{A}-R^{AA},
et un hétérocycle monocyclique contenant de l'azote contient optionnellement un atome de noyau d'azote, d'oxygène ou de soufre supplémentaire, et lorsqu'un atome de noyau d'azote supplémentaire est présent, il est optionnellement substitué par -R^{N}, à l'exception d'un atome de noyau d'azote supplémentaire qui est relié au carbone qui est α au groupe -X-, lequel atome de noyau d'azote est optionnellement substitué par -R^{NA} ;
lorsque -R¹⁷ et -R^{A} ou -R¹⁷ et -R^{B} forment ensemble un hétérocycle bicyclique contenant de l'azote, chaque atome de carbone de noyau dans -R¹⁷ et -R^{A} ou -R¹⁷ et -R^{B} est optionnellement mono- ou di-substitué par -R^{D} ;
et l'hétérocycle bicyclique à atomes de noyau contenant de l'azote contient optionnellement un, deux ou trois hétéroatomes supplémentaires, lorsque chaque hétéroatome est indépendamment choisi dans le groupe constitué de l'azote, de l'oxygène et du soufre, et lorsque des atomes de noyau d'azote supplémentaires sont présents, chaque atome de noyau d'azote supplémentaire est optionnellement substitué par -R^{N}, à l'exception d'un atome de noyau d'azote qui est relié au carbone qui est α au groupe -X-, lequel atome de noyau d'azote est optionnellement substitué par - R^{NA} ;
lorsque -R^{A} et -R^{B} forment ensemble un carbocycle ou un hétérocycle monocyclique de 5 à 10 chaînons, chaque atome de carbone de noyau dans -R^{A} et -R^{B} est optionnellement mono- ou di-substitué par -R^{C}, et un atome de noyau d'azote, le cas échéant dans l'hétérocycle monocyclique, est optionnellement substitué par -R^{N}, à l'exception d'un atome de noyau d'azote qui est relié au carbone qui est α au groupe -X-, lequel atome de noyau d'azote est optionnellement substitué par -R^{NA} ;
lorsque -R^{A} et -R^{B} forment ensemble un carbocycle ou un hétérocycle bicyclique de 5 à 10 chaînons, chaque atome de carbone de noyau dans -R^{A} et -R^{B} est optionnellement mono- ou di-substitué par -R^{D}, et un atome de noyau d'azote, le cas échéant dans l'hétérocycle bicyclique, est optionnellement substitué par -R^{N}, à l'exception d'un atome de noyau d'azote qui est relié au carbone qui est α au groupe -X-, lequel atome de noyau d'azote est optionnellement substitué par -R^{NA} ;
et lorsque R¹⁷ et -R^{A} ou -R¹⁷ et -R^{B} forment ensemble un hétérocycle monocyclique ou bicyclique contenant de l'azote de 5 à 10 chaînons, ou lorsque -R^{A} et -R^{B} forment ensemble un carbocycle monocyclique ou bicyclique de 5 à 10 chaînons, ou forment ensemble un hétérocycle monocyclique ou bicyclique de 5 à 10 chaînons, un atome de noyau de carbone dans -R¹⁷ et -R^{A}, -R¹⁷ et -R^{B}, ou -R^{A} et -R^{B} est optionnellement en variante substitué par un oxo (=O) ; chaque -R^{C} est indépendamment -L^{C}-R^{CC} ;
chaque -R^{D} est indépendamment choisi parmi -R^{C}, un halogéno, -NO₂ , -OH et -NH₂ ;
chaque -R^{N} est indépendamment -L^{N}-R^{NN} ;
chaque -R^{NA} est indépendamment -R^{L}-R^{NN} ou -R^{NN} ;
-R^{AA}, -R^{BB}, et chaque -R^{CC} et -R^{NN} le cas échéant, est indépendamment choisi parmi un alkyle en C₁₋₁₂, un cycloalkyle en C₃₋₁₀, un hétérocyclyle en C₄₋₁₀ et un aryle en C₅₋₁₂ ;
chaque -L^{A}- est indépendamment une liaison covalente ou un groupe de liaison choisi parmi -R^{L}-*, -O-L^{AA}-*, -OC(O)-L^{AA}-*, -N(R¹¹) -L^{AA}-* et -C(O)-L^{AA}-*, où l'astérisque indique le point de fixation du groupe -L^{A}- à -R^{AA} ;
chaque -L^{B}- et -L^{C}- est indépendamment une liaison covalente ou un groupe de liaison choisi parmi -R^{L}-*, -O-L^{AA}-*,-OC (O)-L^{AA}-* , -N(R¹¹)-L^{AA}-*, -N(R¹¹)C(O)-L^{AA}-*, -C (O) -L^{AA}-* ,-C(O)O-L^{AA}-* et -C(O)N(R¹¹) - L^{AA}-*, et en outre optionnellement choisi parmi -N(R¹¹)S(O)-L^{AA}-*, -N(R¹¹)S(O)₂-L^{AA}-*, -S(O)N(R¹¹)-L^{M}-* et -S(O)₂N(R¹¹)-L^{AA}-* où l'astérisque indique le point de fixation du groupe -L^{B}- à -R^{BB} ou du groupe -L^{C}- à -R^{CC} ;
chaque -L^{N}- est indépendamment une liaison covalente ou un groupe choisi parmi -S(O)-L^{AA}-*, -S(O)₂-L^{M}-*, -C(O)-L^{AA}-* et -C(O)N(R¹¹)-L^{AA}-*, où l'astérisque indique le point de fixation du groupe -L^{N}- à -R^{NN} ;
et chaque -L^{M}- est indépendamment une liaison covalente ou -R^{L}- ;
et chaque -R^{L}- est indépendamment choisi parmi un alkylène en C₁₋₁₂, un hétéroalkylène en C₂₋₁₂, un cycloalkylène en C₃₋₁₀ et un hétérocyclylène en C₅₋₁₀, et lorsque -L^{M}- est relié à un groupe alkyle en C₁₋₁₂, -R^{L}- n'est pas un alkylène en C₁-12 ;
et chaque groupe alkyle en C₁₋₁₂, cycloalkyle en C₃₋₁₀, hétérocyclyle en C₄₋₁₀, aryle en C₅₋₁₂, alkylène en C₁₋₁₂, hétéroalkylène en C₂₋₁₂, cycloalkylène en C₃₋₁₀ et hétérocyclylène en C₅₋₁₀ est optionnellement substitué, lorsque -R^{S} est un substituant optionnel au carbone et -R¹² est un substituant optionnel à l'azote ;
chaque -R^{S} est indépendamment choisi parmi -OH, -OR¹²,-OC(O)R¹², un halogéno, -R¹², -NHR¹², -NR¹²R¹³, -NHC(O)R¹²,-N(R¹²)C(O)R¹², -SH, -SR¹², -C(O)R¹², -C(O)OH, -C(O)OR¹²,-C(O)NH₂, -C(O)NHR¹² et C(O)NR¹²R¹³, tel que dans lequel chaque -R^{S} est indépendamment choisi parmi -OR¹², un halogéno et-R¹² ; à l'exception que -R¹² n'est pas un substituant à un groupe alkyle en C₁₋₁₂ ; ou lorsqu'un atome de carbone est di-substitué par -R^{S}, ces groupes peuvent conjointement avec le carbone auquel ils sont fixés former un carbocycle en C₃₋₆ ou un hétérocycle en C₅₋₆, lorsque le carbocycle et l'hétérocycle sont optionnellement substitués par un ou plusieurs groupes -R¹² ;
chaque -R¹² est indépendamment un alkyle en C₁₋₆, un halogénoalkyle en C₁₋₆, un phényle ou un benzyle ;
chaque -R¹³ est indépendamment un alkyle en C₁₋₆, un halogénoalkyle en C₁₋₆, un phényle ou un benzyle ;
ou -R¹² et -R¹³, lorsqu'ils sont fixés à N, peuvent former ensemble un noyau hétérocyclique à 5 ou 6 chaînons, qui est optionnellement substitué par un alkyle en C₁₋₆, un halogénoalkyle en C₁₋₆, un phényle ou un benzyle ;
chaque -R¹¹ est indépendamment un hydrogène ou un alkyle en C₁₋₄ ; et
-R⁸ est un hydrogène ou un méthyle.

2. Composé selon la revendication 1, dans lequel :
(i) -X- est -C(O)- ; et/ou
(ii) -R⁸ est un méthyle ; et/ou
(iii) -R¹ conjointement avec le groupe carbonyle et l'azote alpha au carbone auquel il est fixé est un résidu phénylalanine ou un résidu leucine ; et/ou
(iv) -R² conjointement avec le groupe carbonyle et l'azote alpha au carbone auquel il est fixé est un résidu leucine ; et/ou
(v) -R³ conjointement avec le groupe carbonyle et l'azote alpha au carbone auquel il est fixé est un résidu thréonine ; et/ou
(vi) -R⁴ conjointement avec le groupe carbonyle et l'azote alpha au carbone auquel il est fixé, est un acide α,γ-diaminobutyrique (Dab), un résidu sérine, un résidu thréonine, un résidu lysine, un résidu ornithine ou un acide α,β-diaminopropionique (Dap) , tel que dans lequel -R⁴ conjointement avec le groupe carbonyle et l'azote alpha au carbone auquel il est fixé est un acide α,γ-diaminobutyrique (Dab) ou un acide α,β-diaminopropionique (Dap), tel que L-Dab ou L-Dap ; et/ou
(vii) -R¹⁶ est un hydrogène ; et/ou
(viii) -R¹⁷ est un hydrogène.

3. Composé selon la revendication 1 ou la revendication 2, dans lequel -R¹⁵ est : 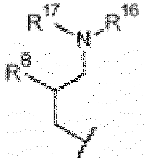 où -R^{B} est -L^{B}-R^{BB}.

4. Composé selon la revendication 3, dans lequel -L^{B}- est une liaison covalente.

5. Composé selon la revendication 3, dans lequel -L^{B}- est un alkylène en C₁₋₁₂.

6. Composé selon l'une quelconque des revendications 3 à 5, dans lequel -R^{BB} est indépendamment un groupe choisi parmi un alkyle en C₁₋₁₂, un hétérocyclyle en C₄₋₁₀ et un aryle en C₅₋₁₂, et chaque groupe est optionnellement substitué par un ou plusieurs groupes -R^{S} ou un ou plusieurs groupes -R^{N}.

7. Composé selon la revendication 6, dans lequel -R^{BB} est un alkyle en C₁₋₁₂, optionnellement substitué par un ou plusieurs groupes -R^{S}, tel que dans lequel -R^{BB} est un alkyle en C₁₋₁₂.

8. Composé selon la revendication 6, dans lequel -R^{BB} est un aryle en C₅₋₁₂, optionnellement substitué par un ou plusieurs groupes -R^{S} ou un ou plusieurs groupes -R^{N}, tel que dans lequel -R^{BB} est un phényle ou un naphtyle, optionnellement substitué par un ou plusieurs groupes -R^{S}.

9. Composé selon la revendication 1 ou la revendication 2, dans lequel -R¹⁵ est : 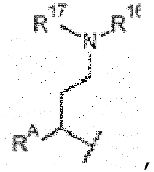 tel que 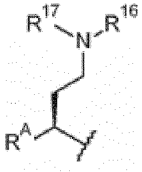 où -R^{A} est -L^{A}-R^{AA}.

10. Composé selon la revendication 9, dans lequel -L^{A}- est -R^{L}-* ou -O-L^{AA}-*.

11. Composé selon la revendication 10, dans lequel -L^{AA}- est un alkylène en C₁₋₁₂, tel que -CH₂-.

12. Composé selon l'une quelconque des revendications 9 à 11, dans lequel -R^{AA} est un aryle en C₅₋₁₂, optionnellement substitué par un ou plusieurs groupes -R^{S} ou un ou plusieurs groupes -R^{N}, tel que dans lequel -R^{AA} est un phényle optionnellement substitué par un ou plusieurs groupes -R^{S}.

13. Composition pharmaceutique comprenant un composé selon l'une quelconque des revendications 1 à 12 et un excipient biologiquement acceptable, optionnellement conjointement avec un second agent actif.

14. Composé selon l'une quelconque des revendications 1 à 12 ou composition pharmaceutique selon la revendication 14 pour une utilisation dans un procédé de traitement.

15. Composé selon l'un quelconque des paragraphes 1 à 12 ou composition pharmaceutique selon la revendication 14 pour une utilisation dans un procédé de traitement d'une infection microbienne, tel qu'un procédé de traitement d'une infection bactérienne Gram-négative, tel que dans lequel la bactérie Gram-négative est choisie dans le groupe constitué des espèces *Escherichia,* des espèces *Klebsiella,* des espèces *Enterobacter,* des espèces *Salmonella,* des espèces *Shigella,* des espèces *Citrobacter,* des espèces *Morganella,* des espèces *Yersinia pseudotuberculosis* et autres Enterobacteriaceae, des espèces *Peudomonas,* des espèces *Acinetobacter,* de *Moraxella,* de *Helicobacter,* de *Stenotrophomonas,* de *Bdellovibrio,* des bactéries à acide acétique, de *Legionella* et des alpha-protéobactéries.
